# EUROPEAN PATENT SPECIFICATION

(11) **EP 4 108 169 B1**
(45) Date of publication and mention of the grant of the patent: **19.08.2026**
(21) Application number: 20890729.5
(22) Date of filing: 13.11.2020
(51) Int. Cl.: A61B 5/145, A61B 5/1455, G01N 33/68, G01N 33/573, A61B 5/03

(54) **METHOD AND DEVICE FOR LIVING TISSUE SPECTROSCOPY**
VERFAHREN UND VORRICHTUNG ZUR SPEKTROSKOPIE VON LEBENDEM GEWEBE
PROCÉDÉ ET DISPOSITIF DE SPECTROSCOPIE DE TISSUS VIVANTS

(30) Priority: 18.11.2019 RU 2019136897
(43) Date of publication of application: 28.12.2022
(73) Proprietor: Musin, Ramil Faritovich, Moscow 123007 (RU)
(72) Inventor: Musin, Ramil Faritovich, Moscow 123007 (RU)
(74) Representative: Long, Giorgio
(86) International application number: PCT/RU2020/050324
(87) International publication number: WO 2021/101416

(56) References cited:
- EP-A1- 3 466 320
- WO-A1-2008/071832
- WO-A1-2017/204677
- RU-C2- 2 288 636
- US-A1- 2004 127 777
- US-A1- 2017 266 321

## Description

### PRIOR ART

The present group of inventions refers to the medicine and medical technology and more particularly to methods and devices for monitoring blood glucose level by measuring the calorimetric method of the thermal effect and metabolic rate of the local area of living tissue. Also, the present invention relates to methods of producing a biopolymer molecule with predetermined biological properties and a predetermined spatial structure. The use of the group of inventions will allow non-invasive monitoring of blood glucose levels in diabetic patients, including early diagnosis of diabetes mellitus.

### BACKGROUND OF THE INVENTATION

One of the prevalent and dangerous diseases of the times is diabetes mellitus, the most common form of which, accounting for over 90-95% of all diabetics, is type 2 diabetes mellitus (DM2), which is one of the most pressing problems of modern medicine. The social importance of this chronic disease is determined by several factors, the most important of which is the high prevalence of the disease throughout the world, while the methods of its treatment are still under development. According to the forecast of the International Diabetes Federation (IDF), the number of patients with diabetes by 2035 will increase up to 592 million compared with 422 million in 2018.

U.S. Federal health statistics center shows that 12.3% of Americans aged 20 and more are living with diabetes. And about 3% are not even aware of this, thereby aggravating their condition. The other 37% live with pre-diabetes (a condition characterized by elevated blood glucose levels). By comparison, 10 years ago this figure was only 27% instead of 37%. Researchers from the US Centers for Disease Control say, if people with pre-diabetes do not change their lifestyle for the better, in 30% of cases they will develop type 2 diabetes within 5 years. According to the prognosis of International Diabetes Federation (IDF), spending on diabetes treatment will rise up to $936 billion by 2035, from $586 billion in 2012.

Diabetes often leads to the development of complications such as blindness, renal disorders, nervous diseases and cardiovascular diseases. Diabetes is a leading disease resulting in blindness at the age of 20 to 74 years. Approximately from 12,000 to 24,000 persons annually loss vision because of diabetes. Diabetes is a leading cause of renal diseases in about 40% of new cases. About 40 to 60% of patients with diabetes are predisposed to different forms of nervous diseases, which can result in amputation of limbs. Patients with diabetes are approximately 2 to 4 times more predisposed to cardiac diseases, in particular myocardial infarction.

Diabetes is a disease associated with insufficient production or inefficient use of insulin by cells of the body. In spite of the fact that causes of the disease are not completely understood, some factors such as genetic, environmental, viral have been identified.

There are two main forms of diabetes: type 1 and type 2.

Type 1 diabetes (known as insulin-dependent diabetes) is an autoimmune disease wherein insulin production completely terminates; and it most often develops in childhood and youth. Patients with type 1 diabetes need daily insulin injections.

Type 2 diabetes is a metabolic disease caused by that the body cannot produce a sufficient amount of insulin or utilization thereof is inefficient. Patients with type 2 diabetes make up about 90 to 95% of a total amount of diabetics. Morbidity of type 2 diabetes in the USA approaches an epidemiologic threshold, mainly due to increase in the number of elderly Americans and a significant prevalence of a sedentary lifestyle leading to obesity.

Insulin promotes glucose penetration into a cell with subsequent cleavage thereof to obtain energy for all metabolic processes. In diabetics, glucose cannot penetrate into a cell, it accumulates in blood and cells experience energetic hunger.

Patients with type 1 diabetes inject insulin to themselves using a special syringe and a cartridge. Continuous subcutaneous injection of insulin through an implanted pump is also possible. Insulin is typically prepared from swine pancreas or is synthesized chemically.

Generally accepted medical methods of diabetes treatment prescribe patients taking insulin to carry out independent monitoring of blood glucose level. Knowing blood glucose level, patients can adjust insulin dose in subsequent injection. Adjustment is necessary, since due to different reasons, blood glucose level fluctuates during a day and from day to day. In spite of the importance of such monitoring, several conducted studies showed that a portion of patients who carry out such monitoring at least once a day, diminishes with age. This fall occurs mainly because the currently used method of monitoring is associated with invasive drawing a blood sample from a finger. Many patients consider drawing a blood sample from a finger to be a more painful procedure that insulin injection.

Research and development (R&D) aimed at addressing the problem of diabetes mellitus are actively pursued in the following technological areas:
1) Creation of a non-invasive (bloodless) method for continuous monitoring of the patients' blood glucose level;
2) Creation of invasive methods of blood glucose level measurement based on new-generation biosensors;
3) Development of wearable electronic devices for preventive medicine for early diagnosis of diabetes mellitus based on new-generation biosensors and biotechnologies;
4) Creation and implementation into clinical practice of novel effective and safe anti-diabetic drugs.

Thus, creation of a non-invasive (bloodless) method for monitoring blood glucose level in diabetic patients and a medical device on the basis thereof capable of replacing industrial invasive glucometers is one of the topical unsolved problems of modern medicine and public healthcare. The task of developing new methods and devices for preventive medicine for early diagnosis of diabetes mellitus based on noninvasive continuous recording of glucose curves is closely related to the development of wearable electronic devices for noninvasive continuous blood glucose level monitoring based on new-generation biosensors and biotechnology.

A commercial glucose meter for measuring blood glucose levels in an invasive manner, is a biosensor that uses a glucose oxidase enzyme for blood glucose cleavage. During the cleavage process, the enzyme first oxidizes glucose and uses two electrons to reduce FAD (a component of the enzyme) to FAD-H2, which, in turn, is oxidized in several steps by the electrode. The resulting current is proportional to the concentration of glucose. In this case, the electrode is the transducer and the enzyme is the bioselective element.

Methods and devices for noninvasive measuring blood glucose level are known: [1-10].

Method of calorimetry is known to be widely used in biology for the study of thermal processes at the molecular and cellular levels [1-10]. The microcalorimetry method is also successfully used to study thermal processes (heat release and absorption) in separate organs, in particular, in active muscles and nerve fibers.

Increased activity over the past decade in studies aimed at creating the microcalorimetry method for studying in physiological conditions in vivo, the thermal processes associated with the metabolism of a local area of human living tissue, is largely due to the work on the development of a noninvasive method for monitoring blood glucose level (noninvasive glucometer) based on monitoring of heat production of a local area of human tissue.

The direct calorimetry and indirect calorimetry are the known methods of physiological calorimetry. The direct calorimetry method contemplates immediate determination of a total amount of irradiated heat using a calorimetric chamber for live objects.

The indirect calorimetry method allows for determining an amount of irradiated heat in an indirect way based on accounting respiratory gas exchange dynamics using respiratory chambers and different systems. Two possible modifications of the indirect calorimetry method are distinguished: a method of complete gas analysis (accounting absorbed O2 and evolved CO2) and a method of incomplete gas analysis (accounting absorbed O2).

A known method of measuring the basal metabolic rate of the human body using a wholebody calorimeter (direct calorimetry) is described in US 4386604 A, Publ. 07.06.1983. By change in an air temperature and a total water amount evaporating from the whole-body surface, a total whole-body heat irradiation is determined and the basal metabolic rate is calculated. The main drawback of this method consists in that for embodiment thereof, cumbersome, stationary and expensive whole-body calorimetric chambers are required. Furthermore, the direct calorimetry method is characterized by a low accuracy. The method and device for microcalorimetric measurement of the rate of local metabolism of tissue, water content of intercellular tissue, concentration of biochemical components of blood and pressure in the cardiovascular system, described in [11], is known, wherein the measurement of heat effect and a rate of metabolism of a local tissue site determine the blood glucose level (RU 2396897). The metabolic rate is determined by measuring the total amount of water evaporating during an insensible perspiration from the surface of a local area of the skin and measuring the temperature and humidity of the ambient air. The method of measurement makes it possible to realize a high accuracy in laboratory conditions with controlled parameters of a microclimate (temperature and humidity of the room) wherein the measurement is carried out, with constant values of climatic parameters of the environment (temperature and humidity of the environment, atmospheric pressure). The main disadvantage of this method, limiting its practical application, is that the results of measurements depend on the physic-and-climatic factors of the environment; in each case, a new calibration of the measuring channel is required when the climatic factors change, with constant values of the microclimate parameters of the room where measurements are taken.

Spectral methods for non-invasive measurement of blood glucose concentration are known from [1], as well as from EP3466320, which described a calorimetric method for non-invasive tissue monitoring. The main disadvantage of known non-invasive devices for measuring blood glucose concentration based on diffuse reflectance spectroscopy and absorption spectroscopy is a high measurement error due to the low ratio of the registered signal to noise, which limits the accuracy of spectral measurements at characteristic frequencies of blood glucose molecules in blood capillaries located 0.5-1 mm deep from the epidermal surface.

The well-known method of optical-calorimetric spectroscopy is based on light absorption with excitation of energy levels of the substance molecules of a biological object, followed by emission-free relaxation of levels and heating of the object. **In** contrast to the method of isothermal calorimetric spectroscopy described above, in the method of optical-calorimetric spectroscopy, the temperature of the substance is a variable; the information parameter in this method is the change in the temperature of the testing sample. The degree of heating is determined by the magnitude of the substance absorption capacity, the light intensity and the efficiency of competing processes (fluorescence, photochemical and photoelectric effects). The main advantage of optical-calorimetric methods is the possibility of recording absorption spectra of highly scattering media, which is very important in biology and medicine. Since the measured parameter is a change in temperature, non-selective radiation receivers are used as detectors, i.e. no restrictions on the receiver wavelengths are presented.

However, none of the existing non-invasive continuous blood glucose monitoring techniques allows accurate real-time monitoring of the content and temporal dynamics of the biochemical components of living tissue, which are the main factor used for early diagnosis and therapy of diabetes mellitus.

### DISCLOSURE OF THE INVENTION

A technical task and technical result of the present invention consist in the development of a fundamentally new method of living tissue spectroscopy under in vivo conditions - absorption isothermal calorimetric spectroscopy, which allows to determine the content of biochemical components of living tissue in real time, and a device that allows this method to be carried out.

The developed method and device for non-invasive continuous monitoring of blood glucose level are used for early diagnosis of diabetes mellitus and monitoring of blood glucose level in the process of hypoglycemic therapy and are based on the use of new-generation biosensors.

An additional technical task and a technical result of the present invention is the creation of a new class of biosensors and biological catalysts to be used in a variety of fields of human activity, in addition to early diagnosis of diabetes: in the fields of new-generation drug development, biomedicine and protein and genetic engineering, food industry, safety and environmental quality monitoring.

One of the promising application areas for the group of inventions is the creation and introduction into clinical practice of new effective and safe medicinal products, in particular, glucokinase is one of the promising targets for the creation of new antidiabetic drugs. An additional technical task and technical result of the present invention is the development of a method for obtaining a biopolymer molecule with specified biological properties and a specified spatial structure. The proposed method for obtaining a biopolymer molecule allows to determine the primary structure of a biopolymer molecule, which, with a given biochemical composition of a solvent, under certain conditions (temperature, pressure, acidity) can collapse into a biopolymer molecule with a heterophase structure, having the functions of a biosensor and/or biological catalyst, with bioselectivity to a given solvent molecule S, catalyzing a biochemical reaction involving the given molecule S. The polymer chain of the biopolymer molecule with specified properties, the structure of which is determined by the proposed method, is synthesized by protein and/or genetic engineering methods at the next stage of the technological chain. On the basis of biopolymer molecules with a heterophase structure obtained by the proposed method, a new generation of biological catalysts and effective and safe drugs for the treatment of, in particular, diabetes mellitus and other chronic socially significant diseases can be created. **In** addition, the proposed method includes the stage of determining the three-dimensional spatial structure and biological properties of the biopolymer molecule. The proposed method for determination of the three-dimensional spatial structure and biological properties of the biopolymer molecule based on the known primary structure of the polymer chain has important practical application for the development of new drugs and new methods of treatment. The problem of predicting the spatial structure of the biopolymer molecule and its relation with biological function is one of the biggest unsolved problems of modern science, solution to which has made it possible to create principally new biotechnologies that can be applied in protein and gene engineering.

The proposed method and device for its implementation, focused on non-invasive continuous monitoring of blood glucose level, allows to determine the blood glucose level by measuring with the help of fundamentally new method of in vivo absorption isothermal calorimetric spectroscopy, which allows determining the content of biochemical components of a living tissue in real time. The magnitude of electromagnetic radiation power at the characteristic frequency of the biochemical component under study, absorbed in a local volume of substance of a living tissue, is measured not by the change in tissue temperature, but by the change in osmotic pressure value (or amount of water in the intercellular space), characterizing the volume of intercellular substance, which is in heterophase condensed state, at the phase transition temperature (corresponding to the thermodynamic equilibrium
of the crystalline and liquid phases of the intercellular substance). **In** this case, the amount of absorbed power of electromagnetic radiation falling on the surface of the studied local area of living tissue epidermis is determined by the amount of intercellular substance that has changed its phase state (melting of the globular phase of hyaluronic acid), by measuring the intercellular substance osmotic pressure in real time.

The proposed method of early diagnosis and therapy of diabetes mellitus can be implemented using a fundamentally new method of in vivo absorption isothermal calorimetric spectroscopy of a living tissue, which allows to determine the content of biochemical components of a living tissue in real time.

The said technical result is achieved by implementing a method of isothermal calorimetric spectroscopy of biochemical components of a patient's living tissue, comprising the following steps:
- apply at least one heat- and waterproof applicator on a skin surface with a dosed pressure, forming a closed system in the local area of tissue under the applicator;
- exert a local effect on the tissue area under the applicator by electromagnetic radiation at one or several wavelengths corresponding to the characteristic frequencies of absorption of biochemical components of intercellular and/or intracellular substance;
- measure the value of physiological parameter, characterizing the thermodynamic phase state of the intercellular substance under the applicator and its temporal dynamics, depending on the power of incident electromagnetic radiation;
- determine the concentration of the biochemical component of the intercellular substance and/or intercellular fluid and/or blood and its temporal dynamics based on the temporal dynamics of the measured physiological parameter.

In particular embodiments of the invention, the intensity of the electromagnetic radiation may be constant or variable, varying at a constant rate and/or modulated frequency and/or amplitude.

In particular embodiments of the invention, the physiological parameter characterizing the thermodynamic phase state of the intercellular substance is the osmotic pressure of the intercellular substance and/or the amount of water in the intercellular space and/or the elastic pressure of the living tissue under the applicator.

In particular embodiments of the invention, the biochemical components of intercellular and/or intracellular substance are selected from the group consisting of water, hyaluronic acid, glucose, triglycerides and other biochemical components of intercellular substance, cells and blood.

In particular embodiments of the invention, the wavelength of the electromagnetic radiation applied to the tissue region is selected from the range of electromagnetic radiation, which is determined from the characteristic absorption frequencies of the living tissue biochemical components in the optical and/or near infrared and/or mid and far infrared and/or terahertz and/or microwave range.

In particular embodiments of the invention, the concentration of the biochemical component of the intercellular substance and/or intercellular fluid and/or blood is additionally measured based on diffuse reflectance spectroscopy.

In particular embodiments of the invention, the concentration of the biochemical component of the intercellular substance and/or intercellular fluid and/or blood is additionally measured based on Raman spectroscopy.

In particular embodiments of the invention, the biochemical component is blood glucose, the concentration of which is determined from the concentration of glucose bound to the polymer chain monomers of the intercellular substance.

In particular embodiments of the invention, the osmotic pressure of the intercellular substance or the amount of water in the intercellular space is determined based on spectral measurements at wavelengths corresponding to characteristic absorption frequencies of biochemical components of the intercellular and/or intracellular substance.

In particular embodiments of the invention, the spectral measurements are based on a two-frequency spectroscopy method, wherein the wavelength of the electromagnetic radiation is selected by the characteristic frequency of the hyaluronic acid intercellular tetramer in the terahertz region, and the wavelength of the intercellular osmotic pressure spectrometer is selected by the characteristic frequencies of water absorption in the stratum corneum in the infrared region.

In particular embodiments of the invention, the osmotic pressure of the intercellular substance or the amount of water in the intercellular space is determined from the amount of water in the stratum corneum based on measurements of physical characteristics of the stratum corneum, which are selected from the group consisting of electrophysical characteristics, spectral and optical-acoustic characteristics, and thermal physical characteristics.

In particular embodiments of the invention, the osmotic pressure of the intercellular substance or the amount of water in the intercellular space is determined from the amount of water in the stratum corneum based on measurements of spectral characteristics of the stratum corneum at wavelengths corresponding to characteristic frequencies of water in the stratum corneum, using a spectral method that is selected from the group consisting of: IR spectroscopy, Raman spectroscopy, optical-acoustic spectroscopy, double-beam spread spectroscopy.

In particular embodiments of the invention, the osmotic pressure of the intercellular
substance or the amount of water in the intercellular space is determined from the amount of water in the stratum corneum by measuring the electrical characteristics of the stratum corneum, which are selected from the group consisting of the transverse electrical conductivity of the stratum corneum at DC and/or AC currents, as well as the dielectric permittivity.

In particular embodiments of the invention, the blood glucose concentration is determined based on the temporal dynamics of a physiological parameter, the physiological parameter being the osmotic pressure of the intercellular substance of the tissue region below the applicator, with the intercellular substance below the applicator being a natural biosensor with a heterophase structure with selectivity to the glucose molecule and sensitivity to external heat flows, and the individual calibration of the patient's local tissue area is determined by the content of hyaluronic acid in the intercellular substance and the amount of water in the tissue volume under the applicator, measured by the spectral method.

In particular embodiments of the invention, the method is applicable for early diagnosis of diabetes mellitus based on the temporal dynamics of the biochemical component concentration.

In particular embodiments of the invention, the method is applicable for early diagnosis of diabetes mellitus based on the temporal dynamics of intercellular osmotic pressure.

The specified technical result is also achieved through the development and creation of a device for isothermal calorimetric spectroscopy of biochemical components of living tissue, containing a heat- and waterproof applicator, made with the possibility of application to the skin of a patient with dosed pressure, a temperature sensor, one or several sensors of physiological parameters, which characterize the thermodynamic phase state of the intercellular substance under the applicator, one or several electromagnetic radiation sources, a device for creating a calibration effect, with the sensors of physiological parameters located under the applicator, with the signals from the above sensors coming in series to the inputs of an amplifier module and/or a synchronous detector and/or an analog-digital converter, installed on the upper surface of the applicator, to the information processing unit and information display unit.

In particular embodiments of the invention, the electromagnetic radiation source is made to emit with constant and/or modulated intensity and/or frequency modulation.

In particular embodiments of the invention, sensors of physiological parameters characterizing the thermodynamic state of the intercellular substance under the applicator are made as sensors of osmotic pressure of the intercellular substance or sensors of amount of water in the intercellular tissue space in the local volume under the applicator and/or sensors of elastic pressure of the living tissue under the applicator.

In particular embodiments of the invention, a device for creating a calibration effect is a source of thermal power, made in the form of a resistor and/or a Peltier element and/or a source of electric current and/or voltage; a device for creating dosed pressure on the surface of the applicator.

In particular embodiments of the invention, the radiation range of electromagnetic radiation sources is selected from a group including optical and/or near infrared band; middle infrared band; far infrared and/or terahertz band; microwave radiation band.

In particular embodiments of the invention, an osmotic pressure sensor of the intercellular substance or a sensor of the amount of water in the intercellular space of tissue in a local volume under the applicator is an electrometric sensor based on measuring the electrophysical characteristics of the stratum corneum and tissue under the applicator, the measurement principle of which is selected from a group including measurement of the transverse electrical conductivity of the stratum corneum at a constant and/or alternating current; measurement of the dielectric constant of the stratum corneum; measurement of electrical conductivity and/or dielectric permittivity of the tissue under the applicator.

In particular embodiments of the invention, an osmotic pressure sensor of the intercellular substance or a sensor of the amount of water in the intercellular space of tissue in a local volume under the applicator is an electrometric sensor based on measuring the electrophysical characteristics of the stratum corneum and tissue under the applicator, the measurement principle of which is selected from a group including measurement of the transverse electrical conductivity of the stratum corneum at a constant and/or alternating current; measurement of dielectric permittivity of the stratum corneum; measurement of electrical conductivity and/or dielectric permittivity of the tissue under the applicator.

In particular embodiments of the invention, the wavelengths of electromagnetic radiation from the source of the spectral sensor are determined by the characteristic absorption frequencies of the biochemical components of the intercellular substance and / or intracellular substance, which are selected from a group including water, hyaluronic acid, glucose and other biochemical components of the intercellular substance and intercellular fluid.

In particular embodiments of the invention, the osmotic pressure sensor of the intercellular substance is a spectral sensor based on spectral measurements of the intercellular substance characteristics in the terahertz range at a wavelength corresponding to the cross-coupling energy between the monomers of the hyaluronic acid polymer chain.

In particular embodiments of the invention, the spectral sensor is based on
spectral measurements of the amount of water in the stratum corneum by the characteristic frequencies of water in the stratum corneum based on a spectral method that is selected from a group including isothermal calorimetric spectroscopy, absorption spectroscopy, diffusion reflectance spectroscopy, Raman spectroscopy, optical-acoustic spectroscopy.

In particular embodiments, the device according to the invention additionally contains a spectral device including a source and receiver of electromagnetic radiation for spectral measurements based on diffusion reflectance spectroscopy.

In particular embodiments of the invention, the sensor of physiological parameters characterizing the thermodynamic phase state of the intercellular substance under the applicator is made using a piezoelectric measurement method.

In particular embodiments of the invention, the device is applicable for early diagnosis of diabetes mellitus based on the time dynamics of the biochemical parameter concentration and / or the value of the osmotic pressure of the intercellular substance.

The specified technical result is also achieved by implementing a method for obtaining a biopolymer molecule with specified biological properties and a specified spatial structure consisting of one or several subunits capable of spontaneous folding into a spatial configuration with a heterophase structure having specificity to one or several specified substrate S molecules, with a certain solvent composition, under certain conditions for temperature and pressure, which consists in the fact, that the number of subunits is determined based on the three-dimensional spatial structure of a macromolecule, while the primary structure of each subunit of a biopolymer molecule is determined as follows:
- determine or set the equilibrium constant of the binding process of the substrate molecule S with the monomer of the polymer chain of the biopolymer molecule through the formation of a hydrogen bond;
- determine the activation energy and/or hydrogen bonding energy of the substrate S molecule with the monomer of the polymer chain of the biopolymer molecule corresponding to the value of the equilibrium constant is determined;
- by the magnitude of the activation energy and/or binding energy, identify monomers that form the primary structure of each subunit of a biopolymer macromolecule, namely, at least a pair of monomers A and B forming a repeating monomer -A- B- of the polymer chain in which A is a monomer containing a group having a negative electric charge, and B is a neutrally uncharged monomer with which the substrate S molecule can form a hydrogen bond, the energy of which corresponds to a given equilibrium constant;
- by the magnitude of the activation energy and/or binding energy, identify monovalent solvent ions corresponding to two different compounds capable of forming a weak ionic bond with a charged monomer, while one of the ions, ion M, is selected with an equilibrium constant close to the equilibrium constant of the substrate S;
- determine the acidity of the solvent pH, which determines the negative charge of monomer A, ion concentrations, temperature and pressure;
- determine the number of monomers in the chain;
- obtain a biopolymer molecule with a given primary structure using genetic and/or protein engineering methods;
- obtain a solution of a biopolymer molecule in a solvent, the spatial structure and characteristics of which correspond to the specified ones.

In particular embodiments of the invention, the polymer chain monomer A has a positive charge, and the solvent ion M has a negative charge.

In particular embodiments of the invention, the solvent ion M is a monovalent positively charged metal ion.

In particular embodiments of the invention, the primary structure of a biopolymer macromolecule consisting of one subunit is a polysaccharide chain, the repeating monomer of which contains at least one type of disaccharide pair formed by one monosaccharide A with a negatively charged group and one neutral monosaccharide B with no charged group.

In particular embodiments of the invention, the primary structure of a biopolymer macromolecule consisting of a single subunit is a hyaluronic acid polymer chain, the substrate molecule is a glucose molecule, the monovalent metal ion M is a sodium ion, while the chemical composition of the solvent is close to the chemical composition of blood plasma and the intercellular medium of a living system, under physiological conditions in vivo.

In particular embodiments of the invention, a divalent ion and/or a substance is added to the solvent, the molecule of which has the ability to form a divalent ionic bond with negatively charged monomers of the hyaluronic acid polymer chain.

In particular embodiments of the invention, a biopolymer molecule consisting of one subunit is a polypeptide chain of amino acids, with repeating monomers containing at least one amino acid with a negatively charged group Rₐ, and one amino acid with a neutral uncharged group R_{b}. The monovalent metal ion M is the potassium ion, while the chemical composition of the solvent is close to the chemical composition of the intracellular environment of the living system under physiological conditions in vivo.

In particular embodiments of the invention, additional divalent metal ions or divalent compounds are added to the solvent, which are selected from a group including magnesium ion Mg⁺², manganese Mn⁺², ions of other metals and compounds.

In particular embodiments of the invention, the substrate is a D-glucose molecule and/or a molecule of another monosaccharide.

In particular embodiments of the invention, a biopolymer molecule with a heterophase structure is a biosensor with selectivity to a given substrate molecule S, active in an aqueous medium, converting a signal of substrate concentration S into a signal proportional to the volume of the macromolecule and/or intramolecular osmotic pressure, which is measured by an electronic device.

In particular embodiments of the invention, the biopolymer molecule is a hyaluronic acid polysaccharide, which has selectivity to the glucose molecule.

In particular embodiments of the invention, a biopolymer molecule with specified biological properties, consisting of two subunits, capable of spontaneous folding in a spatial configuration with a heterophase structure, having specificity to a given substrate molecule S, with a certain solvent composition, under certain conditions of temperature and pressure, and the method is that for each of the two subunits, the primary structure is determined as follows:
- determine or set the equilibrium constant of the binding process of the substrate molecule S with the monomer of the polymer chain of the biopolymer molecule through the formation of a hydrogen bond;
- determine the activation energy and/or hydrogen bonding energy of the substrate S molecule with the monomer of the polymer chain of the biopolymer molecule corresponding to the value of the equilibrium constant is determined;
- by the magnitude of the binding energy, identify monomers that form the primary structure of the first subunit of a biopolymer macromolecule, namely, at least a pair of monomers A1 and B1 forming a repeating monomer -A1- B1- of the polymer chain, in which A1 is a monomer containing a group with a negative electric charge, and B1 is neutrally uncharged monomer with which a substrate S molecule can form a hydrogen bond, the energy of which corresponds to a given equilibrium constant;
- identify monomers that form the primary structure of each subunit of a biopolymer macromolecule, namely, at least a pair of monomers A and B forming a repeating monomer -A-B- polymer chain in which A is a monomer containing a group having a negative electric charge, and B is a neutrally uncharged monomer with which the substrate S molecule can form a hydrogen bond, the energy of which corresponds to a given equilibrium constant;
- by the magnitude of the binding energy, identify monovalent solvent ions corresponding to two different compounds or substances capable of forming a weak ionic bond with charged monomers, while one of the ions, ion M, is selected with an equilibrium constant close to the equilibrium constant of the substrate S;
- determine the acidity of the solvent pH, which determines the negative charge of monomer A, ion concentrations, temperature and pressure;
- determine the number of monomers in the chain;
- obtain a biopolymer chain consisting of two subunits with a given primary structure using genetic and/or protein engineering methods;
- obtain a solution of a biopolymer molecule in a solvent which characteristics correspond to the specified ones.

In particular embodiments of the invention, the subunits are polypeptide chains of amino acids.

In particular embodiments of the invention, for each subunit, the location of repeating monomers in the chain is determined, characterizing the interaction between subunits, forming a unique amino acid configuration of the active center of a protein molecule, which is formed during spatial folding in the region of space between the domains of subunits.

In particular embodiments of the invention, a bionolymer molecule with a heterophase structure is a biological catalyst with specified characteristics and selectivity to a given substrate molecule S, active in an aqueous medium, catalyzing biochemical reactions involving a substrate molecule S.

In particular embodiments of the invention, a biopolymer molecule is an enzyme with specified characteristics based on a biopolymer molecule with a heterophase structure.

In particular embodiments of the invention, the primary structure of a biopolymer molecule with specified biological properties is determined by the characteristics of the hexokinase enzyme, the equilibrium constant and the rate of the biochemical reaction catalyzed by the enzyme.

In particular embodiments of the invention, the primary structure of a biopolymer molecule with specified biological properties is determined by the characteristics of the glucokinase enzyme, the equilibrium constant and the rate of the biochemical reaction catalyzed by the enzyme.

In particular embodiments of the invention, with a given solvent composition and a given primary structure of a biopolymer molecule consisting of 2 or more subunits, the quaternary spatial structure of a macromolecule is determined as follows:
- determine the amino terminal groups of the protein biopolymer molecule, which separate the polypeptide chains of subunits forming the primary structure of the protein biopolymer molecule;
- for each subunit, determine the structure of the elementary chain of interaction characterizing the interaction of the polymer chain with the solvent; for this, the substrate molecule is determined and a monovalent ion in the solvent composition is identified for each subunit;
- for each subunit, calculate the equilibrium constant, activation energy and determine the volume of the three-dimensional domain;
- determine the most probable three-dimensional spatial configuration of domains corresponding to subunits.

In particular embodiments of the invention, the biopolymer molecule, the primary structure of which consists of 2 or more subunits, is an oligomeric protein.

The present invention also includes a biopolymer molecule with a heterophase structure, which is a biosensor with selectivity to a given substrate molecule and obtained by the method of the invention, showing activity in an aqueous medium.

The present invention also includes a biopolymer molecule with a heterophase structure, having selectivity to a given biological molecule of the substrate S, which is a biological catalyst for catalyzing a biochemical reaction involving a substrate S molecule, obtained by the method of the invention. A biopolymer molecule with a heterophase structure, having selectivity to a given substrate S molecule, which is an enzyme for catalyzing a biochemical reaction involving a substrate S molecule, obtained by the method of the invention.

In particular embodiments of the invention, the biopolymer molecule is characterized by specificity to the glucose molecule, as well as the by the following:
- a biopolymer molecule is a copolymer, which is a polysaccharide of hyaluronic acid;
- the components of a solvent with a given acidity are simultaneously sodium and potassium ions, and a glucose molecule.

In particular embodiments of the invention, the biopolymer molecule is characterized by specificity to the glucose molecule, and differs in that the biopolymer molecule is a polypeptide chain, the repeating monomer of which contains an amino acid with a negatively charged side group, which is selected from a group consisting of glutamic acid, aspartic acid, glycine, cysteine, tyrosine, while an amino acid with an uncharged side group is selected from a group containing serine, asparagine, threonine, glutamine.

In particular embodiments of the invention, the amino acid with a negative side group charge is the glutamic acid; the amino acid with an uncharged side group is asparagine; the substrate of the biochemical reaction is D-glucose.

In particular embodiments of the invention, the amino acid with a negative side group charge is the aspartic acid; the amino acid with an uncharged side group is the amino acid that is selected from the group of asparagine, threonine, cysteine, serine; the substrate of the biochemical reaction is D-glucose.

In particular embodiments of the invention, the polypeptide chain additionally contains amino acids with positively charged side groups, which are selected from the group of lysine, arginine, histidine.

In particular embodiments of the invention, the solvent additionally contains a divalent metal ion, which is selected from a group including magnesium ion Mg⁺², manganese ion Mn⁺², ions of other metals and compounds or another compound.

In particular embodiments of the invention, the polypeptide chain of the first subunit contains an amino acid with a negatively charged side residue group, and the polypeptide chain of the second subunit contains an amino acid with a positively charged side residue group.

In particular embodiments of the invention, the amino acids of the active center of the protein are serine, the side residue of which has a negatively charged group, and histidine, the side residue of which has a positively charged group.

In particular embodiments of the invention, a biopolymer molecule is a molecule for creating a biopolymer substance or a pharmaceutical composition based on hyaluronic acid for the treatment of wounds and tissue inflammation.

In particular embodiments of the invention, a biopolymer molecule is a molecule for creating a drug or pharmaceutical composition for the treatment of diabetes mellitus.

### BRIEF DESCRIPTION OF THE DRAWINGS

The accompanying drawings, which are incorporated in and constitute a part of this specification, illustrate embodiments of the inventive group and, together with a general disclosure of the group of the invention and the following detailed disclosure of the embodiments, serve to explain the principles of the present invention.
Figure 1 presents the spatial (tertiary) structure of a globular protein.
Figure 2 shows a repeating disaccharide monomer of the hyaluronic acid polymer chain.
Figure 3a shows a graph of the dependence of the osmotic pressure of the intercellular substance of the human epidermis on the power of incident infrared thermal radiation (with a maximum spectral power at a wavelength of λ ≈ 10 microns).
Figure 3b shows a graph of the dependence of the osmotic pressure of the intercellular substance of the human epidermis on the concentration of the blood glucose. The glucose concentration in mmol/L is plotted on the abscissa; the P₀ - Pₒₛₘ function is plotted on the ordinate, where P₀ is a constant, which is determined by individual calibration, Pₒₛₘ is the osmotic pressure of the intercellular substance of the epidermis.
Figure 4 shows a graph of the dependence of an arbitrary substance temperature on the amount of heat introduced into the system (heating curve).
Figure 5 shows the most probable configuration of the combined system (on the left) of polymer chain - solvent, which is realized by a uniform distribution of N/2 adsorbed Na⁺ ions at the nodes of the chain and a uniform distribution of N/2 free Na⁺ ions and N/2 free negatively charged monomers M⁻ in the volume of the macromolecule; the least probable configuration the maximum linear entropy density is presented on the right.

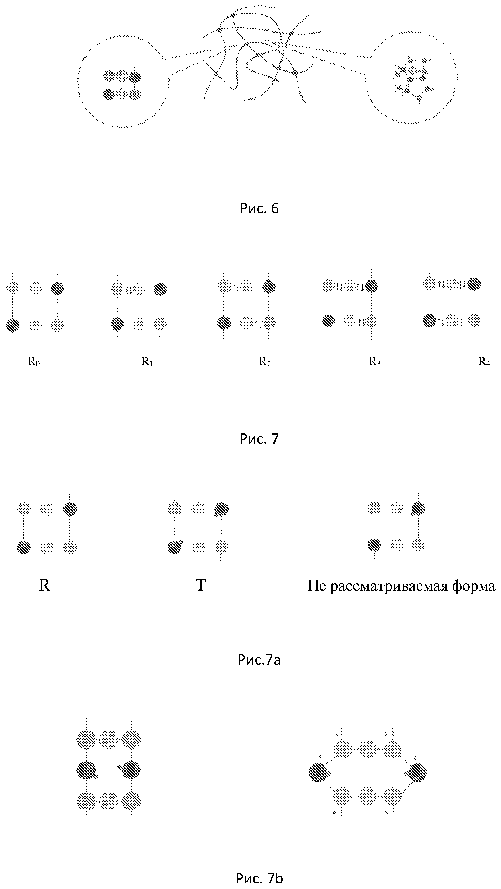

Figure 6 shows the configurations of the polymer network and the network of water hydrogen bonds. Crosslinking of the polymer network by means of hydrogen bonds can be realized in different possible tetramer configurations.
Figure 7 shows the microscopic shapes (G) of the tetramer corresponding to different numbers of hydrogen bonds and discrete values of the binding energy of the tetramer.
Figure 7a shows the configuration (G) of the tetramer in the equilibrium conformations (R) and (T), passing into each other in the process of thermal motion.
Figure 7b shows the equilibrium configuration of the (Na) tetramer, formed from 6 links (1-6) of non-adjacent sections of the chain (1-5-4 and 2-6-3).

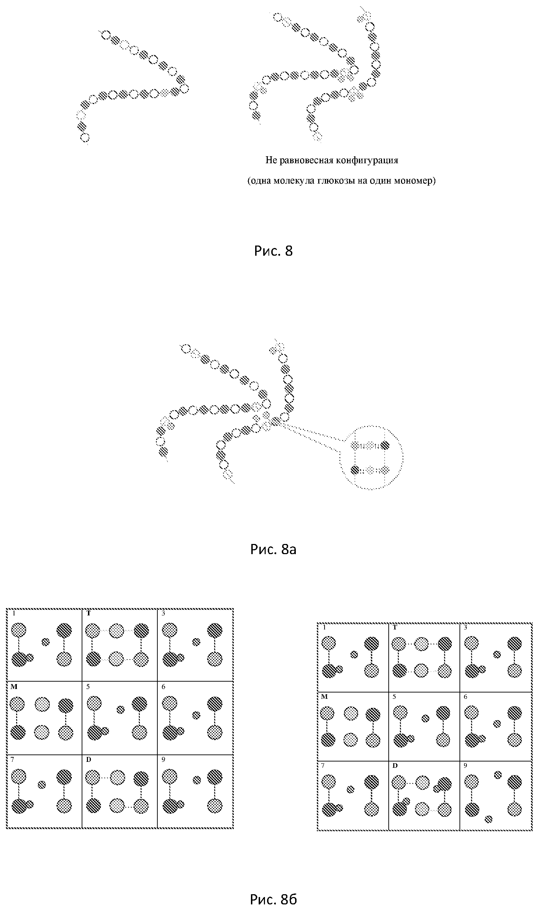

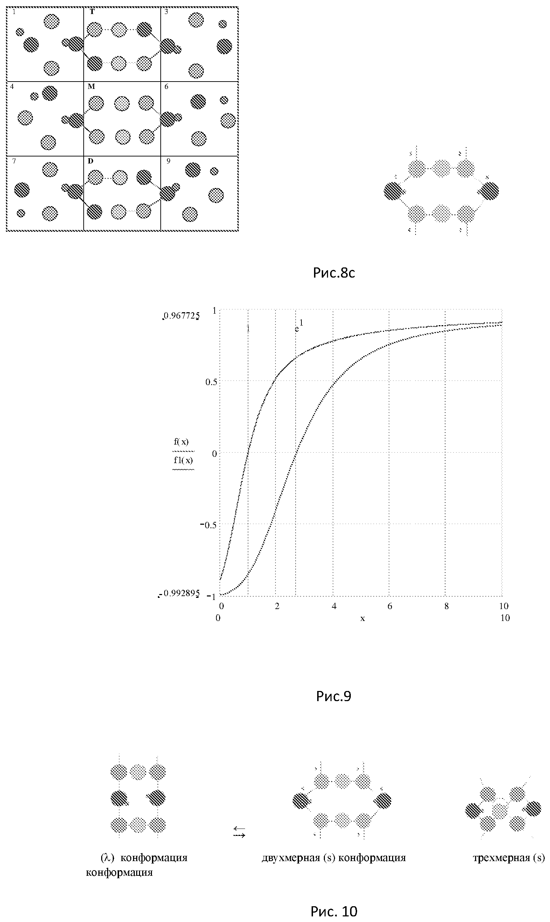

Figure 8 shows the most probable equilibrium configuration of the polymer chain in a solvent with an excess of Na and K ions, at zero glucose concentration: nodes with K ions are shown in yellow, nodes with Na ions are shown in blue.
Figure 8a shows the most probable configuration of interacting elementary chains, which is realized as a result of the pairing of chains by means of hydrogen bonds in the configuration of R₄ (*l* = 4) tetramer;
Figure 8b shows the quantization configuration of the intramolecular space resulting from the volumetric interactions of a pair of elementary chains.
Figure 8c shows a random distribution of equally possible tetramer (Na) configurations in microscopic cells T(R₄), D(R₂), M(R₀), elementary cell: configuration R₄ in cell T (2).
Figure 9 shows the distribution functions Θ(α, γ = 8) and Θ(α, γ =139,740210...), obtained from the universal distribution function Θ(α, γ) describing the dependence of the entropy (free energy of bulk interactions) of the polymer chain, out of the composition of the solvent, for different values of the interaction constant (order parameter) y; the dimensionless glucose concentration α = C/K_{R} is expressed in units of the microscopic equilibrium constant K_{R}.

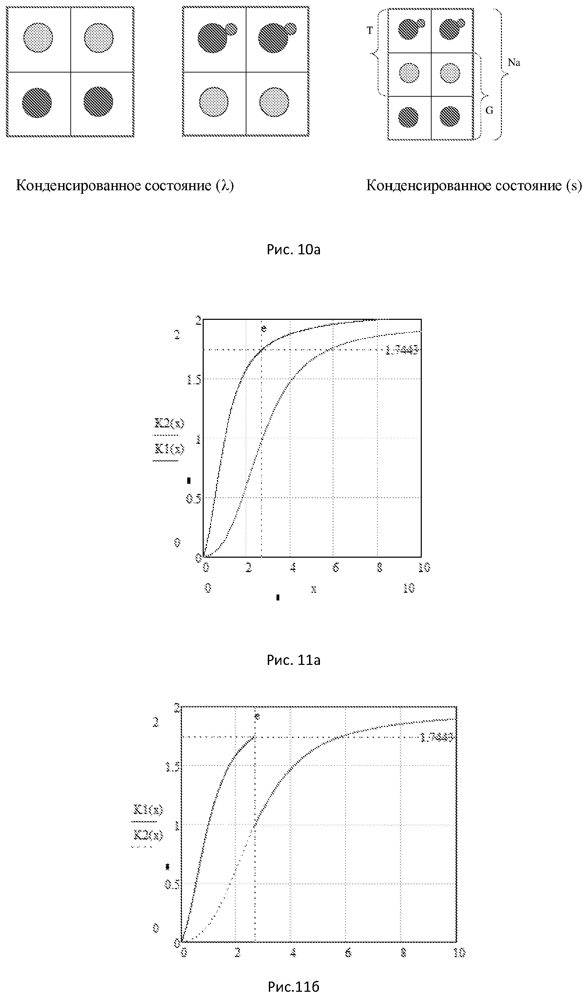

Figure 10 shows the isomeric conformations of the (Na) tetramer formed by hydrogen bonds. On the right, a possible three-dimensional configuration of an isomeric conformation (s) formed from 6 links of a polymer network located on a plane oriented perpendicular to a straight line passing through 2 glucose molecules is schematically shown.
Figure 10a shows the distribution of monomers in the volume of a microscopic cell at concentrations of 2 and 3 monomers/ 1 microcell.
Figure 11a shows a graph of the relative entropy of the polymer chain in the most probable configuration corresponding to the minimum free energy of the elementary bulk interaction for the thermodynamic phases (λ) and (s) of the solvent glucose concentration.
Figure 11b shows a graph of the free energy of volumetric interactions depending on the concentration of glucose.
Figure 12 shows a diagram of the energy levels of tetramers (λ, s, g) of the natural macromolecule polymer network.
Figure 13 shows graphs of the distribution functions (g) of the process:
   Θ_{1g}(α) - green curve; Θ_{2g}(α) - blue curve; Θ_{3g}(α) - red curve.
Figure 14 shows graphs of distribution functions for (a) unpopulated (λ, s, g) tetramers: (λ) tetramer - red curve; (s) tetramer - blue curve; (g) tetramer - purple curve.
Figure 15 shows graphs of the distribution function of dimers and tetramers (λ, s, g): (λ) tetramer - purple Θ_{1λ}(α) and red Θ_{2λ}(α) curves; (g) tetramer - light blue Θ_{1g}(α) and blue Θ_{2g}(α) curves; (s) tetramer - dark green Θ₁ₛ(α) and green Θ₂ₛ(α) curves.
Figure 16 shows graphs of the repulsion energy function of an ideal gas of free monomers in condensed phases (g, λ, s): U_{g rep} (α) - phase G (green); U_{λ rep} (α) - phase λ (red); U_{s rep} (α) - phase s (blue).
Figure 17 shows a graph of the energy function of the elementary bulk interaction (repulsion) *U*_{λ*s rep*} (α) of free monomers in phases (λ , s).

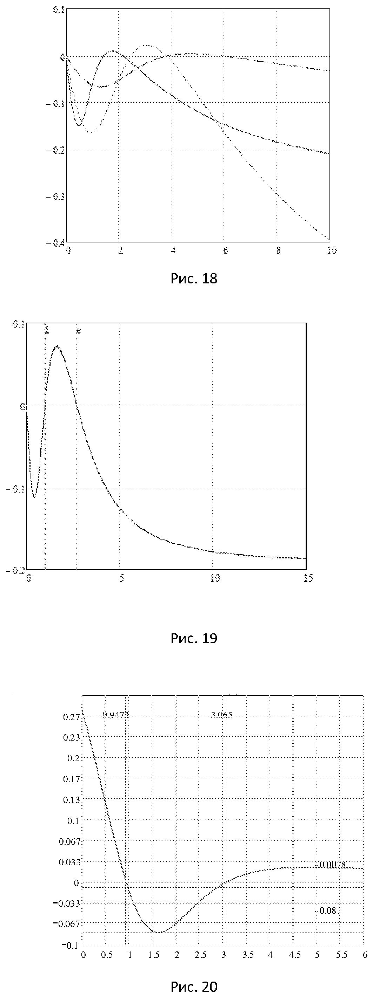

Figure 18 shows graphs of the energy functions of paired interactions and the internal energy of an ideal gas of free monomers for each condensed phase (λ, s, g). The figure shows graphs of 6 functions: 3 functions of the energy of attraction U_{g attr} (α), U_{s attr} (α); U_{λ attr} (α); 3 functions of the energy of repulsion U_{g rep} (α), U _{λ rep} (α), U_{s rep} (α).
Figure 19 shows graphs of the energy of elementary volumetric interaction from the concentration of glucose of the solvent: the repulsion energy is exactly equal to the energy of attraction at any concentrations of glucose of the solvent U_{λs attr} (α) + U_{λs rep} (α) = 0. The figure shows graphs of 2 functions, the attractive energy U_{λs attr} (α) and the repulsive energy U_{λs rep} (α), which are exactly equal at any concentrations of glucose solvent.
Figure 20 shows a graph of osmotic pressure Pₒₛₘ×V_{0g}, depending on the concentration of glucose of the solvent, at a critical value of the microscopic equilibrium constant k_{λ2} = 5,032...
Figure 21 shows graphs of the energy of elementary bulk interactions U_{λs} (α) (red curve) and reversible compression work A (α) (blue curve), performed due to the free chemical energy (hydrogen bonds) of monomers (G) over an ideal gas of free monomers, at a critical value of the microscopic equilibrium constant k_{λ2} = 5,032...
Figure 22 shows a graph of the enthalpy function of a macromolecule from the glucose concentration of the solvent, with a critical value of the equilibrium constant k_{λ2} = 5,032....
Figure 23 shows a graph of the enthalpy isotherm - glucose concentration in the three-phase region of the macromolecule, at a critical value of the microscopic equilibrium constant k_{λ2} = 5,032...., at which gaseous, liquid and crystalline forms can coexist; the gaseous phase is an ideal gas of free monomers of the polymer chain, condensed liquid phase (λ), condensed crystalline phase (s).
Figure 24 shows a diagram of an isothermal phase transition calorimeter.
Figure 25 shows a diagram explaining the principle of an isothermal microcalorimeter of living tissue.
Figure 26 shows a block diagram explaining the principle of operation of the intercellular substance glucose biosensor.
Figure 27 shows a diagram of a spectral device for isothermal calorimetric spectroscopy (ICS) of biochemical components of the living tissue intercellular substance in vivo.
Figure 28 shows the dissociation of the tetramer, as a result of the absorption of a quantum of energy of terahertz electromagnetic radiation, accompanied by the formation of a quantum of thermal energy
Figure 29 shows the scheme of isothermal calorimetric spectroscopy of biochemical components of a living cell.

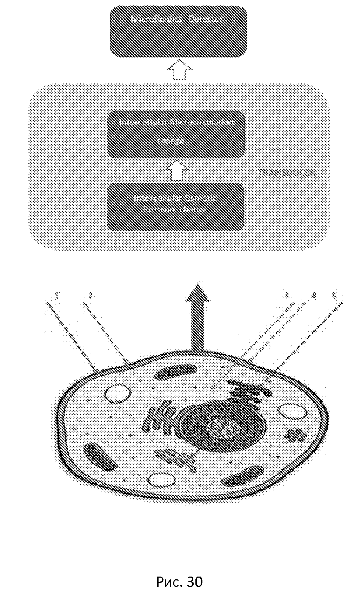

Figure 30 shows the scheme of absorption isothermal calorimetric spectroscopy of a living cell.
Figure 31 shows the absorption spectrum of water in a wide frequency range (from ultraviolet to microwave).
Figure 32 shows the spectra of glucose absorption in the IR region and transmission in the near IR region in the wavelength range from 1,428 microns (7000 cm⁻¹) to 20 microns (500 cm⁻¹).
Figure 33 shows the absorption spectrum of D-glucose in the near IR region for different values of glucose concentration.
Figure 34 schematically shows the structure of an intercellular substance representing a branched macromolecule of hyaluronic acid having the configuration of a polymer network surrounding a living cell.
Figure 35 shows the spectral absorption lines of glucose in the infrared region of the spectrum, corresponding to the characteristic frequencies of the valence bonds of the molecule: C - H; O - H; C = O.
Figure 36 shows the IR spectra of hyaluronic acid, glucose, protein and lipid: 1-triglyceride of fatty acid (tallow); 2-hyaluronic acid; 3- protein (egg albumin); 4- glucose.
Figure 37 shows a fragment of IR spectra: 1 - triglyceride of fatty acid (tallow), 2 - glucose, 3 - hyaluronic acid, 4 - protein (egg albumin).
Figure 38 shows a diffuse reflectance spectroscopy scheme for measuring the content of hyaluronic acid and free fatty acids in the intercellular substance of the surface layer of the living tissue epidermis.
Figure 39 shows the spectrum of hyaluronic acid in the near IR region.
Figure 40 shows the absorption spectra of hyaluronic acid in the near IR region and the transmission spectrum in the IR region.
Figure 41 shows the theoretical absorption spectra of glucose, melanin and water in the red and near infrared range.
Figure 42 shows a schematic representation of the lipid matrix of the stratum corneum.
Figure 43 shows a schematic diagram of a spectral device for implementing a method of spectroscopy of intercellular substance of a living tissue local area.
Figure 44 shows a diffuse reflectance spectroscopy scheme for determining the osmotic pressure of the intercellular substance based on spectral measurement of the temporal dynamics of the amount of water in the stratum corneum at a wavelength corresponding to the characteristic frequency of water (1190, 1455, 1945, 945 nm).
Figure 45 (a, b) shows photos of the working prototype (a) and the proposed design of a commercial device (b).
Figure 46 shows the characteristic abrupt stepwise temporal dynamics of blood glucose level in patient N with type 2 diabetes mellitus (62 years old, female).
Figure 47 shows the abrupt dynamics of blood glucose level in the form of a single pulse, recorded using an isothermal microcalorimeter, and the temporal dynamics of insulin levels in blood, calculated on the basis of a glucose curve.
Figure 48 shows characteristic glucose curves (a, b) with abrupt temporal dynamics in a patient with type 2 diabetes mellitus (66 years old, male): characteristic dynamics of blood glucose level in the form of a time sequence of single pulses.
Figure 49 shows the temporal dynamics of the osmotic pressure of the intercellular substance during the isothermal phase transition of living tissue (a patient with type 2 diabetes mellitus) during the dissolution of the crystalline (globular) phase of the intercellular substance.
Figure 50 shows the characteristic monitoring curves of the osmotic pressure of the intercellular substance of a healthy patient and a patient with type 2 diabetes mellitus.
Figure 51 (a-e) shows a representative glucose curve with stepwise temporary dynamics: Fig. 51 a (test N° 6 patient N° 24), Fig. 51 b (test N° 1 patient N°41), Fig. 51 c (test N°5 patient N°41). Fig. 51 d (test N°6 patient N° 41). All graphs present the results of invasive measurements with green circles, and the results of non-invasive monitoring are shown with a purple curve.
Figure 52 shows the results of tests on 27 patients with type 1 and type 2 diabetes mellitus.
Figure 53 shows the results of tests on 14 patients with type 1 and type 2 diabetes.
Figure 54 shows the results of tests performed on a group of 42 patients (252 tests, 756 invasive measurements) obtained by combining the results of subgroup 1 and subgroup 2.
Figure 55 shows the structural formulas of amino acids.
Figure 56 shows the quaternary structure of proteins consisting of several polypeptide chains resulting from the association of several protein subunits in space.
Figure 57 schematically shows a change in the spatial structure of the protein enzyme hexokinase as a result of binding of a glucose molecule.
Figure 58 shows schematically the main steps of an enzymatic reaction: (a) Diffusion of ATP molecule from liquid phase into crystalline phase; (b) Binding of the phosphate group (red) to the D-glucose molecule (yellow) to form the G6P molecule by a chemical reaction in the crystalline phase of the enzyme; (d) Conformational transition of a tetramer with a bound G6P molecule from the crystalline phase to the liquid phase through the energy barrier separating the liquid phase from the crystalline phase; (g) Disruption of the tetramer in the liquid phase with release of the product molecule G6P.
Figure 59a shows the saturation and rate curves of the enzymatic reaction of glucokinase as a function of glucose concentration.
Figure 59b shows a graph of the energy barrier of glucokinase.
Figure 60a shows the saturation and rate curves of the enzymatic reaction of hexokinase as a function of glucose concentration.
Figure 60b shows a graph of the energy barrier of hexokinase.
Figure 61 shows structural formulas of the glycosidic bond with amino acids.
Figure 62 shows structural formulas of the glycosidic bond in nucleosides.

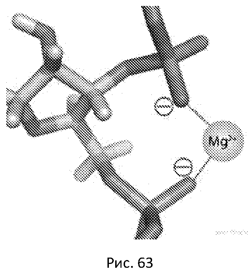

Figure 63 schematically shows the ionic bonds of a divalent metal ion in a macromolecule.

### DETAILED DESCRIPTION OF THE INVENTION

Physical basis of heat exchange of living tissue with the environment.

Heat exchange is a spontaneous and irreversible process of heat transfer caused by temperature gradient. The following forms of heat exchange are distinguished: heat conductivity, convection, radiant heat exchange, heat exchange in phase transitions.

Heat transfer is heat exchange between the body surface and a medium (liquid, gas) contacting therewith.

Evaporative cooling is heat exchange between tissue and the environment caused by evaporation of water delivered to the epidermis from deep tissue layers. Heat flow density is determined by the product of evaporation heat (steam generation heat) by flow density of water evaporating from the surface.

Radiant heat exchange (radiation heat exchange, radiant transfer) is energy transfer from one body to another caused by the processes of emission, propagation, scattering and absorption of electromagnetic radiation. Each of these processes adhere to definite regularities.

Thus, under the conditions of equilibrium heat radiation emission and absorption adhere to the Plank's law of radiation, to the Stephan-Boltzmann law, to the Kirgoff law of radiation.

Essential difference of radiant heat exchange from the other forms of heat exchange (convection, heat conductivity) consists in that it can occur in the absence of a material medium separating surfaces of heat exchange, as electromagnetic radiation also propagates under vacuum.

The Plank's law of radiation establishes relation between radiation intensity, spectral distribution and temperature of the black body. **In** elevation of temperature, radiation energy rises. Radiation energy depends on wavelength. A total energy irradiated by the black body and measurable by a contact-less infrared thermometer is a total energy irradiated at all wavelengths. It is proportional to the Plank's equation integral by wavelengths and it is described in physics by the Stephan-Boltzmann's law.

Radiant heat exchange between tissue surface and the environment is determined by the ratio: ${ΔW}_{R} = β \times \left({{T}_{skin}}^{4}-{{T}_{air}}^{4}\right) = {W}_{0} \times \left[4\times \left({T}_{skin}-{T}_{air}\right)/{T}_{skin}\right] ,$ ΔT << Tₛₖᵢₙ, Tₛₖᵢₙ is skin surface temperature, Tₐᵢᵣ is ambient air temperature. ${W}_{0} = β \times {{T}_{skin}}^{4} .$ ΔW_{R} is heat radiation from tissue surface to the environment.

Heat conductivity is one of the forms of heat transfer from more heated body parts to less heated parts. Heat conductivity results in leveling temperature. In heat conductivity, energy transfer is effected as a result of direct energy transfer from particles having a greater energy to particles with a lower energy. If relative change in T at an average free run distance length of particles is small, then the main heat conductivity law (the Fourier's law) is fulfilled: heat flow density is proportional to temperature gradient ΔT: W_{ΔT} = -λ×ΔT,

Where λ - is heat conductivity coefficient of heat conductivity independent on ΔT. The λ - coefficient depends on aggregate state of a substance, molecular structure, temperature, pressure, composition thereof etc.

Convection is heat transfer in liquids and gases by substance flows. Convection results in leveling substance temperature. In stationary heat delivery to the substance, stationary convection flows occur therein. Intensity of convection depends on difference of temperatures between layers, heat conductivity and viscosity of medium.

Evaporative cooling is heat exchange between tissue and the environment caused by evaporation of water delivered to the epidermis surface from deep tissue layers through intercellular water transfer (by intercellular space). Heat flow density is determined by the product of evaporation heat (steam generation heat) by flow density of water evaporating from the surface.

In a temperature (thermal) neutral zone under normal conditions, water transport by sweating is known to be practically absent and the main contribution into evaporative cooling process is determined by water transport to the body surface. In physiology and medicine, this process is known as insensible (non-perceivable) perspiration.

Intercellular insensible perspiration is observed under so called "thermal comfortable (neutral) conditions":
Ambient temperature: 18-23°C,
Atmospheric pressure: 730-770 mm Hg (millimeters of mercury).

Intensity of evaporative cooling process under thermal comfortable (neutral) conditions is known to make up 400 to 700 mL/day or 10⁻⁸-10⁻⁷ g/second× cm². This corresponds to values of heat flows of 1 to 10 mW/cm².

It is known that the physiological process of intercellular insensible perspiration has a high sensitivity towards the external heat fluxes. Threshold of the process sensitivity to heat fluxes is 0,1 mW/ cm², which is equivalent to sensitivity of the process to ambient temperature equal to 0,005 degrees.

An important consequence of high sensitivity of the physiologic process to an insensible perspiration towards external heat fluxes practical importance is dependence of the physical characteristics of the human skin epidermis on external physical and climatic factors, in particular, on the temperature and humidity of the environment and atmospheric pressure. In particular, the results for measurements of the physiological parameter of human skin epidermis - speed of imperceptible perspiration, with constant values of microclimate of the room, which is used for measurements, are dependent on the physical and climatic factors of the environment (temperature and humidity of the environment, atmospheric pressure). Low- frequency fluctuations in the temperature of the outside air within even one degree, with constant parameters of the microclimate of the room, which is used for measurements, lead to significant low-frequency fluctuations of the measured physiological parameter and, as a result, increase in measurement error.

Physiological and biochemical basis of heat production of human living tissue

The process of oxidation of glucose which is one of the main energy suppliers in the body, occurs in accordance with the equation that may be presented in the following equation:

Glucose + Oxygen ⇒ CO₂ + H₂O.

The change of the standard free energy in this reaction under physiological conditions is equal: $ΔG = - 686 kcal / mol .$

For comparison, a male weighing seventy kilograms who goes upstairs for an hour, expends about 1,000 kcal. From this, it is clear that 686 kcal, mentioned above are a vast amount of energy. Work done by man is of course much less than energy expended during this work as in irreversible process, not all change in free energy is converted into work. Real efficacy of this conversion (as will be described below) is not higher than 40%. Moreover, food is not "burned" immediately in oxygen releasing energy in the form of heat and this release occurs in steps and includes a number of rather complex chemical conversions each of which gives a small "portion" of energy.

Glucose is oxidized in the body forming carbon dioxide and water; this is one of the most universal processes underlying respiration and digestion processes.

In breaking each glucose molecule accompanied by lowering free energy, energy is released that is sufficient to form 93 ATP molecules by binding of phosphate groups to ADP molecules. Not all 93 molecules appear to be actually formed. At the same time, all the process includes a large number of enzymatic reactions. Nutrients (carbohydrates, fatty acids and amino acids) enter into a series of reactions forming the Krebs cycle (or the cycle of tricarboxylic acids) during which carbonic backbone of molecules is broken down with formation of CO₂ but ATP is not formed here. On the following reaction steps transfer of electrons using special enzymes (respiratory chain) occurs. At these steps, ATP is synthesized and the last step on the way of a long process of electron transfer consists in binding thereof to molecular oxygen. Generally, electron transfer process along the respiratory chain resulting in accumulation of energy in ATP molecules is called oxidative phosphorylation. As a result of this process, 38 molecules of ATP as calculated per every consumed glucose molecule are formed. Efficacy of such transformation equals to 38/93=40%.

A value of heat production or heat power of the body can be quantitatively assessed originating from the following simple considerations.

An energetic value of human nutrition is about 2400 kcal/daily. In a first approximation, $2400 kcal = {10}^{4} J , 1 day \left(24 hours\right) = 86400 seconds = {10}^{5} seconds .$

Then energy consumed by human body per one second will be 10⁴/10⁵=0,1 kJ×s⁻¹ or 100 J×s⁻¹ = 100 W;
thus, heat power of a human body is approximately equal to power of an electric bulb having power 100 W.

In muscular contraction, ATP which is energy donor for muscular contraction process, during reaction with myosin, allows for obtaining at most 50 J×g⁻¹ energy. This means that an ideal muscular system (i.e. with efficiency equal to 100%) for lifting a load weighing 1 kg to a 5 m height, would require expenditure of 2×10⁻³ mole ATP. Actually, muscular efficiency is about 30-40% and the rest portion is released in the form of heat.

Under normal conditions of the body's vital activity, glucose is a main energetic substrate. Normal human blood plasma glucose concentration depending upon nutrition conditions is maintained within the limits of 50 to 120 mg %. Postprandial glucose concentration in the portal vein system during absorption phase can achieve more than 270 mg %. Elevation of blood glucose level always causes increase in insulin secretion.

In resting human body, fasting glucose metabolism rate averages 140 mg/hour per 1 kg body mass, 50% glucose being consumed by the brain, 20% by muscles, 20% by red blood cells and kidneys, and only 10% glucose are left for the rest tissues.

Glucose utilization rate (metabolism rate) in healthy man is a linear function of blood plasma glucose concentration. A mathematical relationship between glucose utilization and blood concentration thereof in normal humans is expressed by the equation: ${R}_{u} = 0.02554 C + 0.0785 ,$

And in patients with non-ketotic diabetes: ${R}_{u} = 0.004448 C + 2.006 ,$ where Rᵤ is glucose utilization rate, mg/min per 1 kg of body weight, and C - blood plasma glucose concentration, mg %.

The term glucose "utilization" in physiological sense means the rate of glucose transport from blood into a general fund of tissue glucose and exit from it during metabolism. From biochemical point of view, glucose utilization rate is determined by transport through cytoplasmic membrane and by intracellular oxidative phosphorylation of glucose. The terms "turnover rate", "assimilation" and "consumption" of glucose which are widely spread in the literature are synonyms of the notion glucose "utilization" and they are in any respect equivalent.

Under physiologic conditions, practically in all tissues glucose transport from intercellular medium into a cell is a first limiting reaction in glucose utilization by cells as in the absence of insulin, flow of transportable glucose is always less than glucose phosphorylation rate. Equilibrium between glucose transport and phosphorylation rates is achieved only at high glucose concentrations (400 to 500 mg %). In further increase in glucose concentration, phosphorylation becomes a limiting reaction. In other words, glucose transport rate from intercellular medium through cytoplasmic membrane into intracellular medium is a process limiting glucose utilization rate by a live tissue.

Originating from the above consideration, it appears logical and completely reasoned to draw a conclusion that heat generation as well as glucose utilization rate is a linear function of blood glucose concentration and measurement of local heat generation value allows for determining blood glucose level.

### Condensed matter physics of the biopolymer molecule.

The object of the present study, performed within the work on the group of inventions, is biopolymer molecules capable of spatial self-organization and acquisition of stable molecular structure due to weak intramolecular volume interactions of non-valent nature.

Examples of biopolymer molecules capable of spatial self-organization under physiological conditions are polysaccharides and protein molecules with a polypeptide chain.

The behaviour of proteins is entirely determined by an exceptional, intrinsic spatial structural organization, which is not due to valence interactions and is determined exclusively by weak volumetric interactions. Deprived of it, proteins lose all their biological properties.

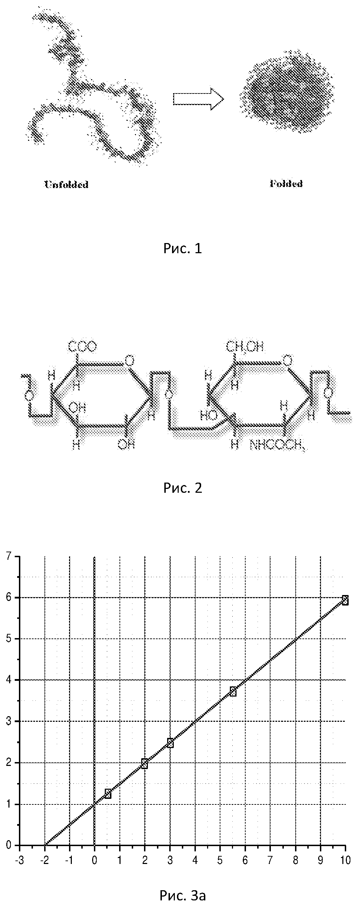

Protein chains are capable of spontaneously folding into strictly deterministic structures which geometry and conformational dynamics under physiological (native) conditions are completely determined by the amino acid sequence. The three-dimensional structures of proteins are individualized, very complex and have a strict order, not reducible, however, to periodicity. Fig. 1 schematically shows the process of self-organization (folding) of a biopolymer molecule that occurs spontaneously as a phase transition of a statistical system of many interacting particles into a spatial configuration with a heterophase structure with minimal free energy. The ability of the natural polypeptide chain to spatially self-organize and to acquire a specific molecular structure is the most striking feature of proteins, which is absent in artificial polymer molecules, including human-made poly-amino acids.

Hence, by this the understanding of the extreme complexity of the task of experimental in vitro investigation of the biopolymer molecules properties is explained. Many experimental methods to study the substance structure, effective in the studies of molecules (which structure is determined by covalent bonds), are of limited applicability for the study of biopolymer molecules, the spatial structure of which is determined by weak non-valent interactions.

Modern methods enable determination of the primary structure of biopolymer molecules, consisting of a sequence of monomers linked by strong covalent bonds, while the determination of the spatial structure of such molecules still remains a challenging problem. For example, the number of sequences (primary structure) of protein molecules in the UniPare database (as of August 2016) was over 124 million, while the number of structures in the PDB (Protein Data Bank) database was only just over 121 thousand, representing less than 0.1% of all known sequences, with the gap between the two increasing rapidly and likely to grow further. This strong lag is due to the relative complexity of current methods of structure determination.

Another side of the problem is the lack of understanding of the fundamental nature of weak non-valent intramolecular interactions. By the moment of the present study, humanity with all its computational power and arsenal of experimental data still did not learn to build models that would describe protein folding process and predict the three-dimensional structure of protein on the basis of its primary structure (that is amino acid sequence) that directly indicates the lack of understanding of fundamental nature of weak bulk interactions determining biological properties of biopolymer molecules.

Thus, the spatial structure of biopolymer molecule and its relation to biological function is one of the biggest unsolved problems of modern science, the solution of which is possible under the condition of progress in fundamental studies of physical and chemical nature of weak volume interactions, which determine the three-dimensional structure of biopolymer molecule.

It should be noted that solving the folding problem is directly related to the scientific problem of biological catalysis, a unique phenomenon which physicochemical nature remains unclear.

Hence, the conclusion that solving the problem of folding and biosynthesis is directly connected with the development of new physical and chemical methods for in vivo studies of biopolymer molecules and the possibility of making real progress in the field of condensed matter physics of biopolymer structures.

In the present study, this scientific problem was solved by combining an experimental study that was carried out using a unique experimental method based on noninvasive measurements of the intercellular substance characteristics under in vivo conditions, and a theoretical study of the condensed state of a biopolymer molecule that was performed on the basis of statistical physics methods to generalize the obtained experimental data.

### Biosensor based on biopolymer molecule with heterophase structure.

A biopolymer molecule of hyaluronic acid polysaccharide turned out to be a unique model object for studies in the field of condensed state physics of biopolymer molecules capable of self-organization of spatial structure. Its choice as an object of study was conditioned by the following considerations:
Hyaluronic acid high molecular weight polysaccharide is the main component of the intercellular substance surrounding a living cell, which plays an important role in the self-organization of physical and chemical processes of cellular metabolism, the most important of which is the process of physical thermoregulation of living tissue, maintaining temperature constancy. As it has been known from experience, all mammalian organisms, which main intercellular component is the biopolymer molecule hyaluronic acid, which structure has remained unchanged during the long biological evolution, have a close body temperature (36- 38°C), which directly indicates the heterophase state of intercellular substance, caused by the thermodynamic properties of the biopolymer molecule of hyaluronic acid.

Experimental study of the thermodynamic properties of hyaluronic acid biopolymer can be performed under in vivo conditions based on non-invasive measurements of the characteristics of human epidermal intercellular substance using sensors placed on the skin epidermis surface.

A biopolymer molecule of hyaluronic acid has the simplest primary structure shown on Fig. 2, consisting of the same repeating monomers (disaccharides), consisting of 2 types of sugar residues (links), in contrast to more complex primary structure of globular proteins (consisting of 20 types of links), DNA and RNA (consisting of 4 types of links), so it is a unique model object of biopolymer molecule for theoretical study.

In order to study the thermodynamic properties of the intercellular substance, in vivo experimental studies were carried out in which the dependence of the osmotic pressure of the intercellular substance of human living tissue epidermis, characterizing the thermodynamic phase state of the intercellular substance in the local area under the heatproof applicator (applied with dosed pressure to the skin surface and forming an enclosed living tissue system in the local area under the applicator), on the external heat flow, external pressure and blood glucose concentration.

Experimental studies of the dependence of intercellular osmotic pressure on the power of external electromagnetic radiation fluxes falling on the surface of human skin epidermis were carried out in a wide range of frequencies from optical to microwave: optical and near infrared radiation 0.4 - 0.9 µm; infrared radiation 1-10 µm; microwave radiation 5-10 mm.

Figure 3a presents the experimental dependence of the osmotic pressure of the intercellular substance of the epidermis, which was measured noninvasively in real time, on the power of the external infrared incident heat radiation fluxes (with a maximum spectral power at a wavelength of λ ≈ 10 µm) at a constant blood glucose level (in the time interval of the experiment). In one experiment with one patient (with a fixed location of the sensor on the surface of the epidermis), 5 measurements were performed corresponding to different values of the influencing power (in mW/cm²: 10; 5.5; 3; 2; 0.5. The threshold sensitivity of the intercellular substance is limited by intrinsic fluctuations in the osmotic pressure of the intercellular substance and is < 100 µW/cm² for observation times of 10²s; the resulting sensitivity threshold is equivalent to an ambient temperature difference of 0.08 °C.

It has been experimentally proved that the process of heat exchange between the local area of living tissue epidermis and the environment is reversible and isothermal: reversible changes of incident thermal radiation power in the experiment lead to reversible isothermal changes of osmotic pressure in real time.

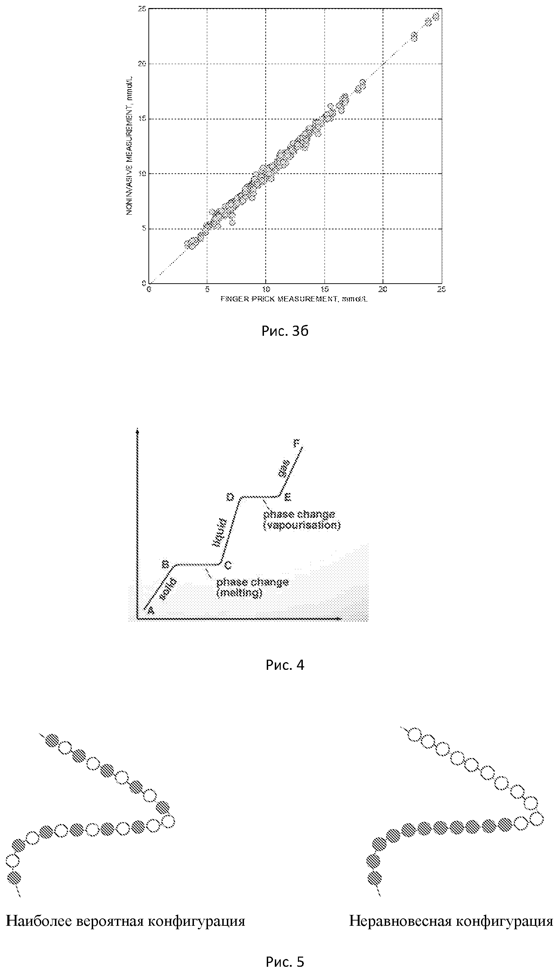

As it is known, thermodynamic reversibility and isothermal character of the substance heat exchange with the environment are fundamental properties of the real substance, which appear in the triple point, in which the substance is in the heterophase state. Figure 4 shows the graph of temperature dependence of an arbitrary substance on the amount of heat introduced into the system (heating curve); there are areas of constant temperature near phase transitions in which the substance is in heterophase state, which are due to the fact that in the process of substance melting the amount of heat equal to the latent heat of melting (or vaporization) ΔH is absorbed by the system without temperature change, but with a change in entropy ΔH/T = ΔS; the effective heat capacity of the substance in the heterophase state is equal to infinity.

The example of a system in which thermodynamic equilibrium between three phases of a substance is realized is a mixture of water, ice and steam at a triple point. It is well-known that in a mixture of water and ice at 273.16 K, two phases are in equilibrium, and melting and freezing processes flow reversibly at constant entropy: when the mixture is heated, ice melts, and when cooled, on the contrary, crystallization occurs - the formation of ice from the liquid phase, while the temperature of the water remains constant (isothermal property).

Thus, the results of experiments on measuring the threshold of sensitivity of intercellular substance to heat flows based on noninvasive measurement of osmotic pressure of intercellular substance directly prove the conclusion about the heterophase structure of intercellular substance of living tissue, at physiological body temperature of 36.6 °C. Hence, the understanding of the generalization that the same temperature of warm-blooded organisms is a consequence of the heterophase state of the warm-blooded organism's intercellular substance, which is a fundamental property of the intercellular substance of these organisms due to the thermodynamic properties of the biopolymer molecule of hyaluronic acid.

**Selectivity of hyaluronic acid glucose molecule.** The peculiarity of the biopolymer molecule of hyaluronic acid with heterophase structure is that along with extremely high sensitivity to heat fluxes, the molecule has selectivity to the glucose molecule due to binding by formation of hydrogen bonds between the solvent glucose molecule and polymer chain monomers. In order to investigate the selectivity of the biopolymer molecule of hyaluronic acid to the glucose molecule, experimental studies of the dependence of the osmotic pressure of the epidermis intercellular substance on the blood glucose concentration were carried out.

Fig. 36 shows the dependence of intercellular substance osmotic pressure on blood glucose concentration obtained experimentally in a clinical study in 50 patients. As follows from the presented graph, the inverse value of intercellular osmotic pressure depends linearly on the blood sugar concentration in the concentration range of 3 mmol/L to 25 mmol/L. The conditions of the experiment are described in detail in the section "Practical application".

The property of thermodynamic reversibility of intercellular substance transitions from one state to another has been experimentally proved - reversible changes in blood glucose concentration and, as a consequence, metabolic rate of living tissue leads to reversible changes in osmotic pressure of the intercellular substance: reversible physiological changes in the concentration of glucose in blood lead to reversible changes in osmotic pressure, similar to reversible changes in osmotic pressure in the process of heat exchange of living tissue with the environment - a decrease in glucose concentration leads to an increase in osmotic pressure, and on the contrary, an increase in the concentration of glucose in blood leads to a decrease in osmotic pressure - a response parameter similar to response to the substance cooling.

The property of thermodynamic reversibility of substance transitions from one state to another is a fundamental property of a real substance in a heterophase state at a triple point, therefore, the property of thermodynamic reversibility of the intercellular substance of living tissue, discovered experimentally, is a direct proof of the heterophase state of the intercellular substance under physiological conditions.

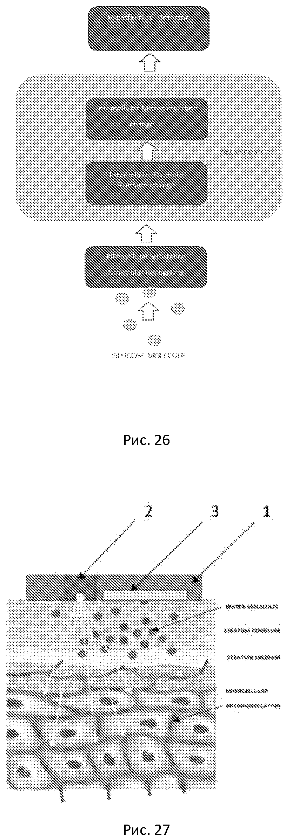

The intercellular substance of living tissue, which properties are determined by the thermodynamic properties of a biopolymer molecule of hyaluronic acid with a heterophase structure, is thus a natural glucose biosensor consisting of two elements - a recognition element (molecular recogniser) and a transducer (transducer), which converts changes in glucose concentration into changes in the osmotic pressure of the intercellular substance. Figure 26 shows a block diagram explaining the principle of operation of the glucose biosensor of the intercellular substance.

Thus, the results of experimental studies in vivo prove the unique biosensory properties of the intercellular substance of living tissue due to the heterophase structure of the biopolymer molecule of hyaluronic acid - the intercellular substance is a unique natural biosensor with the property of thermodynamic reversibility and bioselectivity to the glucose molecule and extremely high sensitivity to external heat flows.

The theoretical study was carried out in order to clarify the physical and chemical mechanism of selectivity of the biopolymer molecule of hyaluronic acid to glucose and the statistical mechanism of the heterophase condensed state of the combined system of the biopolymer molecule with a solvent near the boundary of the phase transition on the basis of generalization of experimental results. The description of the statistical method and the main results of the study are given below.

The property of thermodynamic reversibility and selectivity is united the polysaccharide molecule of hyaluronic acid with protein molecules, in particular, with enzymes that have a unique selectivity to certain substrate molecules and the property to catalyze biochemical reactions involving these molecules. Therefore, a conclusion is understood that the property of the biological specificity of enzymes, that was confirmed by the results of a large number of biochemical studies, and the selectivity of hyaluronic acid to glucose have a single physical and chemical basis.

Within the framework of theoretical study, a generalization for enzyme molecules was performed using a statistical method, which enabled to obtain solutions for the thermodynamic phases of the substance within the macromolecules. For this purpose, a theoretical study of the physicochemical mechanism of enzymes was carried out using hexokinase as reference enzymes, as they show selectivity to the glucose molecule and catalyze a biochemical reaction involving glucose. The obtained data confirmed heterogeneous statistical mechanism of the enzymatic catalysis.

In this document, the term "substrate S" refers to a solvent molecule that interacts with the monomers of the polymer chain through the formation of a weak non-valent bond. **In** particular, according to the invention, the solvent is an aqueous solution containing sodium, chlorine, potassium ions and glucose, and the difference between sodium and chlorine concentrations is approximately 40 mmol/L (excess sodium ions), and the glucose content in the heterophase range can vary over a wide range from 3 to 15 mmol/L.

In this document, the term "monomer" refers to the repeating links (structural units) in the biopolymer molecular chain. An example of monomer A is D-glucuronic acid containing a negatively charged group, an example of monomer B is O-Acetylglucosamine, neutrally charged, forming a repeating disaccharide in the hyaluronic acid polymer chain. In case of a polymer chain of a protein molecule, examples of monomers A are negatively charged glutamic and aspartic acids, and positively charged lysine, arginine, histidine; examples of monomers B are uncharged serine, threonine, glutamine, and asparagine.

In this document, the term "macromolecule with a heterophase structure" means ... the only biopolymer molecule in which, under certain conditions, several thermodynamic phases - crystalline phase (globule), liquid (molten globule), gaseous (coil) - may coexist.

In this document, the term macromolecule with "specified biological properties
and a specified spatial structure" refers to a biopolymer molecule that has a uniquely defined biochemical characteristics (equilibrium constant, biochemical reaction rate) and a uniquely defined three-dimensional spatial structure.

In this document, the term "solvent" means a substance capable of dissolving other solid, liquid, or gaseous substances without causing their chemical changes. Usually, at ambient pressure and room temperature, the solvent is a liquid substance. Examples are water, sea water, glucose solution.

In this document, the term "subunit" means biopolymer
(polypeptide) chain, which together with other components is assembled into a multi- or oligodimensional protein complex. Many natural enzymes and other proteins consist of several protein subunits.

In this document, the term "biological catalyst with specified characteristics" refers to a catalyst with specific rate constant and rate of the biochemical reaction which it catalyzes.

In this document, the term "enzyme with specified characteristics" refers to a protein macromolecule with a uniquely defined three-dimensional spatial structure and specific values of the rate constant and the rate of the biochemical reaction catalyzed by this enzyme.

In this document, the term "biosensor selective to the specified substrate molecule" refers to a biopolymer molecule with selectivity (specificity) to a particular solvent molecule.

### Non-equilibrium thermodynamics of the biopolymer molecule in a condensed state.

### Isothermal microcalorimeter of living tissue.

As has been shown isothermy in warm-blooded animals - the relative stability of temperature, at reversible physiological changes in the concentration of glucose in blood (which determines cellular metabolism rate and the intensity of heat production) and the thermal balance between the living tissue and the environment in the field of physical thermoregulation of the body, has a physical nature and is based on the thermodynamic properties of the intercellular substance of a local microvolume of the living t issue. The intercellular substance, in the physiological normal range, is in a heterophase condensed state, in which the crystalline solid phase (globule) is in the thermodynamic equilibrium with the metastable liquid phase (molten globule) and the gaseous phase (coil). As a result of the impact of the ΔWᵢₙₚᵤₜ external heat flux on the local epidermis surface area, the heat introduced into the local volume of the substance leads to the melting of the solid phase - the phase transition of the substance from the solid state ("globule") to the liquid state ("molten globule"); the melting process of the globular phase of hyaluronic acid proceeds as a first-order phase transition with a change in the volume of the substance (which has changed the physical state) due to a change in the osmotic pressure of the substance. A change in the osmotic pressure of the intercellular substance (the amount of water in the intercellular space) as a result of melting of the globular phase, leads to a change in the intercellular microcirculation intensity - microhydrodynamics (microfluidics) directed towards the surface of the epidermis, which enables isothermal heat exchange between the tissue and the environment during evaporative cooling with an intensity Wₒᵤₜ; the heat balance of a reversible isothermal process, at constant entropy, has the following form: ${ΔW}_{input} = {ΔW}_{out} .$

The physical analogue of a system in which thermodynamic equilibrium between three phases of a substance is realized is a mixture of water, ice and steam at a triple point. It is well-known [12], in a mixture of water and ice at 273.16 K, two phases are in equilibrium, and melting and freezing processes flow reversibly at constant entropy: when the mixture is heated, ice melts, and when cooled, on the contrary, crystallization occurs - the formation of ice from the liquid phase, while the temperature of the water remains constant (isothermal property).

The process of melting of a substance at the phase transition temperature occurs under isothermal conditions, without a change in temperature; the amount of heat introduced into the system can be determined by measuring the amount of a substance that has transferred into a liquid state during the melting process (isothermal calorimetry - measuring the amount of heat introduced into the volume of a substance at the phase transition temperature). The principle of isothermal calorimetry is used in practice in a well-known device - isothermal phase transition calorimeter, in which the introduced heat does not change the temperature of the calorimetric system, but causes a change in the physical state of a substance that is part of this system (for example, the melting of ice in the Bunsen ice calorimeter).

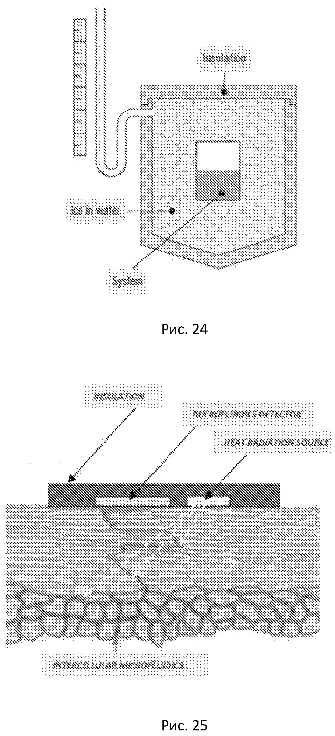

In the process of melting a substance, an amount of heat equal to the latent heat of melting ΔH is absorbed by the system without a change in the temperature, but with a change in entropy ΔH/T = ΔS. In this case the amount of heat introduced is calculated according to the mass of the substance that have changed the state of aggregation (for example, the mass of melted ice, which can be measured by the change in the volume of the mixture of ice and water), and the heat of the phase transition. The principle of action of the ice calorimeter is based on the fact that the amount of heat introduced into the calorimeter is measured not according to the heating temperature, as it occurs in the variable-temperature calorimeter, but according to the flow rate of a mixture of water at ice melting temperature. The diagram of an isothermal ice calorimeter is shown in Fig. 24. The amount of heat introduced is calculated according to the mass of the melted ice, which can be measured by the change in the volume of the mixture of ice and water and the heat of phase transition.

The principle of operation of the isothermal calorimeter of the living tissue is similar to that of the ice calorimeter and is based on the fact that the amount of heat introduced into a local volume of living tissue or formed in this volume during cellular metabolism is measured not by a change in the temperature of the living tissue, as in the variable-temperature calorimeter, but by the change in the volume of the intercellular substance, determined by the volume of the mixture of the globule (crystalline phase), molten globule (liquid phase), at the melting point of the hyaluronic acid globule. Changes in the volume of the intercellular substance can be determined by measuring the osmotic pressure of the intercellular substance. A diagram explaining the principle of an isothermal microcalorimeter of the living tissue is shown in Fig. 25. The change in the volume of the intercellular substance during the melting of the crystalline (globular) phase of the hyaluronic acid caused by the absorption of heat is determined by measuring the osmotic pressure of the intercellular substance.

Intercellular hyaluronic acid biopolymer of the living tissue is a natural isothermal biosensor with high sensitivity to thermal energy due to the low binding energy of ≈ 3,5 k_{B}T of the tetramer molecular complex: thermal energy (amount of heat) introduced into the volume of the substance causes isothermal melting of the crystalline phase with the absorption of energy, and, as a result, causes a change in the osmotic pressure of the intercellular substance, which can be measured using a sensor located on the surface of the epidermis. As it was shown above, the conformational transitions of the Na-tetramer from conformation (s) of the crystalline phase to conformation (λ) of the liquid phase may occur only with the absorption of an energy quantum of 1k_{B}T per tetramer. The amount of heat that must be transferred to one unit of mass of a crystalline substance in an equilibrium isobaric-isothermal process in order to transfer it from solid (crystalline) to liquid state (the same amount of heat is released during the crystallization of a substance) is defined as the specific heat of melting (enthalpy of melting).

Experimental studies were carried out to investigate the correlation between intercellular osmotic pressure and the power of external electromagnetic radiation fluxes acting on the surface of the human epidermis in a wide frequency range from optical spectrum to microwave: optical and near infrared radiation 0.4 - 0.9 µm; infrared radiation 1-10 microns; microwave radiation 5-10 mm. Figure 3a shows experimental correlation between the osmotic pressure of the intercellular substance and the power of infrared thermal radiation fluxe acting on the surface of the epidermis at a constant blood glucose concentration (in the 30-60 minutes interval within the experiment, there were virtually no changes in the blood glucose level). The principle of operation of an isothermal calorimeter of the living tissue is based on the fact that the amount of heat introduced into the calorimeter is measured not by a change in temperature, as in the variable temperature calorimeters, but by a change in the volume of an intercellular substance consisting of a mixture of crystalline (globule) and liquid (molten globule) phases, at the crystalline globular phase melting temperature.

Figure 3a shows experimental correlation between the osmotic pressure of the intercellular substance of the epidermis and the power of external infrared thermal radiation flux (with a maximum spectral power at a wavelength of ~ 10 µm) at a constant blood glucose concentration (within the experimental time interval). In one experiment with one patient (with a fixed location of the sensor on the surface of the epidermis), 5 measurements were performed corresponding to different values of the influencing power (in mW/cm²): 10; 5.5; 3; 2; 0.5. The threshold sensitivity of the calorimetric spectrometer is limited by the intrinsic fluctuations in the osmotic pressure of the intercellular substance and is < 100 µW/cm² for 10²s observation time.

The intercellular fluid flow rate through the epidermis (mass transfer rate) is determined by the osmotic pressure gradient of the intercellular substance. The slope in Figure 3a corresponds to the epidermal mass transfer coefficient, the value of which is determined by the blood glucose concentration, an increase in which leads to an increase in the proportion of the crystalline phase of the substance in the intercellular space. Blood glucose concentration can be determined by the slope of the graph of osmotic pressure of the intercellular substance of the epidermis vs power of the external thermal radiation; the slope is determined by the glucose concentration in the intercellular fluid (in blood).

**Selectivity of intercellular substance to glucose.** A specific feature of the isothermal microcalorimeter of living tissue is that the intercellular substance of the living tissue, along with extremely high sensitivity to heat flow, is selective towards the glucose molecule, the concentration of which determines the concentration of tetramers (crosslinks) of the hyaluronic acid polymer chain and, as a result, determines the ratio between crystalline (globule) and liquid (molten globule) thermodynamic phases. An increase in glucose concentration in the liquid phase of the intercellular substance leads to its crystallization, which is accompanied by a decrease in volume and osmotic pressure; a decrease in glucose concentration leads to the reverse process - dissolution, which is accompanied by an increase in volume and osmotic pressure. Therefore, an increase in the concentration of glucose in a substance leads to a response similar to that in case of cooling.

The intercellular substance of the living tissue is a glucose biosensor, which consists of two elements - a molecular recognizer (tetramer) and a transducer, which converts changes in glucose concentration into changes in the osmotic pressure of the intercellular substance. Figure 26 shows a block diagram explaining the principle of operation of the glucose biosensor of the intercellular substance.

The substance of the living tissue is a natural glucose biosensor, which consists of

two elements: a molecular recognizer, the role of which is played by the crystalline phase of the macromolecule, and a transducer, the role of which is played by the liquid phase, which converts changes in glucose concentration into changes in the osmotic pressure of the intercellular substance. These changes are recognized using a sensor located on the epidermis surface.

**Caloric equation.** The caloric state equation, which relates the variables of the thermodynamic state of living tissue in a local volume under heat- and waterproof applicator in an isothermal calorimeter, is based on the fundamental equation of thermodynamics in the following way.

The fundamental equation of thermodynamics, which describes the reversible quasi-static changes in the state of a closed system under the applicator in the time interval Δt = τ, equal to the time constant of the transient process, can be transformed and represented as an equation describing the reversible changes in the system in the time interval Δt = dt →0, where flow rate can be considered constant: $\begin{matrix}ΔH \times dt / τ = \left({W}_{MET}\right) \times dt + \left({W}_{ΔT}\right) \times dt + {K}_{M} \times \left(ΔM/Δt\right) \times dt + {K}_{p} \times Δp \times dt = \\ = \left({W}_{MET}\right) \times dt + {K}_{ΔT} \times \left({ΔT}_{skin}\times /Δt\right) \times dt + {K}_{M} \times \left(ΔM/Δt\right) \times dt - {K}_{p} \times Δp \times dt\end{matrix}$

In the equation (44a), which relates the flows of coupled processes of energy and substance transfer, which are measured in a real experiment, the following definitions are used:
W_{MET} - the power of the heat generation process, which is based on the required value
ΔQ_{MET} = ∫(W_{MET})dt with integration within the range from 0 to τ.
W_{ΔT} - power of the process of transfer of the heat entering the system from the deep layer to surface layer, due to the temperature difference ΔTₛₖᵢₙ = T_{deep} - T_{surface},
which is determined by the requirement ΔQ_{ΔT} = ∫(W_{ΔT}) dt = K_{ΔT}×∫(ΔTₛₖᵢₙ×/Δt) dt with integration within the range between 0 and τ.

K_{M}×(ΔM/Δt) = J - the power of the mass transfer process entering the system from the deep layer, due to the difference in the amount of substance ΔM = M_{deep}. - M_{surface}. which is determined on the basis of the requirement µΔN = K_{M}×ΔM = ∫JT(t)dt with integration in the range from 0 to τ, where µ is the chemical potential, N is the amount of substance, J(t) is the substance flow rate.

Kₚ×Δp = W_{blood} - is the power of the heat transfer process, carried out by capillary blood flow due to the difference in hydraulic pressure in the microcirculatory system, $Δp = {p}_{arter} - {p}_{venul} ,$ which is determined by the requirement $VΔp = \int {K}_{p} \times Δp dt = \int {W}_{blood} \left(t\right) dt ,$ with integration within the range between 0 and τ.

The heat transfer component in the volume of tissue under the applicator, due to the difference in blood pressure, depending on the mass transfer flow index, can have a positive value (heat is introduced into the system), negative value (heat leaves the system) or equal to zero. The constant of this process is expressed in the following form: K_{P} = V - V₀ > 0, where V₀ is the volume of the intercellular substance, at zero flow rate J = 0. The volume of the substance V > V₀, at positive flow J > 0; volume of the substance V < V₀, at negative flow J < 0.

K_{ΔT}, K_{M}, K_{P} - constants, phenomenological coefficients, which are determined by calibration.

The equation of state which correlates the power of coupled processes of energy and substance transfer under the applicator, which are measured in a real experiment, is derived from the equation (44a) by dividing all terms by dt and has the following form:

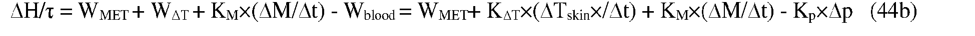

It should be noted that the fundamental equation of thermodynamics is an integral form of equation (44b), which correlates the energy flow and mass transfer processes. Integration of equation (44a) in the time range from t₀ = 0, corresponding to the application of the applicator with dosed pressure on the surface of the stratum corneum up to t = τ leads to the original form of the fundamental thermodynamic equation (3), which describes reversible changes in the system in the range Δt = τ, equal to the transient time constant: $\begin{matrix}ΔH \times \int dt / τ = \int \left({W}_{MET}\right) dt + \int \left({W}_{ΔT}\right) dt + {K}_{M} \times \int \left(ΔM/Δt\right) dt - {K}_{p} \times \int pdt = \\ = ΔH = T \times Δ \left({S}_{MET}+{S}_{ΔT}\right) + μΔN + VΔp = {ΔQ}_{MET} + {ΔQ}_{ΔT} + μΔN + VΔp .\end{matrix}$

Equation (4a) can be expressed as follows for calculating the magnitude of the thermal effect of metabolism: ${W}_{MET} = ΔH / τ - {K}_{ΔT} \times {ΔT}_{skin} - {K}_{M} \times ΔM + {K}_{p} \times Δp .$

The latter equation (45) may be presented in the following form: ${W}_{MET} = \left({H}_{deep}-{H}_{suf}\right) / τ - {K}_{ΔT} \times \left.\left({T}_{deep}-{T}_{suf}\right)/Δt\right) + {K}_{M} \times \left.\left({M}_{deep}-{M}_{suf}\right)/Δt\right) + {K}_{p} \times \left({p}_{arter}-{p}_{venul}\right)$

In the abovedescribed equations:

| | |
|---|---|
| H_{deep} | - deep layer enthalpy, |
| H_{surface} | - superficial layer enthalpy, |
| T_{deep} | - deep layer temperature, |
| T_{surface} | - superficial layer temperature, |
| M_{deep} | - deep layer water content, |
| M_{surface} | - superficial layer water content, |
| Pₐᵣₜₑᵣ | - hydrostatic pressure at the capillary entry (in the microcirculatory system), |
| Pᵥₑₙᵤₗₑ | - hydrostatic pressure at the capillary exit (in the microcirculatory system), |

Parameters K_{ΔT}, K_{M}, K_{P} correspond to the coefficients of proportionality and are equal to constant values determined using calibration method.

The value enthalpy is determined by the amount of water in a unit volume, which at the specified climatic parameters depends on the content of hyaluronic acid in the intercellular space. It may vary in diabetes mellitus and other chronic diseas, therefore, it is a patient's individual characteristic, that can be determined using calibration method. On the other hand, water content in a unit volume of tissue at the specified hyaluronic acid content, depends on the environmental conditions.

The last equation (46) describes the relationship between the state variables, provided that the volume of the cell and the intercellular space do not change (ΔVₜᵢₛₛᵤₑ = 0) with changes in glucose concentration and/or metabolic rate.

In general, changes in glucose concentration and/or the rate of cellular metabolism leads to changes in cell volume and intercellular space (ΔVₜᵢₛₛᵤₑ = ΔV_{intercell} + ΔV_{cell}). The caloric equation in the general case can be represented in the following form: ${H}_{0} / τ - \left(p\times {ΔV}_{intercell}+{K}_{V}+{ΔV}_{cell}\right) / Δt = {K}_{ΔT} \times \left({ΔT}_{skin}/Δt\right) + {K}_{M} \times \left({ΔM}_{intercell}/Δt\right) .$

The blood sugar level C can be determined using the caloric equation (46a) from the condition K_{C}×C = p×ΔV_{intercell}, by measuring the amount of water in the volume of the tissue ΔVₜᵢₛₛᵤₑ ≈ ΔV_{cell} and the amount of water in the intercellular space ΔM_{intercell} under the applicator of microcalorimeter.

Equation (46a) follows from the condition that the change of internal energy ΔU_{intercell} of the intercellular substance occurs as a result of a change in heat transfer from the depth to the surface: ${ΔU}_{intercell} / Δt = {K}_{ΔT} \times {ΔT}_{skin} / Δt + {K}_{M} \times ΔM / Δt .$

It takes into account, that
ΔU_{intercell} = ΔU_{C} + ΔUₘₑₜ, where ΔU_{C} is the contribution to the change in the internal energy of the intercellular substance due to a change in glucose concentration; ΔUₘₑₜ is the contribution to the change in the internal energy of the intercellular substance due to a change in heat generation in the cell.

The expression for ΔUₘₑₜ, as follows from the first law of thermodynamics, has the form: ${ΔU}_{met} = {ΔQ}_{MET} - p \times {ΔV}_{tissue} = {K}_{V} \times {ΔV}_{cell} , ΓAe {ΔQ}_{MET} - thermal effect of cell metabolism$

The expression for ΔU_{C}, as shown above (37), has the form: ${ΔU}_{C} = {H}_{0} - p \times {ΔV}_{intercell} ,$ where H₀ is the heat content of the intercellular substance in the region of the physiological norm.

### The method of isothermal calorimetric spectroscopy of living tissue.

Based on the above described isothermal microcalorimetry method (measurement of the magnitude of the electromagnetic radiation power absorbed by the living tissue using an isothermal phase transition microcalorimeter), a fundamentally new method of *in vivo* spectroscopy of living tissue can be created - absorption isothermal calorimetric spectroscopy, which enables real-time assessment of the content of biochemical components of living tissue on the basis of spectral measurements at the specific frequencies of the target molecules.

The magnitude of electromagnetic radiation power at the characteristic frequency of the biochemical component under study, absorbed in a local volume of substance of a living tissue, is measured not by the change in tissue temperature, but by the change in osmotic pressure value (or amount of water in the intercellular space), characterizing the volume of intercellular substance at the phase transition temperature (corresponding to the thermodynamic equilibrium between crystalline and liquid phases of the intercellular substance).

Characteristic frequencies of biochemical components of the living tissue substance, which include intercellular substance components (hyaluronic acid, water, glucose etc.), as well as intracellular biochemical molecules and organoids, form an electromagnetic absorption spectrum of the living tissue, which can be determined *in vivo* using isothermal calorimetric spectroscopy (ICS).

The method of isothermal calorimetric spectroscopy of living tissue is based on the detection of the magnitude of the absorbed power of electromagnetic radiation acting on the surface of the target local area of the epidermis of the living tissue, according to the amount of intercellular substance that has changed the phase state (melting of the globular phase of hyaluronic acid), by real-time measurement of the amount of water or osmotic pressure of the intercellular substance.

Figure 27 shows a diagram of the spectral device based on the principle of isothermal calorimetric spectroscopy.

The source of electromagnetic irradiation at a wavelength corresponding to the characteristic wavelength of the biochemical component of the living tissue, is integrated into an isothermal microcalorimeter. Following definitions were introduced: 1 - applicator (heat- and waterproof), which can be applied on the skin with gradual pressure, and formation of the closed thermodynamic living tissue system; 2 - source of the electromagnetic irradiation with adjustable wavelength; 3 - sensor of the osmotic pressure of the intercellular substance or intercellular hydrodynamic microflow.

The function of the response of the osmotic pressure of the intercellular substance vs wavelength of the irradiation (at a fixed power) is in direct correlation with the absorption spectrum of the substance; the response (changes) of osmotic pressure to the power of the irradiation is proportional to the absorbed power, and has a large value at the wavelengths at which the energy corresponds to the energies of excitation of vibrations in the studied molecules.

In some variants the invention design enables amplitude-modulation of the irradiation. **In** a spectral scheme using electromagnetic radiation with amplitude modulation in the tissue unit, under the applicator, oscillations of the elastic pressure of the intercellular substance occur due to the oscillations of the osmotic pressure at the irradiation modulation frequency. In this variant, the amount of absorbed power of electromagnetic radiation can be determined by measuring the elastic pressure of the tissue under the applicator at the irradiation modulation frequency. The closest analogue of the described method is the well-known method of optoacoustic (OA) spectroscopy, based on the effect of gas pressure pulse oscillations in a closed volume upon absorption of radiation modulated at an acoustic frequency. Such effect is due to the transformation of a part of energy of the iradiation-excited molecule into a thermal energy of the medium via radiationless transition. The optical-acoustic effect occurs due to the conversion of a part of the absorbed energy into thermal energy, which leads to the development of the acoustic vibrations in the sample itself or in the gas in contact with it. Registration of acoustic vibrations directly in the substance is carried out by a piezoelectric sensor (liquid and solid samples) or a microphone (gases).

In biochemistry, spectral measurement of a multicomponent system is carried out on the basis of a principle of additivity, according to which the absorption of an individual substance does not depend on other substances that have their own absorption. Thus, at a specified wavelength, the spectral density of a solution containing a mixture of non-interacting components is equal to the sum of the spectral densities of the individual components at the same wavelength. To assess the extent of absorption of the test solution containing any compound, the intensity of the irradiation penetrating this solution is compared to the intensity of the irradiation that has passed through the blank reference solution.

In living tissue spectroscopy, to assess the peak absorbance of the test compound spectral signal (irradiation intensity) at the wavelength corresponding to the characteristic frequency of the test compound is compared to the spectral signal of absorbance of the purified water at this wavelength, intensity of which can be analyzed on the basis of spectral measurement at another wavelength, corresponding to the water absorbance peak (not overlapping peaks of absorbance of the test substance).

The possibility of practical implementation of the isothermal calorimetric spectroscopy of living tissue biochemical components was proved (confirmed) by our experiments in which the correlation between the response of the osmotic pressure of the intercellular substance of the epidermis and the wavelength and power of the electromagnetic irradiation was measured; the correlation between osmotic pressure and electromagnetic irradiation in the optical and near-infrared (0.4 - 0.9 µm), infrared (1 µm - 10 µm) and microwave spectra (5 mm - 10 mm) was studied. The correlation between osmotic pressure and the power of infrared irradiation (with a maximum spectral power at a wavelength of 1 ~ 10 µm) is shown in Fig. 3.

### The method of tetrahertz (THz) calorimetric spectroscopy.

Terahertz (THz) spectroscopy of living tissue is based on the measurement of the absorbed power of terahertz irradiation acting on the surface of the studied local area of the epidermis of living tissue, by the amount of intercellular substance that has changed the physical state, by measuring the amount of water or osmotic pressure of the intercellular substance in real time.

With regard to biological systems, terahertz irradiation enables assessment of the role of water in biological processes, as well as characteristic oscillatory processes that can characterize the state of a biosystem. An important advantage of terahertz spectroscopy from the medical point of view is that this method is non-contact and non-invasive - irradiation in this spectral range does not cause ionization (like ultraviolet) and significant heating of the subject (like microwave). Having a high sensitivity to the phase state of water and the substance of living tissue, the terahertz range allows to determine the extent of linkage of water molecules in biological systems, the level of solvation of ions in the solution, and the level of hydration of surfaces at the phase boundary. It is known that the most effective spectral measurements in the terahertz range are carried out with samples that represent plane-parallel layers of various thicknesses (from a few millimeters to an order of micrometers or hundreds of angstroms).

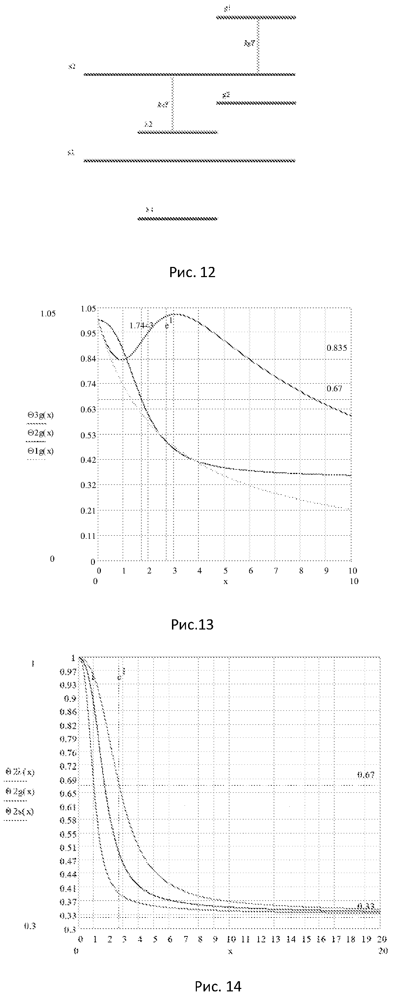

Fig. 12 schematically shows the energy level diagram (λ, g, s) of the hyaluronic acid tetramer in the intercellular substance, which allows to calculate the energy of a quantum of tetraherz radiation. The energy of transition between tetramer levels, equal to the binding energy ΔE ≈ 7k_{B}T/2 = 3,5k_{B}T ≈ 0,115 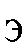 B of the tetramer molecular complex, determines the characteristic frequency of the tetramer, which corresponds to the wavelength of electromagnetic radiation λ ≈ 152 µm (65,78 cm⁻¹) in the terahertz spectrum range.

The binding energy of the tetramer is determined by the barrier height between the (1, s) conformations of the Na-tetramer: ΔE = Eₘₐₓ - Eₘᵢₙ ≈ 3,5 k_{B}T, where Eₘᵢₙ is a minimum energy value to the right from the energy barrier.

The wavelength of electromagnetic radiation corresponding to the energy of the transition of the tetramer to the excited state ΔE = Eₘₐₓ - Eₘᵢₙ ≈ 3,5 k_{B}T, depends only on the physiological temperature: λ = c×h/ΔE = c×h/3,5k_{B}T = 532 µm/3,5 ≈ 152 µm (65,78 cM⁻¹), where c - is the speed of light, *h* - is Planck's constant.

The resulting wavelength value corresponds to the terahertz (THz) range of the electromagnetic spectrum, which is in the wavelength range between 100 µm (far IR) and 1 mm (microwave).

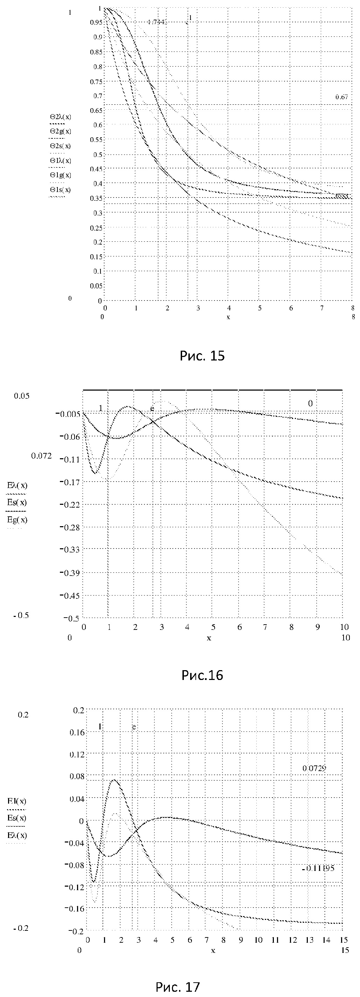

Exposure to electromagnetic radiation at a wavelength of λ ≈ 152 µm corresponding to an excitation energy of the tetramer of 3,5 k_{B}T, leads to the breakage of the hydrogen bonds of the tetramer and the conformational transition of the Na - tetramer from the conformation (s) of the crystalline phase to the conformation (λ) of the liquid phase, which is accompanied by absorption of a quantum of thermal energy, the value of which can be determined using Fig.16 by the energy barrier between (λ, s) conformations of Na - tetramer ΔE = (Eₘₐₓ - Eₘᵢₙ) - (Eₘₐₓ - E₀) =3,5 k_{B}T - 2,5 k_{B}T = 1k_{B}T, where E₀ - minimum amount of energy on the left from the energy barrier.

### Sensitivity of the living tissue to the thermal flux.

A feature of the natural biosensor of living tissue is that the hyaluronic acid tetramer (molecular recognizer) is highly sensitive not only to electromagnetic quantum *hv* at the characteristic frequency of the tetramer (λ ≈ 152 µm), corresponding to the dissociation energy of the tetramer ΔE =3,5 k_{B}T, but also has an extremely high sensitivity to thermal energy quanta, which are formed in the substance as a result of the absorption of electromagnetic radiation at frequencies that do not coincide with the characteristic frequency of the tetramer (λ ≈ 152 µm). For example, thermal energy quanta are formed in the intercellular substance as a result of absorption of the electromagnetic radiation at the water absorbance bands.

It is well-known that the spatial distribution of biochemical components in the microvolume of living tissue is non-uniform: hyaluronic acid, which is the main component of the intercellular substance, is not part of the intracellular substance; glucose concentration in the intercellular space is higher than inside the cell where glucose oxidation reaction occur; the total amount of water in the intercellular space of a unit volume of living tissue is much less than the total amount of intracellular water, since the volume of the cell is much larger than the volume of the intercellular space. Therefore, a conclusion is understood that the power of electromagnetic radiation at a wavelength corresponding to the characteristic frequencies of the hyaluronic acid tetramer and other fragments of this polymer macromolecule, as well as the frequencies of the glucose molecule, is absorbed mainly in the volume of the-intercellular substance. On the other hand, electromagnetic radiation at a wavelength corresponding to the characteristic frequencies of water is mainly absorbed in the volume of the cell. Radiation energy absorbed by intracellular water (and/or other intracellular molecules) in an isothermal process is converted into the energy of thermal motion of molecules in the cell and the intercellular space surrounding the cell; interaction of the intercellular substance tetramer with a thermal energy quantum (thermal collision of a substance molecule with a tetramer), which was formed in the cell volume, leads to an isothermal change in the osmotic pressure of the intercellular substance, as a result of tetramer dissociation. The thermal energy arising in the intercellular substance as a result of the absorption of the external electromagnetic radiation energy leads to isothermal melting of the crystalline phase of the intercellular substance.

Therefore, a living cell surrounded by intercellular substance, under physiological conditions, is an isothermal calorimetric power detector that converts electromagnetic energy absorbed inside the cell (or produced in the cell during metabolism) into the osmotic pressure of the intercellular substance.

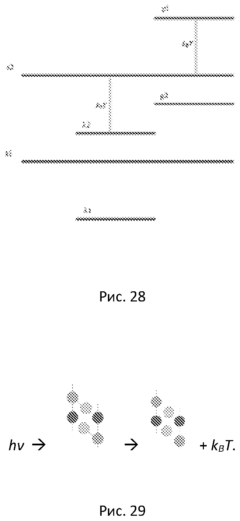

Figure 29 shows the scheme of isothermal calorimetric spectroscopy of biochemical components of a living cell. A living biological cell surrounded by an intercellular substance is a natural isothermal microcalorimeter of electromagnetic radiation power, which allows to determine the absorption spectrum of a living tissue substance based on the measurement of the correlation between the osmotic pressure of the intercellular substance and spectral power of the radiation acting on the tissue (1, 2, 3, 4, 5): 1 - electromagnetic radiation at the frequency characteristic of hyaluronic acid; 2 electromagnetic radiation at the frequency characteristic of glucose; 3, 4 - electromagnetic radiation at the frequency characteristic of water; 5 - electromagnetic radiation at the frequency characteristic of intracellular biochemical molecules or organelles.

Electromagnetic radiation at a wavelength corresponding to the characteristic frequency of water is absorbed in the cell: the energy of such radiation is converted into the energy of thermal motion of water molecules and free links of the polymer chain, which leads to the dissociation of the hyaluronic acid tetramer and an isothermal change in the osmotic pressure of the intercellular substance. A living cell surrounded by intercellular substance is an isothermal calorimetric power detector that converts electromagnetic energy absorbed inside the cell (or produced in the cell during metabolism) into the osmotic pressure of the intercellular substance.

Thus, in the absorption spectra of a living tissue substance (intercellular and intracellular), along with the characteristic frequency of the hyaluronic acid tetramer in the terahertz range (_ _ ≈ 152 µm), other frequencies corresponding to different groups of hyaluronic acid, water, glucose and other biochemical molecules will appear. Therefore, the spectral detection scheme should be developed taking into account the mutual influence on the results of spectral measurements of power absorption by adjacent absorption bands, in particular, terahertz radiation near the peak absorption region of the tetramer will also be absorbed by water molecules, the main part of which in a living tissue is located within the cell. For this reason, the response of the osmotic pressure of the intercellular substance of a local area of the living tissue to terahertz radiation at a wavelength (λ ≈ 152 µm), will consist of two contributions: the spectral response of the tetramer of the intercellular substance and the spectral response to the absorption of this radiation by water molecules. The spectral contribution due to the absorption of water exceeds the spectral contribution due to the absorption of electromagnetic energy by the tetramer, since, in a unit volume of living tissue, the volume of intracellular substance significantly exceeds the volume of intercellular substance. Water spectrum in the terahertz region of the intense absorption peak is close to the characteristic frequency of the tetramer; therefore, the use of the differential method of double-beam spectroscopy allows to isolate the osmotic pressure signal due to the absorption of radiation by the tetramer of the intercellular substance by subtracting the contribution from the total signal due to the absorption of intracellular water. To isolate the spectral signal of the tetramer, it is necessary to determine the intensity of the absorption peak of the tetramer using the described method; To increase the accuracy, one can use a triple-beam (three-wavelength) spectroscopy scheme, in which the wavelengths correspond to the local extrema of the absorption peak (λ ≈ 152 µm) and two side frequencies corresponding to the peak minima. In this case, the contribution to the signal due to the absorption of intracellular water is determined at a wavelength corresponding to the side frequencies of the hyaluronic acid tetramer absorption peak. From the resulting spectral distribution of the signal measured using triple-beam method, a constant signal corresponding to the absorption contribution of water molecules is subtracted. The described scheme of spectral measurement assumes that the spectrum of hyaluronic acid does not contain other absorption peaks adjacent to the tetramer absorption band (λ ≈ 152 µm), due to other fragments of hyaluronic acid.

### Tetraherz (THz) spectroscopy for measurement of blood glucose levels.

The method of isothermal calorimetric spectroscopy can be used in practice to determine the content of biochemical components (intercellular and intracellular substances) of living tissue in vivo. In particular, the method allows to determine the concentration of glucose in blood by measuring concentration of hyaluronic acid tetramers of the intercellular substance, which is proportional to the concentration of glucose bound by hyaluronic acid. It has been shown that 1/3 of the total number of glucose molecules in the hyaluronic acid macromolecule is in a free state, and the remaining 2/3 of the glucose molecules are bound to the monomers of the hyaluronic acid polymer chain. 1/3 of these glucose molecules are linked directly in the molecular complex of tetramer. Therefore, the concentration of free (unbound) glucose in the intercellular fluid (and in blood) can be determined by measuring the concentration of glucose bound to the tetramers of the intercellular substance (or the concentration of glucose bound to the polymer chain of hyaluronic acid).

### Isothermal THz calorimetric spectroscopy of water in the living tissue.

It is well-known that in biochemistry, spectral measurement of a multicomponent system is carried out on the basis of a principle of additivity, according to which the absorption of an individual substance does not depend on other substances that have their own absorption. Thus, at a specified wavelength, the spectral density of a solution containing a mixture of non-interacting components is equal to the sum of the spectral densities of the individual components at the same wavelength. To assess the extent of absorption of the test solution containing any compound, the intensity of the irradiation penetrating this solution is compared to the intensity of the irradiation that has passed through the blank reference solution.

When carrying out living tissue spectroscopy, in order to assess the absorption peak of the test biochemical compound, the spectral signal (radiation intensity) at a wavelength corresponding to the characteristic frequency of the test compound is compared to the spectral signal due to the absorption of purified water at the same wavelength.

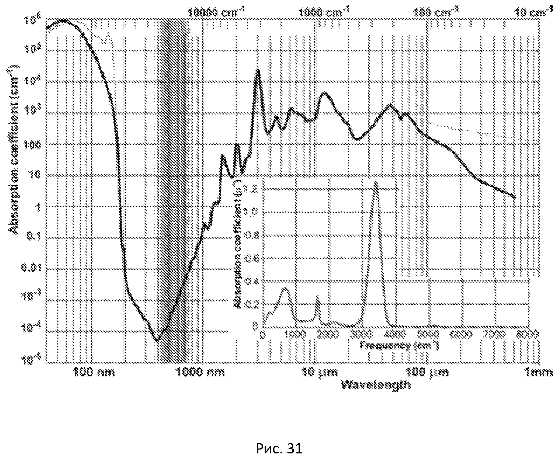

On the other hand, development of a method for real-time measurement of water content in living tissue is an urgent task of applied and fundamental importance. The method of isothermal calorimetric spectroscopy allows to determine the water content (in the intercellular space and cell volume) of a local area of living tissue in vivo by measuring the electromagnetic absorption spectrum at wavelengths corresponding to the characteristic frequencies of water, which are well-known when using purified water. The absorption spectrum of water in a wide frequency range (from ultraviolet to microwave) is shown in Fig. 31.

The energy of the incoming electromagnetic radiation, the wavelength of which corresponds to the characteristic frequencies of water, is absorbed in the cell volume and converted into the energy of the thermal motion of water molecules (the principle of an isothermal calorimeter), which leads to an isothermal change in the osmotic pressure of the intercellular substance as a result of melting of the crystalline phase of the intercellular substance.

For example, let us review the method of spectral detection in the area around the absorption band of water at a characteristic frequency of 3339 cm⁻¹ (2,99 µm) in the range from 3250 cm⁻¹ - 3750 cm⁻¹ (ref. to Fig. 31).

The distribution of the spectral power of the absorption peak can be determined using a triple-beam (three-wavelength) spectroscopy scheme, in which the wavelengths correspond to the local extrema of the peak (the wavelength corresponding to the peak maximum of 3339 cm⁻¹ (2,99 µm) and two side frequencies of 3250 cm⁻¹ and 2750 cm⁻¹, corresponding to the peak minima. The amount of heat absorbed at a given wavelength in the frequency range between 3250 cm⁻¹ and 2750 cm⁻¹ is determined by the magnitude of the change in the osmotic pressure of the intercellular substance.

In case of spectral measurements in the terahertz region (λ ≈ 152 µm), discussed above, the water absorption coefficient can be taken as a constant, since in the water absorption spectrum close to the characteristic frequency (λ ≈ 152 µm) of the intercellular substance tetramer, according to the Fig. 31, no local absorption peaks are observed.

### Spectral method of determination of glucose concentration from the water absorption bands.

The osmotic pressure and the amount of water in the intercellular substance is determined by the concentration of glucose and the magnitude of the external heat flux incident on the surface of the epidermis, determined by climatic parameters. The method of isothermal calorimetric spectroscopy that allows to measure the amount of water in a local microvolume of living tissue, can be practically applied to determine the concentration of glucose in the intercellular substance and blood. As it was shown, the concentration of glucose in blood can be determined based on the experimental correlation between the osmotic pressure and the power of the incident infrared thermal radiation flux, as shown in Fig. 3a, according to the slope of the plot of the osmotic pressure of the intercellular substance in the epidermis vs. the power of the incident thermal radiation.

The method of microcalorimetric spectroscopy described above allows to determine the correlation between the osmotic pressure and thermal power in a different way, using
a modulated electromagnetic radiation (with amplitude or frequency modulation). In the amplitude modulated measurement scheme, radiation is applied at a wavelength corresponding to the selected characteristic frequency of water, with amplitude (intensity) modulation. An important feature of the spectral measurement method using amplitude-modulated radiation is that the method allows to eliminate the dependence of the osmotic pressure signal on low-frequency fluctuations of environmental parameters. The method of spectral measurement with amplitude modulation allows non-invasive calibration of the measuring device by determining the value of the enthalpy and other calibration parameters using the caloric equation (45).

In the frequency modulation variant, spectral measurements are made at several wavelengths corresponding to the characteristic absorption frequencies of water. Measuring the response of the osmotic pressure of the intercellular substance to electromagnetic radiation (with the same power) at several different wavelengths with at the set absorption coefficients allows to determine the correlation between the osmotic pressure signal of the intercellular substance and the power of electromagnetic radiation absorbed in the local area of the living tissue under the applicator.

The correlation between the osmotic pressure and the absorbed power, determined by the described spectral method using modulated radiation, allows to determine the concentration of glucose in blood. An important feature of the method is that the spectral measurement at several frequencies (at least two), which can be performed in real time, allows to eliminate the contribution to the osmotic pressure signal due to low-frequency fluctuations of climatic parameters.

Factors limiting the accuracy of the described spectral method for measuring glucose concentration include the proximity of water absorption peaks to the absorption peaks of other molecules, such as hyaluronic acid and glucose. As it was noted above, in a living biological tissue, the spectral absorption bands of water, the peak intensity of which is determined by the volume of intracellular water, are adjacent to the glucose absorption bands. The resulting osmotic pressure signal is mainly determined by the absorption of radiation power by intracellular water, the contribution of which to the signal exceeds the contribution due to the absorption of electromagnetic energy power by the biochemical components of the intercellular substance (hyaluronic acid and glucose), as the volume of intracellular substance in a unit volume significantly exceeds the volume of intercellular substance. The diagram of spectral measurements at the characteristic frequencies of water, which takes into account the absorption spectrum of glucose, allows to increase the signal-to-noise ratio in the measuring scheme.

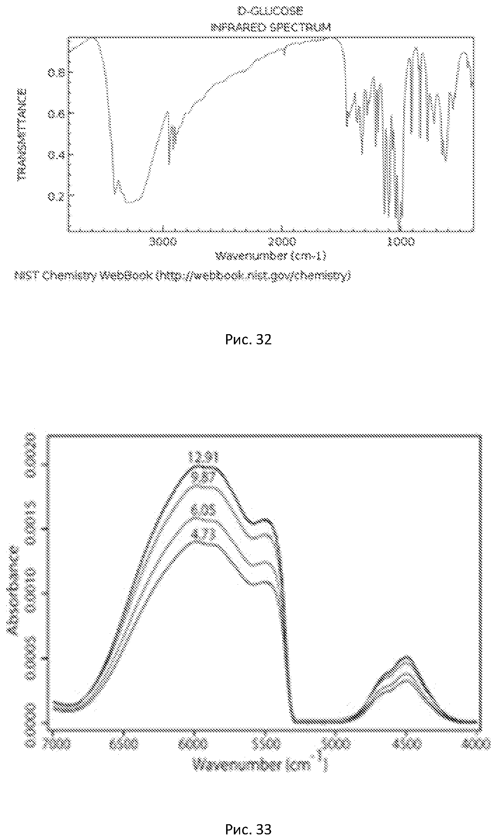

Absorption spectra of glucose in the IR range and transmission in the near IR region in the range of wavelengths from 1,428 µm (7000 cm⁻¹) to 20 µm (500 cm⁻¹), which were studied in detail, are shown in Fig. 32. Fig. 33 shows the absorption spectra of glucose in the near-IR region in the wavelength range between 1,428 µm (7000 cm⁻¹) and 2,5 µm (4000 cm⁻¹). According to the graphs, fluctuations in the blood glucose concentration leads to the fluctuations in the intensity of peaks of the glucose uptake.

To assess the correlation between the osmotic pressure of the intercellular substance and the power of the incident electromagnetic radiation in a single-beam measurement scheme with amplitude modulation of radiation, the wavelength can be selected in one of the intervals between the glucose absorption peaks, for example, 7000 cm⁻¹ (1,428 µm), 5000 cm⁻¹ (2,0 µm), 4000 cm⁻¹ (2,5 µm).

In a differential double-beam measurement scheme using frequency modulated radiation, at two (or more) wavelengths, it is possible to select frequencies that do not fall within the range of the glucose uptake peaks, the side frequencies of the glucose uptake peak, for example, 7000 cm⁻¹ (1,428 µm), 5000 cm⁻¹ (2,0 µm), 4000 cm⁻¹ (2,5 µm).

In the above described spectral measurement scheme using modulated radiation, the possible contribution to the resulting signal of the absorption peaks of intercellular hyaluronic acid, which are adjacent to the water absorption peaks, is disregarded, but they make a contribution to the resulting signal that is much smaller than the contribution due to absorption at water frequencies, since the volume of intracellular substance, in a unit volume of living tissue, significantly exceeds the volume of the intercellular substance.

Thus, the concentration of glucose in blood can be determined based on isothermal calorimetric spectroscopy in two different ways:
*terahertz spectroscopy method* - measurement of the concentration of hyaluronic acid tetramers in the intercellular substance (proportional to the concentration of bound glucose in the intercellular substance) at a wavelength of terahertz radiation (λ≈ 152 µm), corresponding to the characteristic frequency of the tetramer;
*infrared spectroscopy method* - measurement of the concentration of hyaluronic acid tetramers based on the correlation between the osmotic pressure of the intercellular substance and the absorbed radiation power, which is determined in real time based on spectral measurements using radiation with amplitude modulation at one wavelength (or several wavelengths) corresponding to the characteristic frequency of water in the infrared or other region of the electromagnetic spectrum.

The signal-to-noise ratio in the abovedescribed spectral measurement scheme can be improved by additional measurement of other biochemical components of the extracellular substance, in particular, the content of hyaluronic acid. Spectroscopy of hyaluronic acid and other components of the intercellular substance is discussed below.

### Isothermal calorimetric spectroscopy of the intercellular substance.

The absorption spectrum of the intercellular substance (which is a multicomponent biopolymer) has a more complex structure of spectral absorption bands compared to the spectrum of pure water, since, along with intense absorption peaks of water (mainly intracellular), it contains absorption peaks corresponding to the characteristic frequencies of the biochemical components of the intercellular substance, the main components of which are water, hyaluronic acid and glucose associated with the monomers of the polymer chain of hyaluronic acid, intercellular (tissue) fluid, similar in composition to blood plasma, which consists of water, amino acids, sugars, fatty acids, coenzymes, hormones, neurotransmitters, salts, as well as waste products of cells.

It should also be noted that the characteristic binding energies of molecules of various pharmaceutical compounds, as well as molecules of compounds and foreign substance in condensed media of the intercellular substance that are physiologically incompatible with the metabolism of living tissue, can also be found in the infrared and terahertz range of the electromagnetic spectrum and appear in the absorption spectra of the intercellular substances.

To assess the absorption peaks of the studied biochemical component of the intercellular substance of living tissue, the spectral signal (radiation intensity) at a wavelength corresponding to the characteristic frequency of the studied compound is compared to the spectral signal due to the absorption of pure water at a given wavelength.

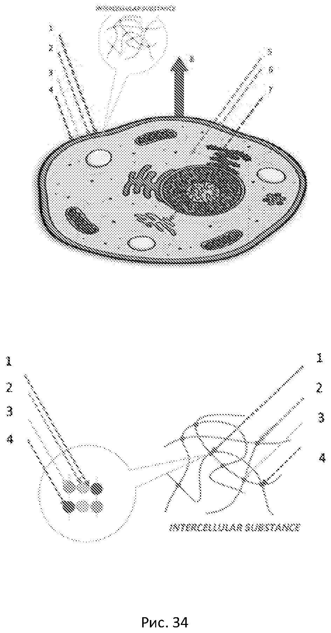

Figure 34 below schematically shows the structure of an intercellular substance representing a branched macromolecule of hyaluronic acid having the configuration of a polymer network surrounding a living cell. Polymer mesh cross-links are formed as a result of volumetric interactions of polymer chain monomers with solvent (intercellular fluid) glucose in the tetramer configuration - a molecular complex consisting of two non-adjacent disaccharides of the hyaluronic acid polymer chain and two D-glucose molecules. Along with the main characteristic frequency (λ ≈ 152 µm) in the terahertz region of the spectrum, corresponding to the dissociation energy of the molecular complex of the crosslink, absorption peaks will be found in the absorption spectrum of the intercellular substance corresponding to the characteristic frequencies of hyaluronic acid disaccharides and the D-glucose molecule bound by the monomers of the polymer chain substances.

A living cell surrounded by an intercellular substance is a natural isothermal power calorimeter that allows to determine the absorption spectrum of the intercellular and intracellular substance of a living tissue based on measuring the correlation between the osmotic pressure of the intercellular substance and the spectral density of electromagnetic radiation at different wavelengths (1, 2, 3, 4, 5 , 6, 7): 1 - terahertz radiation at the characteristic frequency (λ≈ 152 µm) of the hyaluronic acid tetramer; 2- radiation at characteristic frequencies of glucose bound to the monomers of the polymer chain of hyaluronic acid; 3, 4 - radiation at characteristic frequencies of hyaluronic acid disaccharide; 5, 6 - radiation at the characteristic frequencies of water; 7 - electromagnetic radiation at the characteristic frequency of an intracellular molecule.

The absorption of terahertz radiation, the wavelength of which corresponds to the characteristic frequency of the hyaluronic acid tetramer (λ ≈ 152 µm), leads to the dissociation of the molecular complex of the tetramer with the conversion of electromagnetic energy into the energy of thermal motion of molecules, which results in an isothermal change in the osmotic pressure of the intercellular substance. The energy of the incident electromagnetic radiation, the wavelength of which differs from the characteristic frequency of the tetramer, but corresponds to the characteristic frequencies of the biochemical components of the intercellular substance (hyaluronic acid, glucose, water, etc.), is absorbed in the volume of the substance and converted into the kinetic energy of the thermal motion of molecules, which leads to a change in the osmotic pressure of the intercellular substance as a result of the dissociation of the hyaluronic acid tetramer.

### Glucose spectra

Figure 32 shows a graph explaining the nature of the characteristic frequencies of the glucose molecule in the infrared range.

### Spectra of hyaluronic acid and free fatty acids.

Infrared spectra of the substances under study that are components of the constituent parts of biological tissues (glucose, hyaluronic acid, protein - egg albumin, lipid - fat stock), in the frequency range 4000 cm ¹ - 600 cm⁻¹ in this study using Fourier IR spectrometer (Tensor 37 by Bruker) are shown in Fig. 36.

In the high-frequency IR region of the spectrum 4000 cm⁻¹ (2,5 µm) - 3000 cm⁻¹ (3,3 µm), in which an intense water absorption peak was found (Fig. 31), in the spectra of hyaluronic acid, glucose and protein, intense absorption peaks due to the characteristic frequencies of the valence bonds of O-H groups were also observed. In this region (4000 cm⁻¹ - 3000 cm⁻¹) there is no fat absorption peak, which is observed adjacent in the range between 3040 cm⁻¹ (3,29 µm) and 2853 cm⁻¹ (3,5 µm), which also includes the absorption lines of all the considered components, due to frequencies of CHₙ - groups present in the structure of these molecules.

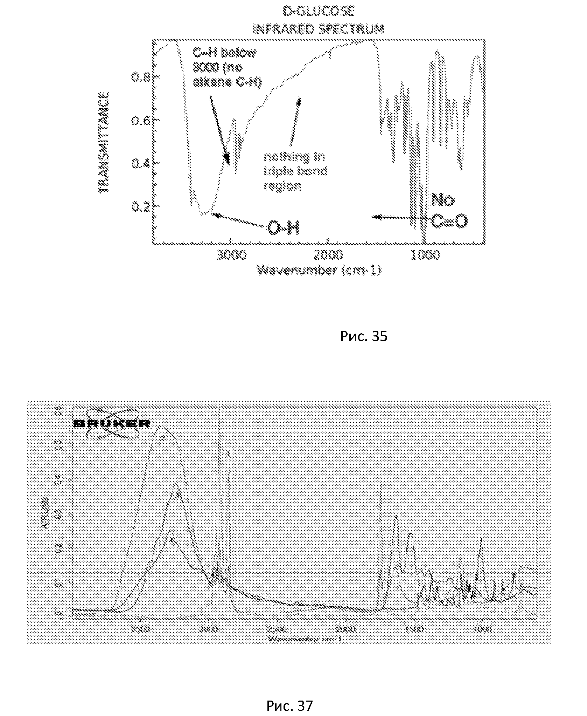

The structure of the spectrum in the lower frequency region of the IR spectrum can be analyzed on the basis of the selected fragment of the spectra of all considered components, shown in Figure 37. A very intense narrow band of carboxylic carbonyls at 1743 cm⁻¹ (5,7 µm) and a less intense at 1553 cm⁻¹ (6,4 µm) is present in the lipid spectrum (fat stock); these two bands, which also present in the spectra of all animal fats (fat stock, pork, chicken, marmot, fish and vegetable oils - olive, almond, linseed, cottonseed, sunflower, etc.), are due to the frequencies of fatty acid triglyceride (mainly unsaturated).

The band of carboxyl carbonyls 1743 cm⁻¹ (5,7 µm) is present in the spectrum of hyaluronic acid (Figure 37, curve 3), the polymer chain of which consists of a large number of repeating monomers, each consisting of a disaccharide having a -COOH group in the structure. Both carbonyl's bands 1743 cm⁻¹ (5,7 µm) and 1553 cm⁻¹ (6,4 µm) are lacking in the spectrum of glucose (fructose, sucrose, cellulose, starch) (Figure 37, curve 2). The band of carboxyl carbonyls 1553 cm⁻¹ (6,4 µm) is observed only in the spectrum of the protein (egg albumin) (Figure 37, curve 4), which allows to attribute it to the appearance of C=0 groups of the polypeptide bond (1600 cm⁻¹ - 1550 cm⁻¹).

### Spectral method for hyaluronic acid content measurement.

Measuring the content of hyaluronic acid in living tissue is an urgent task that has independent practical significance in cosmetology, dermatology, regenerative medicine, etc.; The relevance of the issue is due to the need to monitor hyaluronic acid for early diagnosis of diabetes, as well as for non-invasive monitoring of blood glucose in patients with diabetes mellitus. Clinical studies in a group of patients with diabetes mellitus showed the effect of the changes of the content of hyaluronic acid on the temporal changes in the osmotic pressure, at a constant concentration of glucose in blood, in the time interval of the experiment.

As can be seen from the comparison of the spectra (Fig. 37, the absorption bands of hyaluronic acid and glucose are located adjacent to the intense peak of water absorption in the region (4000 cm⁻¹ - 3000 cm⁻¹), while the intensity of the water absorption peak is determined by the absorption in the cell volume, therefore, it is significantly higher than the intensity of the glucose peaks and hyaluronic acid of the intercellular substance. Therefore, the spectroscopy of hyaluronic acid and glucose, which are components of the intercellular substance, is based on the selection of weak spectral absorption peaks of the components of the intercellular substance against the background of an intense spectral peak of water due to the absorption of water in the cell volume, which is much greater than the intensity of the spectral peaks of glucose and hyaluronic acid of the intercellular
substance; the purpose of spectral measurement of biochemical components of intercellular substance is a more complex experimental issue vs. spectral measurement of the amount of water per unit volume of living tissue.

### IR spectroscopy of hyaluronic acid.

It is well-known that hyaluronic acid is non-homogeneously distributed in the epidermis, its content in the stratum corneum and upper layers of the epidermis is higher than its content in the dermis; the authors of the study [ ...] assume that the transport of hyaluronic acid into the stratum corneum is carried out from keratinocytes located directly under the stratum corneum; keratinocytes are the main cells of the human epidermis (approximately 90% of all epidermal cells), which are present in the basal, spiny, granular, shiny stratum corneum of the epidermis.

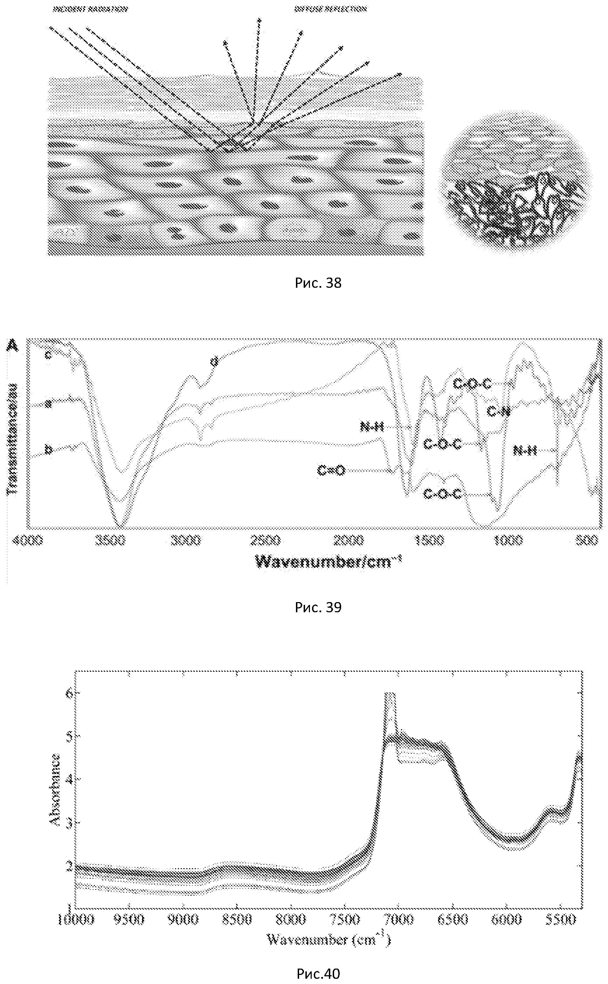

To measure the content of hyaluronic acid in the upper layers of the epidermis, the method of diffuse reflectance spectroscopy can be applied, which is based on the registration of electromagnetic radiation diffusely reflected from the upper layers of the epidermis. The method of spectral measurement of the content of hyaluronic acid in the epidermis, based on the registration of electromagnetic radiation diffusely reflected from the epidermis, is shown in Fig. 38.

Figure 38 shows a diffuse reflectance spectroscopy scheme for measuring the content of hyaluronic acid and free fatty acids in the intercellular substance of the surface layer of the living tissue epidermis. The radiation diffusely reflected from the superficial layer of the epidermis is collected at a wide angle and transmitted to the detector.

To measure the content of hyaluronic acid in the superficial layer of the epidermis, near electromagnetic radiation in the near infrared (or optical) region at a wavelength corresponding to the characteristic frequency of hyaluronic acid can be used. For example, a frequency in the range of the absorption peak of 7000 cm⁻¹ (1,43 µm) in the spectrum near the absorption peak of glucose 6000 cm⁻¹ (1,67 µm) can be used. The spectrum of hyaluronic acid in the near infrared region is shown in Fig.32.

Figure 39 shows the absorption spectra of hyaluronic acid in the near IR region and the transmission spectrum in the IR region.

On the other hand, absorption peaks of hyaluronic acid have been noted to occur adjacent to the intense water absorption peaks. Fig. 40 shows the absorption spectra of hyaluronic acid in the IR and near-IR spectral regions, which indicates that the absorption peaks of hyaluronic acid near 3500 cm⁻¹ (2,8 µm) and 7000 cm⁻¹ (1,43 µm) are overlapping with the absorption peaks of water (Fig. 31).

To isolate the spectral signal corresponding to the peak of hyaluronic acid absorption against the background of an intense water absorption peak, a single-beam (one-wavelength) combined spectral method is used, which is a result of a combination of two methods - isothermal calorimetric spectroscopy, which allows to determine the water content in the epidermis (since the intensity of the absorption peak of intracellular water is much higher than the absorption intensity of intercellular hyaluronic acid), and diffuse reflectance spectroscopy, which allows to determine the total content of water and hyaluronic acid in the superficial layer of the epidermis. The contribution of hyaluronic acid is derived from the resulting spectral signal of diffuse reflectance by subtracting temporal changes of the amount of water in the stratum corneum under the heat- and waterproof applicator applied to the surface with dosed pressure, from the total signal. The measurement is carried out according to the scheme of single-beam spectroscopy at one wavelength corresponding to one of the characteristic frequencies of hyaluronic acid, which is selected in the frequency range of the spectrum in which the absorption peaks of hyaluronic acid are overlap (or are adjacent) the absorption peaks of water (UV, optical, near IR, IR, far IR).

In the near IR region, one can choose, for example, a wavelength of 7000 cm⁻¹ (1,43 µm), in the IR region - 3500 cm⁻¹ (2,8 µm). In the presented variant of the combined scheme, the use of an additional spectral measuring channel based on the registration of electromagnetic radiation diffusely reflected from the superficial layer of the epidermis, as well as the use of the measuring method of synchronous signal detection, allows to increase the signal-to-noise ratio of the measuring channels. Correlation between the osmotic pressure response and the power of the incoming electromagnetic radiation with amplitude modulation at a wavelength corresponding to the characteristic frequency of water allows to determine the concentration of glucose based on the abovedescribed method.

Therefore, a combination of two spectral methods (isothermal calorimetric spectroscopy and diffuse reflectance spectroscopy) in one spectral device enables better signal separation and detection of the content of the three biochemical components of living tissue (glucose, hyaluronic acid and water) using measurements at one wavelength, corresponding to one of the characteristic frequencies of hyaluronic acid (single beam spectroscopy).

### Double-beam spectral method for glucose content measurement.

The above described combined spectral method, based on a double-beam spectroscopy (at two wavelengths) corresponding to the characteristic frequencies of glucose and hyaluronic acid (close to the water absorption peak), enables better signal separation and improves signal-to-noise ratio to determine the concentration of glucose, and also the content of two other components, hyaluronic acid and water, the low-frequency temporal changes of which directly affect the signal-to-noise ratio when measuring glucose concentration. Spectral measurement at each wavelength is carried out in two ways (isothermal calorimetric spectroscopy and diffuse reflectance spectroscopy), the combination of which in one spectral device allows to improve signal separation. As an example, in the near-IR region, a wavelength of 6000 cm⁻¹ (1,67 µm) corresponding to the characteristic frequency of glucose and a wavelength of 7000 cm⁻¹ (1,43 µm) corresponding to the characteristic frequency of hyaluronic acid can be selected.

In another version of the double-beam measurement scheme, the glucose concentration is determined using caloric equation (45), in which the enthalpy ΔH, which depends on the content of hyaluronic acid in the intercellular substance (and has individual values for different patients) and the amount of water in a tissue unit volume, which depends on environmental parameters, is determined by the spectral method based on the hyaluronic acid and water absorption bands.

In another variant, the concentration of glucose is determined using triple-beam spectroscopy at 3 wavelengths corresponding to the selected absorption peaks of glucose, water and hyaluronic acid. Spectral measurements are carried out in the following ranges of the spectrum: in the range of 3550 cm⁻¹ - 3050 cm⁻¹ (2,8 - 3,3 µm) at one frequency of 3339 cm⁻¹ (2,99 µm) corresponding to the water absorption peak (or three frequencies corresponding to the absorption peaks of water, hyaluronic acid and glucose); at a frequency of 1637 cm⁻¹ (6,08 µm), corresponding to the second peak of glucose absorption; at a frequency of 1553 cm⁻¹ (6,4 µm), corresponding to the third peak of glucose absorption.

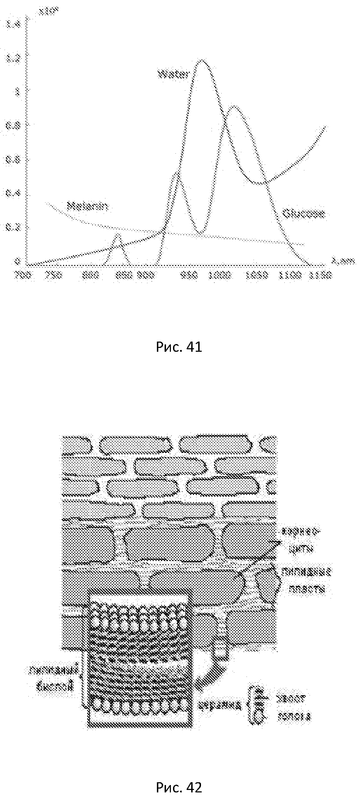

The spectral glucose absorption bands in the optical and near-infrared spectral range are also close to the intense water absorption peaks (Fig. 31) Fig. 41 shows the theoretical absorption spectra of glucose, melanin and water in the red and near- IR range.

The method for measuring glucose concentration in the red and near-IR range is based on the single-beam combined method described above. To isolate the spectral signal corresponding to the glucose absorption peak against the background of an intense water absorption peak, a single-beam combined spectral method is used, which is obtained as a result of a combination of two methods - isothermal calorimetric spectroscopy, which allows to determine the water content in the epidermis (since the intensity of the absorption peak of intracellular water is many times higher than that of intercellular hyaluronic acid), and diffuse reflectance spectroscopy, which allows to determine the total content of water and glucose in the superficial layer of the epidermis. A combination of two spectral methods (isothermal calorimetric spectroscopy and diffuse reflectance spectroscopy) in one spectral device allows to improve signal separation and determine the content of two biochemical components of living tissue, glucose and water, using measurements at one wavelength corresponding to one of the characteristic frequencies of glucose (single-beam spectroscopy).

The measurement channel based on isothermal calorimetric spectroscopy allows to determine the water content in the volume of the epidermis, since the intensity of the peak of intracellular water absorption is many times higher than that of glucose absorption, the concentration of which in the cell volume is significantly lower than its concentration in the intercellular space, as a result of the process of glucose oxidation in the volume of the cell; the second measurement channel, based on diffuse reflectance spectroscopy, allows to determine the total content of water and glucose in the superficial layer of the epidermis.

In a dual beam spectroscopy, to obtain better separation, two wavelengths are used, for example, a wavelength in the range of 930 -1050 nm, corresponding to the width of the water absorption peak, and a wavelength in the range of 1000 -1100 nm, corresponding to the absorption peak of glucose; additionally, a wavelength in the range of 900 - 970 nm, corresponding to the second water absorption peak, can be used. In case of triple-beam spectroscopy, the content of hyaluronic acid is additionally determined at a wavelength that is chosen in the same frequency range, or in another near - IR region, for example, 7000 cm⁻¹ (1,43 µm).

The scheme of spectral measurements in the optical range should be developed taking into account the need for individual calibration for the melanin content in the skin.

### Method for measuring fatty acid triglyceride content.

Electromagnetic radiation at a wavelength corresponding to the characteristic frequencies of free fatty acids will be intensively absorbed in the lipid layer of the epidermis under the stratum corneum, as well as in the fat layer located under the dermis. The lipid layer of the epidermis, located under the stratum corneum, consists of 10-25 layers of cells oriented parallel to the surface and immersed in the lipid matrix, which makes up about 10% of its volume, and has a unique structure and chemical composition. The skin contains both free fatty acids and fatty acids bound with formation of triglycerides, phospholipids and ceramides. Epidermal lipids mainly consist of: ceramides (50%); cholesterol and its esters (25%) saturated fatty acids (10%).

To determine the content of free fatty acids, a spectral method can be used, which is a combination of two spectral methods, isothermal calorimetric spectroscopy (for probing the fatty layer of the skin located under the dermis) and diffuse reflectance spectroscopy, based on the registration of electromagnetic radiation diffusely reflected from the intercellular substance of the lipid layer of the epidermis with a high content of free fatty acids, located under the stratum corneum; which consists of 10 - 25 layers of cells, oriented parallel to the surface and immersed in the lipid matrix, which is about 10% of its volume, having a unique structure and chemical composition. Epidermal lipids mainly consist of: ceramides (50%); cholesterol and its esters (25%) saturated fatty acids (10%) (fig. 42).

As can be seen (Fig. 36), two absorption peaks of hyaluronic acid fall into the bands of intense absorption peaks of fatty acid triglyceride 1743 cm⁻¹ (5,7 µm) and 3040 cm⁻¹ (2,853 µm), which are in the range between two peaks of water absorption 1645 cm⁻¹ (6,08 µm) and 3339 cm⁻¹ (3,0 µm).

The content of fatty acid triglyceride and hyaluronic acid in living tissue can be determined using the method of isothermal calorimetric single-beam spectroscopy at wavelengths corresponding to the characteristic frequencies of fatty acid triglyceride 1743 cm⁻¹ (5,7 µm) and/or 3040 cm⁻¹ - 2853 cm⁻¹ (3,3 - 3,5 µm).

In another variant, a spectral method for measuring fatty acid triglyceride content is based on the use of a double-beam spectral scheme at wavelengths corresponding to one of the characteristic frequencies of hyaluronic acid (combined method described above), for example, 3500 cm⁻¹ (2,8 µm) - 3000 cm⁻¹ (3,3 µm), and one of the characteristic frequencies of the fatty acid triglyceride, for example, 3040 cm⁻¹ - 2853 cm⁻¹ (3,3-3,5 µm). In this variant, the spectral measurement in the range of 2,8 - 3,3 µm is carried out on the basis of a combined method (combination of isothermal calorimetric spectroscopy and diffuse reflectance spectroscopy), and the measurement at the triglyceride wavelength is carried out according to the diffuse reflectance spectroscopy scheme, which allows to determine the content of triglyceride in the superficial lipid layer of the epidermis or according to the isothermal calorimetric spectroscopy scheme, which allows to determine the triglyceride content in the fatty layer under the dermis. The described measurement scheme allows to record a signal proportional to the content of triglyceride, or a total signal proportional to the total content of glucose and lipids, characterizing the rate of glucose and free fatty acids metabolism.

### A method of triple-beam spectroscopy of glucose, hyaluronic acid and fatty acid triglyceride.

Glucose concentration can be determined based on a triple-beam spectroscopy method with the use of modulated emission spectroscopy at a wavelength corresponding to the characteristic frequencies of water by adding a double-beam spectroscopy method at wavelengths corresponding to the characteristic frequencies of hyaluronic acid and fatty acid triglyceride. The advantage of this method is that the intensity of the absorption peaks virtually does not change in the experiments throughout the day (and week), so the measurement is done using double-beam spectroscopy at two wavelengths corresponding to the characteristic frequencies of water and hyaluronic acid. A single-beam scheme of a spectral device at a wavelength corresponding to the characteristic frequency of 1743 cm⁻¹ (5,7 µm) can be used to monitor the content of hyaluronic acid in living tissue.

### Method of spectroscopy of intracellular biochemical macromolecules and organelles

The method of microcalorimetric spectroscopy described above, in general, allows to determine the concentration of macromolecules of an intracellular substance using electromagnetic radiation with frequencies (wavelengths) that coincide with the characteristic frequencies of tetramers of intracellular macromolecules (proteins, RNA, DNA).

The characteristic frequencies of intracellular proteins fall into the far infrared range of the electromagnetic spectrum. For example, the characteristic binding energies in the macromolecule of the globular enzyme glucokinase and other intracellular enzymes are approximately 7 kcal/mol - 20 kcal/mol or 10 k_{B}T - 30 k_{B}T, corresponding to a wavelength interval between 20 and 70 µm in the far IR region of the spectrum.

The concentration of the main biochemical components of the intercellular substance, in general, can be determined using the differential method according to the four-beam (four wave) spectroscopy method, which uses wavelengths corresponding to the characteristic frequencies of the 3 main components of the intercellular substance, hyaluronic acid, glucose, water molecules, as well as a wavelength corresponding to the characteristic frequency of an intracellular macromolecule or complex; in the course of measurement, each of the four biochemical compounds can be represented by a set of 1 (or more) characteristic frequencies. Therefore, the number of wavelengths that characterize a group of 2 biochemical components of the intercellular substance, a water molecule and 1 intracellular molecule under study can be more than 4.

Absorption of an incident electromagnetic radiation at wavelengths corresponding to the characteristic frequencies of intracellular biochemical components by intracellular macromolecules (and biochemical complexes) will lead to a change in the heat balance in the study area of the living tissue under the applicator (heating) and, as a consequence, to a change in the osmotic pressure of the intercellular substance due to the breakage of the internal intermolecular bonds of hyaluronic acid tetramers of the intercellular substance, which have a characteristic binding energy of the order k_{B}T.

### DESCRIPTION OF THE PREFERRED EMBODIMENT(S)

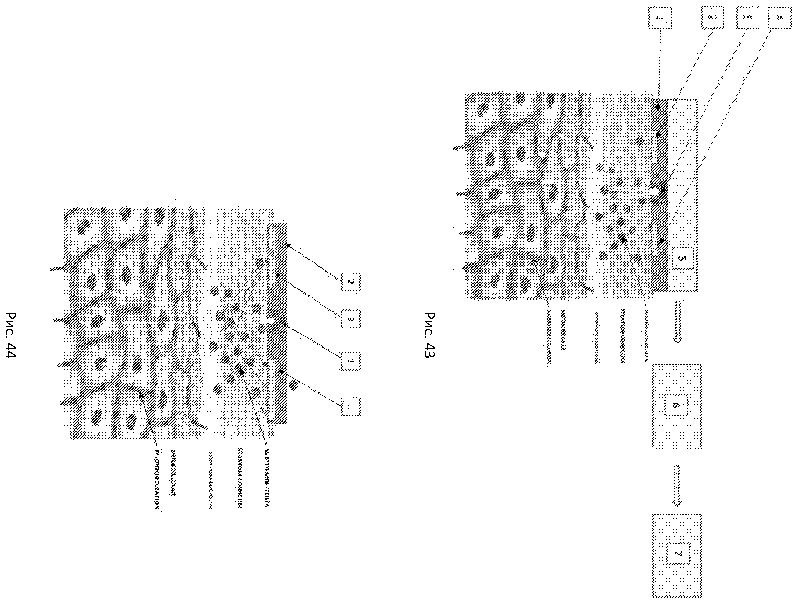

The proposed method for spectroscopy of the intercellular substance of a local area of living tissue can be implemented using a spectral device, which is schematically shown in Fig. 43. Block diagram of a spectral device for isothermal calorimetric spectroscopy of biochemical components of living tissue (isothermal calorimetric spectrometer) includes: 1 - heat- and waterproof applicator; 2 - sensor of osmotic pressure of intercellular substance; 3 - source of electromagnetic radiation with constant and/or modulated intensity; 4 - physiological parameter sensor; 5 - a unit of instrumental amplifiers and/or a synchronous detector and/or an analog-to-digital converter (ADC); 6 - data processing unit; 7 - data display unit.

The device contains several sensors for measuring physiological parameters, one of which is designed to measure the osmotic pressure of the intercellular substance, as well as one or several sources of electromagnetic radiation with a constant or modulated intensity.

The device consists of an applicator 1 (heat- and waterproof, having an upper and lower surface), which can be applied on the surface of the epidermis with dosed pressure, one or several sources 3 of electromagnetic radiation, with a constant or modulated intensity, with a wavelength corresponding to characteristic frequency of the studied biochemical component of the living tissue, a device for creating a calibration effect on the controlled tissue area under the applicator, an osmotic pressure sensor of the intercellular substance (2), sensors for measurement of temperature and other physiological parameters (4) located under the applicator, from the inside of the applicator on the surface of the stratum corneum (SC), while the signals from the above sensors are transferred sequentially to the terminals of an amplifier unit 5 and/or a synchronous detector and/or an analog-to-digital converter (hereinafter ADC) installed on the upper surface of the applicator 1; a data processing unit 6; a data display unit 7.

The microprocessor for real-time processing of signals from sensors of physiological parameters of the local tissue region under the applicator and sensors of environmental parameters is an information processing unit 11.

In one of the preferred embodiments, information processing unit 6 is located on the upper surface of the applicator 1, in the other - it is remote and is wired with AD converter.

The information display unit 7 is a smartphone or a personal computer. In this connection, communication of the multi-sensor device with the information display unit 7 can be in various embodiments, both wireless and wired (via USB channel).

At least the osmotic pressure sensor 2, which helps determining the amount of water in the intercellular space of tissue in the local volume under the applicator at a controlled depth on which the local thermodynamic equilibrium is being achieved; the SC temperature sensor 4, which allows determining the temperature of skin surface under applicator, the sensor of the elastic pressure of tissue under the applicator is used as sensors for measuring the dynamics of physiological parameters of the local tissue area under applicator.

The signals from the sensors of physiological parameters (osmotic pressure sensor 2, temperature sensor 3 and elastic pressure sensor 4) located on the inner surface of the applicator 1 are transferred to the terminals of instrumental amplifiers and/or an analog-to-digital converter (unit 10), after which they are transferred to the data processing unit 11 for signal processing. Processed information is transmitted to the display device 12, on the screen of which the current values of the measured parameters and / or metabolic rate of the parameters are displayed in real time.

In case of using electromagnetic radiation with amplitude signal modulation, in order to increase the signal-to-noise ratio, the method of coherent detection of AM signals is used, which can significantly increase the signal-to-noise ratio of the recorded signal and reduce its distortion. In this case, coherent detection of the signal at the output of the osmotic pressure sensor is carried out using an instrumentation amplifier (with a built-in synchronous detection circuit) at the AM frequency of the electromagnetic radiation modulation.

Fig. 44. Diffuse reflectance spectroscopy scheme for determining the osmotic pressure of the intercellular substance based on spectral measurement of the temporal dynamics of the amount of water in the stratum corneum at a wavelength corresponding to the characteristic frequency of water (1190, 1455, 1945, 945 nm). The radiation diffusely reflected from the epidermis is collected at a wide angle and transmitted to the detector. 1 - radiation source at a wavelength corresponding to the characteristic frequency of water in the stratum corneum; 2 - heat and waterproof applicator; 3- radiation at a wavelength corresponding to the characteristic frequency of the studied biochemical component of the epidermis (living tissue); 4- receiver of electromagnetic radiation.

For practical implementation of the multi-sensor device, industrial certified sensors for measuring climatic parameters, as well as certified sensors for measuring the temperature of the stratum corneum and the elastic pressure of the epidermis can be used. For example, Honeywell sensors can be used as sensors for measuring the temperature of the stratum corneum.

Piezoelectric element-based sensors can be used as a sensor for measuring elastic pressure.

The osmotic pressure sensor (2), which registers the dynamics of water transfer in the epidermis by recording the temporal dynamics of the water content in the stratum corneum, can be based on different physical and chemical methods based on different physical principles. In particular, the following methods are applicable to measure the amount of water in the stratum corneum -electrometry methods based on measuring the electrophysical characteristics of SC (electrical conductivity, dielectric constant); spectral methods based on measurement of spectral characteristics (reflection and absorption coefficients); optical- acoustic methods; thermophysical methods based on measurement of thermophysical characteristics (thermal conductivity, heat capacity); electrochemical methods, etc. Also, sensor for measuring the amount of water can be based on the measurement of elastic pressure. Devices for measuring the amount of water in the epidermis are described in the patent [10].

In case of using an electrometric sensor, the amount of water in the epidermis can be determined by measuring electrical characteristics (transverse electrical conductivity and/or permittivity) of the stratum corneum on AC or DC current.

If the spectral sensor is used, the amount of water in epidermis can be determined by measuring the spectral characteristics (reflection coefficient and/or absorption coefficient) of the stratum corneum for electromagnetic radiation with a wavelength near the absorption line due to water content in the SC. In particular, temporal dynamics of the process of swelling of intercellular substance can be recorded according to the temporal dynamics of the coefficient of reflection of the stratum corneum. Increase in the amount of water in the intercellular space leads to a change in the spectral characteristic of SC, caused by a change in the amount of water in SC.

The spectral sensor may be based on a method in which the osmotic pressure of the intercellular substance or the amount of water in the intercellular space is determined by measuring the spectral characteristics of the intercellular substance in the range of the electromagnetic radiation spectrum, which is determined based on the characteristic absorption frequencies of the intercellular substance, and is selected from the group including characteristic frequencies of water in the optical and/or infrared and/or microwave range; group frequencies of hyaluronic acid of the intercellular substance in the infrared and/or terahertz and/or microwave range.

In another variant, the spectral sensor is based on the method for determining the osmotic pressure of the intercellular substance by measuring the amount of water in the stratum corneum based on the physical characteristics of the stratum corneum, which are selected from the group including electrophysical characteristics, spectral and optical-acoustic characteristics, thermophysical characteristics.

In the spectral method, the osmotic pressure of the intercellular substance or the amount of water in the intercellular space is determined based on the amount of water in the stratum corneum by measuring the spectral characteristics of the stratum corneum at the characteristic frequencies of water absorption in the stratum corneum using a spectral method, selected from the group including IR spectroscopy, Raman spectroscopy, optical-acoustic spectroscopy, differential double-beam spectroscopy.

The dosage calibration action is selected from the group consisting of external pressure, local decompression, heating, cooling, electric current or voltage and magnetic field.

For example, the device that generates pulses of electrical voltage or current through SC can be used as a device for creating a calibration effect, such device can determine current value and direction of microcurrents of tissue fluid in the intercellular substance in the deep layer of epidermis, by measuring the kinetic characteristics of mass transfer caused by electro-kinetic phenomena and more particularly by electro-osmosis.

In alternative embodiments, the device for generating calibration action is a thermal power source made in the form of a resistor or Peltier element, or a device for creating a dosed pressure on the surface of the applicator.

Industrial laser diodes (based on quantum-well heterostructures and other types) can be used as sources of electromagnetic radiation in the optical and infrared range; they are widely used and are produced in a large variety of irradiation characteristics, tailor-made for specific application. There are no official standards for the types and sizes of laser diode housings, but sometimes large manufacturers agree to unify packages. In addition, there are services for the development of the emitter housing according to customer requirements.

Industrial photo receivers can be used as receivers of electromagnetic radiation in the optical and infrared range. The leading worldwide companies produce quite a few types of microcircuits in which the radiation receiver (photodetector) and amplifier are combined in one housing. Such systems are widely used in IR remote control systems (such modules are also called IR receivers). The leading worldwide companies produce quite a few types of such microcircuits.

Domestic industry, for example, LED Microsensor NT LLC and IBSG Co., Ltd., produce a wide range of mid-infrared optoelectronic devices - light emitting diodes, LED arrays, photodiodes for the spectral range from 1,6 to 5,0 µm, as well as compatible electronic devices (drivers for LEDs and amplifiers for photodiodes).

In the optical and near-IR range, domestic LEDs can also be used: SIM-012ST (950-970 nm); OIS-150-1020 (1020-1060 nm); KM2520F3C03 (930-950 nm); EDEF-1LS3 (740-760 nm); EDEI-1LS3 (830-850 nm). A photodiode sensitive to optical radiation in the wavelength range from 740 to 1060 nm, for example, a BPW34 type photodiode, is used as an optical radiation receiver.

Miniature sources and receivers of terahertz radiation with radiation characteristics acceptable for applications in practice, have not yet been produced by the world industry and are at the stage of research and development. In particular, scientists fr om Princeton University (Xue Wu; Kaushik Sengupta, Department of Electrical Engineering, Integrated Micro-systems Research Lab, Princeton University, Princeton, NJ, USA) have achieved a radical reduction in the size of equipment for processing of terahertz electromagnetic radiation. Such devices can become an important element of medical diagnostics, communications and drug development. There are two publications on this topic in the IEEE Journal of SolidState Circuits. In the article, the scientists explained how they managed to create a miniature microcircuit for generating terahertz radiation. It still works in a narrow range, but the researchers realized that this issue can be solved by creating several waves of different lengths at once on a single chip and combining them using precise synchronization. Daniel Mittleman, vice chairman of the Society of Infrared, Millimeter, and Terahertz Waves, said that the work is "highly innovative and potentially has many applications" and also noted that there is still a lot of work to be done before these projects will be implemented in routine devices, but they are promising.

The heat- and waterproof applicator (1) with sensors of intercellular osmotic pressure 2 (or sensors of water ammount in stratum corneum) fixed to its lower surface, temperature 3 and elastic pressure of the epidermis on the applicator 4, is fixed on the surface of the stratum corneum, for example, on the surface of the hand, with external dosage pressure and is attached in one embodiment using straps. It follows that the measurement can be made on any part of the surface of the human body, free from the hair, with the exception of the areas of the body where the sweat glands are located, which are activated as a result of emotional impact. Palms of hands are the example of such area.

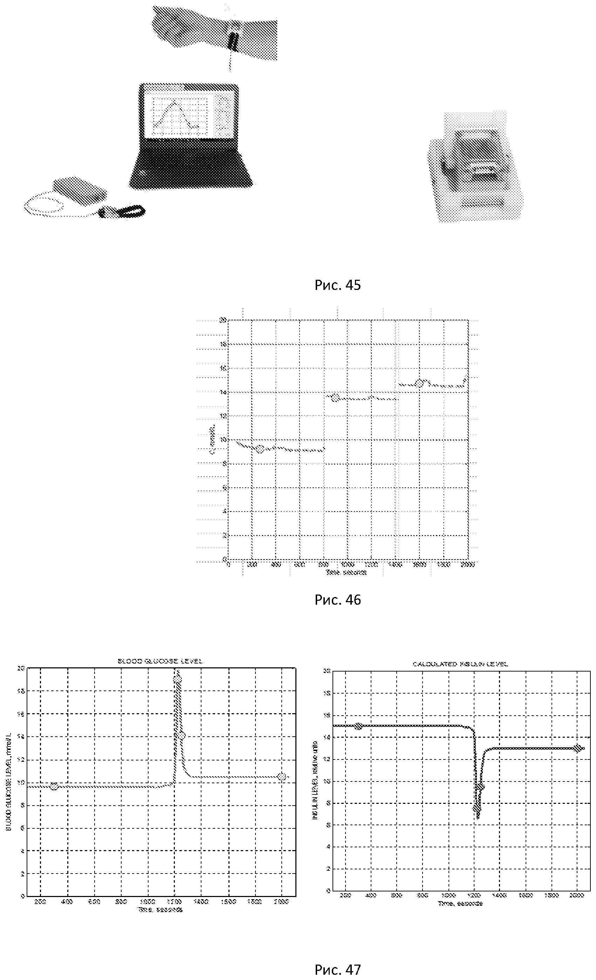

Provisional dimensions, appearance and design of the multisensor device are shown in Fig. 45.

Process of measuring (signals recording) starts immediately at the moment of pressing at least one heat- and waterproof applicator to the SC surface or with 10-50 seconds delay from the moment of pressing.

Temporal dynamics of physiological parameters in the local closed tissue area under the applicator is measured in the "microcalorimeter" mode after applying the applicator to the skin surface, in particular, at least the following parameters are being measured - heat flow through the epidermis area under the applicator or surface temperature of the skin under the applicator, elastic pressure of tissue under the applicator, pressure created by the applicator on the surface of the skin, amount of water in the intercellular space of tissue at a controlled depth at the local thermodynamic equilibrium level. Heat flux is measured through a patch of epidermis under the applicator or a temperature at a controlled depth of Tₛₖᵢₙ, wherein local thermodynamic equilibrium is established, at a fixed pressure Pₛₑₙₛₒᵣ of applicator.

To calculate blood glucose level, a calibration procedure is carried out and calibration parameters are determined to obtain the constant coefficients necessary for calculation of the level of glucose in blood and the thermal effect of metabolism of the local area of living tissue.

The number of sensors of the multi-sensor device described above can be increased by including additional sensors and sensors in the device that characterize the metabolism of a local area of living tissue and more particularly to blood biochemical parameters (for example, lactate in the blood), blood acidity, frequency of cardiovascular contractions. Additional sensors for through-cut measurements can be installed on the inside of the applicator and integrated with the physiological and climatic parameters sensors described above into a single measuring circuit as part of a multi-sensor device.

### Methods for early diagnosis of diabetes mellitus.

**A method for diagnosing diabetes mellitus based on the changes in the glucose curve.** Modern guidelines define Impaired Glucose Tolerance (IGT) as blood glucose concentrations (according to the oral glucose tolerance test results, 2 hours after intake of 75 g of glucose - from 7,8 to 11,0 mmol/L) between normal and diabetic values; the glucose concentration during the tolerance test is measured invasively using a glucometer with several blood samples taken during the oral test. IGT is thought to be a sign of a pre-diabetic state, although not all individuals with IGT develop diabetes. In USA every tenth adult individual has IGT the rate thereof increasing with age achieving every fourth among persons aged from 65 to 74 years.

The abovedescribed method of isothermal calorimetry of living tissue is a promising approach for creating methods for the early diagnosis of diabetes mellitus, in particular, it allows to determine the temporal changes of blood glucose in IGT in the continuous monitoring mode (glucose monitoring curve). Fig. 46a shows a characteristic continuous non-invasive glucose monitoring curve in a healthy patient in IGT (1 measurement per second), which differs from the glucose monitoring curves in patients with diabetes mellitus and is characterized by an increase in blood glucose concentration (approximately 30 minutes) and a decreasing section (approximately 30 minutes).

A clinical study in a group of patients with diabetes mellitus showed that the glucose monitoring curves in diabetics, recorded continuously (1 measurement per second) using a prototype isothermal microcalorimeter, demonstrate stepwise (step-like, jumping) changes. Characteristic glucose monitoring curves with a stepwise temporal dynamic, recorded in a clinical study, are show in figure 46b. The blood glucose level in patient D with type 1 diabetes mellitus (33 years old, male) changes spontaneously in a stepwise manner at 800 sec and 1400 sec time points.

It is well-known that blood glucose level in healthy subjects is determined by the level of insulin, which is produced by the pancreatic cells proportionally to the level of glucose in blood, which as a rule shows monotonous changes without spikes. Abnormal changes in the pancreas functions can lead to a nonhomogeneous stepwise flow of insulin into blood and, as a result, to spiking of blood glucose. Figure 46c shows the abrupt dynamics of blood glucose level in the form of a single pulse, recorded using an isothermal microcalorimeter prototype, and the temporal dynamics of insulin levels in the blood, calculated on the basis of a glucose curve.

The study showed that the stepwise changes in blood glucose level can lead to the stepwise changes in sugar concentration in the interstitial fluid in the form of sequential single impulses; a characteristic glucose curve is shown in Figure 47. Figure 47 shows characteristic glucose curves (a, b) with abrupt temporal dynamics in a patient with type 2 diabetes mellitus (66 years old, male): characteristic dynamics of blood glucose level in the form of a time sequence of single pulses.

The method of blood glucose monitoring based on the method of isothermal calorimetry can be applied in practice for the early diagnosis of diabetes mellitus (types 1 and 2) according to the features of the stepwise changes in the glucose monitoring curve.

### A method for diagnosing diabetes mellitus based on living tissue enthalpy monitoring.

The method of isothermal microcalorimetry of living tissue enables early detection of the diabetes mellitus (pre-diabetes) in patients based on the content of hyaluronic acid in the epidermis, through monitoring of the osmotic pressure of the intercellular substance (or the amount of water in the intercellular space).

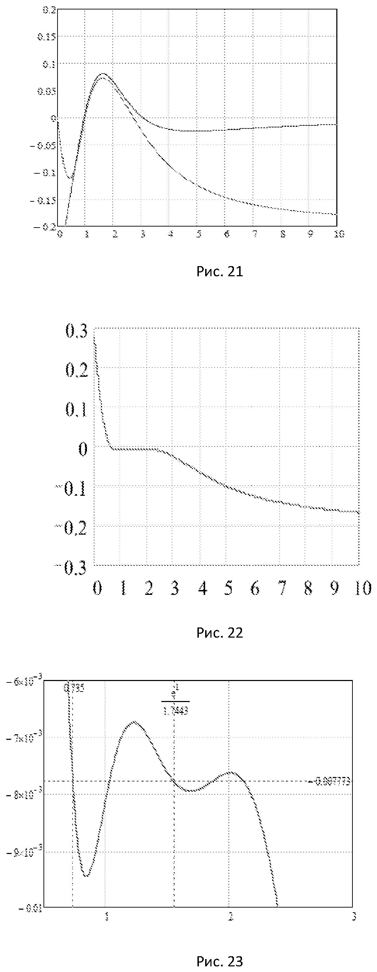

In a clinical study with the use of a prototype isothermal microcalorimeter of living tissue, we have found that the value of the enthalpy of the intercellular substance in virtually healthy patients, determined experimentally (at a specified power of external heat flow due to climatic parameters of the environment) based on a comparison of non-invasive prototype readings with invasive certified blood glucose meter readings (calibration method) do not change or change very slowly over time, with a characteristic time constant exceeding than one month or more. The result obtained experimentally is in good agreement with the conclusions obtained on the basis of a theoretical study of the thermodynamics of the biopolymer molecule in the condensed state, described above, which predict the stability of the enthalpy value in the range of parameters, corresponding to the physiological normal value, fig. 22, 23. The intercellular substance within the physiological normal range, which is determined by the critical value of the concentration of disaccharides (monomers) of hyaluronic acid (the critical contour length of the polymer chain), at physiological temperature, is in a heterophase state, in which the crystalline (globular) phase of the substance is in thermodynamic equilibrium with liquid (molten globule) phase, in which the concentration of monomers corresponds to the concentration of a saturated solution of the polymer chain of hyaluronic acid. The enthalpy of the intercellular substance of the patient is determined by the content of hyaluronic acid, which can change in chronic diseases. In particular, in diabetes, the content of hyaluronic acid may change as a result of changes in the contour length (number of disaccharides) of the polymer chain; therefore, the content of hyaluronic acid in the intercellular substance is a biochemical parameter, the monitoring of which enables early diagnosis of diabetes mellitus.

An increase in the number of links in the polymer chain, equivalent to the introduction of an additional amount of hyaluronic acid disaccharides into the solvent, at a fixed volume of the substance, does not lead to an increase in its concentration in the liquid phase (solution supersaturation) - but leads to an increase in the number of monomers in the crystalline phase during crystallization (process separation from the solid phase solution). An increase in the volume of a substance due to an increase in the volume of a solvent (water containing glucose and sodium ions) will lead to a process of reverse crystallization - dissolution (transition of a substance from a crystalline to a liquid phase). The dissolution process, as well as melting, leads to the destruction of the crystal mesh, which is usually accompanied by cooling of the solution. Dissolution can occur under isothermal conditions, but in this case, heat must be supplied from outside.

A clinical study on a group of patients with diabetes mellitus showed that the value of the enthalpy of the intercellular substance, determined by comparing the non-invasive readings of the device prototype with the invasive readings of a certified glucometer (calibration method), in patients with type 2 diabetes mellitus can have abnormal values - abnormally low or abnormally high; thus, the enthalpy of the intercellular substance of the living tissue of patients with diabetes mellitus is characterized by 2 values of enthalpy corresponding to 2 different states of the intercellular substance with different enthalpy values, which in some patients may differ by more than an order of magnitude.

Study data allowed to conduct in-depth analysis with a conclusion that the abnormally high value of the enthalpy of the intercellular substance observed in patients with diabetes mellitus is associated with an increased content (excess) of hyaluronic acid in the intercellular substance of the epidermis of the diabetic subjects. It is well-known that in type 2 diabetes mellitus, the content of hyaluronic acid (HA) in the intercellular substance increases, as a result of an increase in the rate of its synthesis in hyperglycemia (elevated blood glucose level) leading to the production of an excess amount of hyaluronic acid in the crystalline (globular) phase of the intercellular substance of the epidermis, the upper layer of which is characterized by a low solvent content in the intercellular space.

A change in the amount of solvent in the intercellular space of the tissue under the applicator, in the process of transition to a state of local thermodynamic equilibrium, can lead to a phase transition of a substance from a crystalline (globular) to a liquid (molten globule) state with an increase in the intercellular volume as a result of the dissolution of the crystalline (globular) phase, which is in a metastable state; the process of dissolution due to the supply of heat into the volume of tissue under the applicator through the interstitial fluid flow. The disruption of the thermodynamic phase equilibrium of the intercellular substance can also be due to the abrupt changes in the concentration of glucose and mineral ions in blood.

A clinical study in subjects with type 2 diabetes mellitus showed a correlation between the enthalpy value of the intercellular substance and tissue insulin resistance: in type 2 diabetes with high insulin resistance, abnormally low tissue enthalpy values were observed, corresponding to the predominance of the crystalline phase in the intercellular substance; an increase in the proportion of the crystalline component in the substance leads to an increase in the resistance of the intercellular space, due to an increase in the transfer coefficients of the substance for glucose and insulin transfer from the capillary to the cell and an increase in the proportion of bound glucose. In the physical state (molten globule) with an abnormally high level of osmotic pressure, the proportion of free glucose in the local volume of the intercellular substance can increase due to a decrease in the proportion of bound glucose in the crystalline phase without increasing the concentration of insulin and; as a result, it can lead to an increase in the concentration of glucose in the intercellular fluid.

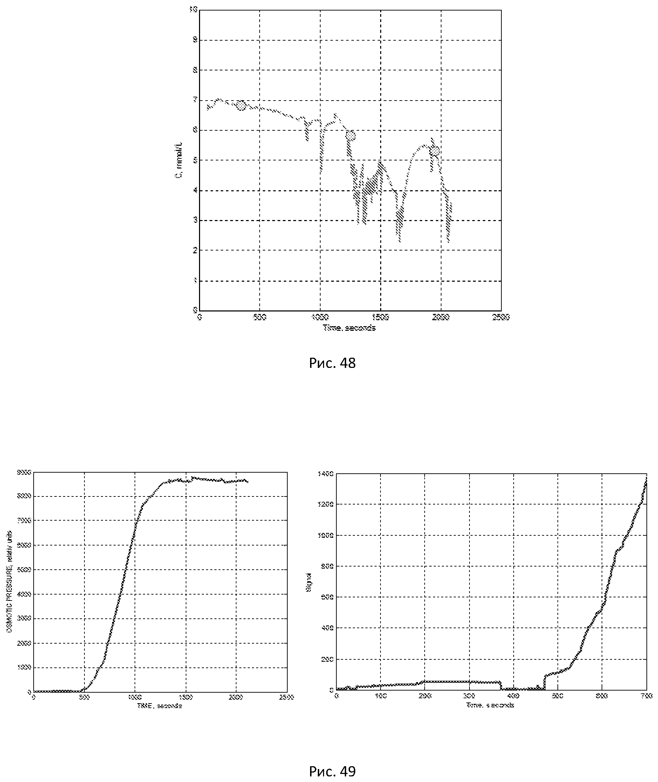

The value of the enthalpy of the intercellular substance, therefore, is an individual characteristic of the patient's muscle tissue, which has a high diagnostic value. Continuous measurement of the intercellular substance enthalpy by monitoring the content of hyaluronic acid and/or monitoring the osmotic pressure during insulin (or other sugar-lowering) therapy can be used in practice for the early diagnosis of diabetes, as well as for real-time diagnosis of insulin resistance in living tissue of patients with diabetes mellitus (and monitoring the effectiveness of sugar-lowering treatment) based on the monitoring of the content of hyaluronic acid in the epidermis. Fig. 49 shows the characteristic changes in the osmotic pressure of the intercellular substance with time (characterizing the amount of water in the intercellular space), which is recorded in the process of spontaneous transition of the intercellular substance of living tissue under the applicator into the state of local thermodynamic equilibrium (corresponding to the maximum entropy); transition of a living tissue into a new state with a high enthalpy occurs as a result of the process of dissolution of the crystalline (globular) phase of the intercellular substance due to the supply of heat by the flow of the intercellular fluid, and is accompanied by an increase in the volume and osmotic pressure of the intercellular substance. Osmotic pressure of the intercellular substance, corresponding to the equilibrium value of enthalpy, which is established during the transition of the tissue under the applicator to the state of local thermodynamic equilibrium, exceeds the initial value of osmotic pressure (corresponding to the initial value of enthalpy) by more than two orders of magnitude (100-fold). The reason for the phase transition of the intercellular substance can be changes in blood glucose level and probably, other biochemical and physiological parameters, as well as other external physical factors (pressure, heat flux, etc.).

It is well-known that in insulin resistance, the sensitivity of muscle cells to insulin decreases. As a result, the ability of glucose to penetrate into muscle cells is impaired. Glucose is accumulated in blood plasma. As a result, the pancreas produces even more insulin, and its level in blood increases above normal threshold. Finally, glucose is deposited in the form of fat, because fat cells are highly susceptible to insulin, and glucose needs to go somewhere from blood. A vicious circle is formed.

Due to an increased rate of synthesis of hyaluronic acid an abnormal content (excess) of hyaluronic acid in the intercellular substance in diabetic patients leads to crystallization of the intercellular substance, which is accompanied by an increased binding of glucose in the crystalline phase and an increase in the transfer coefficients of the intercellular substance due to the need for glucose and insulin transfer from the capillary into the cell; therefore it may be one of the causes of development of type 2 diabetes mellitus and other chronic cardiovascular diseases associated with diabetes. The phase transition of the intercellular substance into an aggregate state with an abnormally high osmotic pressure also leads to an increase in insulin resistance and is accompanied by an elevation of blood pressure due to compression (collapse) of the capillaries as a result of an increase in the osmotic pressure of the substance surrounding the capillary.

Thus, the method of isothermal calorimetry enables early detection and prediction of signs of insulin resistance and diabetes in subjects not diagnosed with type 2 diabetes, based on the nature of the temporal changes of the osmotic pressure of the intercellular substance. A method of isothermal calorimetric spectroscopy described above also allows to detect diabetes mellitus at an early stage on the basis of spectral measurements of the content of hyaluronic acid in the local volume of living tissue.

Jumps in the osmotic pressure of the intercellular substance, which are observed in patients with diabetes mellitus, can lead to jumps in blood pressure. In particular, jumps in the osmotic pressure of the extracellular substance in heart and brain can be a possible cause of heart attacks and strokes. Osmotic pressure jumps in the pancreas can cause a non-homogeneous concentration of insulin in blood, as a result of the stepwise changes in the rate of insulin production in the pancreas. Therefore, a conclusion is understood about the nature of the relationship between diabetes mellitus and cardiovascular disease, which is now clinically proven. The method of isothermal calorimetry described above also allows to diagnose cardiovascular diseases caused by diabetes mellitus at an early stage.

The impact of some external factors, physiological and physical, on the tissue can cause reversible transitions of living tissue from one phase state to another. These factors include, in particular, muscle load and temperature effects. External effects, which cause reversible transitions of the tissue under study, include the effects which lead to reversible changes in a phase state of the intercellular substance. The number of such external physical factors includes the following: an external pressure; a local decompression; an external temperature; electromagnetic radiation causing volume heating of a tissue; a weak direct electric current; a constant magnetic field; a local muscular load to a tissue and others. A method of isothermal microcalorimetry, supplemented by the effect of an external physical factor, allows to determine the value of enthalpy and diagnose diabetes mellitus at an early stage, on the basis of measuring the correlation between osmotic pressure and an external physical factor.

### Impaired glucose tolerance.

The method of isothermal calorimetry of living tissue described above allows to detect IGT and tissue sensitivity to insulin based on the nature of the changes in the osmotic pressure of the intercellular substance (the amount of water in the intercellular space) with time, without determining the absolute value of enthalpy (without calibration). In a clinical study in patient with type 2 diabetes mellitus, we have found a characteristic stepwise changes in the osmotic pressure of the intercellular substance due to the stepwise changes in glucose concentration in the intercellular substance with tissue insulin resistance: in patients with type 2 diabetes mellitus with high insulin resistance, and abnormally low tissue enthalpy values were observed, corresponding to the predominance of the crystalline phase in the intercellular substance.

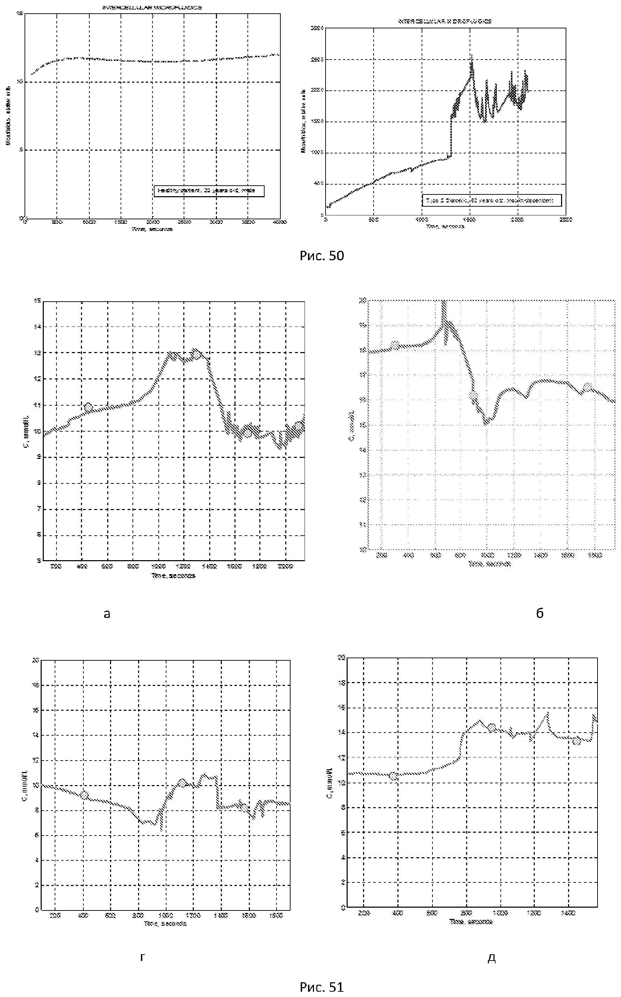

Fig. 50 Characteristic monitoring curves of the osmotic pressure of the intercellular substance of a healthy patient and a patient with type 2 diabetes mellitus. Temporal changes in the osmotic pressure in a patient with diabetes mellitus has a stepwise pattern due to the stepwise changes in glucose concentration in the intercellular substance and in blood, in contrast to the temporal changes in a healthy patient, which is characterized by smooth monotonous changes due to the smooth monotonous changes in the glucose concentration in the intercellular fluid and blood.

A consequence of the stepwise changes in the osmotic pressure of the intercellular substance, which is important for practical medicine, is the step-like nature of blood pressure. Osmotic pressure monitoring method is applicable for early diagnosis of cardiovascular diseases, in particular, hypertension.

The method of isothermal calorimetric spectroscopy described above also allows to detect diabetes mellitus and associated cardiovascular diseases at an early stage on the basis if spectral measurements of the content of hyaluronic acid in a local volume of living tissue.

### A diagnostic method for tracking nutrition by assessing calorie intake.

The epidemiological studies carried out in different countries indicate to a close relation between IGT and obesity. For example, the study carried out in the USA, has found that a mean EBW (excessive body weight) in persons, who consequently developed IGT, was significantly higher than in individuals with a normal EBW. The study carried out in Israel has established that a history of a high EBW was accompanied by a raised frequency of IGT development over a 10-year period.

The method of isothermal calorimetric spectroscopy described above allows to determine the energy value of food intake based on spectral measurements of the glucose and free fatty acid triglicerydes content in the local volume of living tissue. Energy value or calorie intake is the amount of energy released in the human body from food during digestion after its full absorption. The energy value of a product is measured in kilocalories (kcal) or kiloJoules (kJ) per 100 g of product. Kilocalories, which are used to measure the energy content of food, is also called the "food calorie," so the prefix "kilo" is often omitted when referring the energy value in kilocalories. The nutritional value of a product is the content of carbohydrates, fats and proteins per 100 grams of the product.

### Skin condition diagnostic method.

The method of isothermal calorimetry and spectroscopy described above allows to determine the physiological state of the skin based on spectral measurements of the content of hyaluronic acid and free fatty acid triglycerides (and other biochemical
components) in a local living tissue unit volume.

### Examples of practical use. Clinical study data.

The appearance of the prototype of the isothermal calorimeter of living tissue, which was used in clinical study to prove the method (proof of concept study) is shown in Fig. 46.

Assessment of the effectiveness of the method was performed by two different methods described in the clinical trial protocol. A group of 41 patients with diabetes mellitus (types 1 and 2) was divided into two subgroups of 27 patients (subgroup 1) and 14 patients (subgroup 2), each including patients with type 1 and 2 diabetes mellitus. Random sample of patients.

The purpose of the study, involving 27 patients from the first subgroup, was to confirm the effectiveness of the method, to study the reproducibility of test results and to assess the possibility of individual calibration. Each of the patients underwent 5 - 6 tests with 35-minute duration of each test (with three invasive measurements), with an average of 3 tests per day. In total 144 tests were performed on 27 patients with type 1 and 2 diabetes mellitus, with a total of 434 invasive measurements.

Figure 51 (a - e) shows examples of glycemia curves with stepwise temporal changes: 51a (test No. 6, patient No. 24), Fig. 516 (test No. 1 patient No. 41), Fig. 51c (test No. 5 patient No. 41), Fig. 51g (test No. 6 patient No. 41). All graphs present the results of invasive measurements with green circles, and the results of non-invasive monitoring are shown with a purple curve.

The indicator of the effectiveness in the first assessment method is the correlation, with 15% bias interval, of the results of non-invasive measurements with the results of invasive measurements, at fixed values of the individual scale coefficient of the given patient (individual calibration).

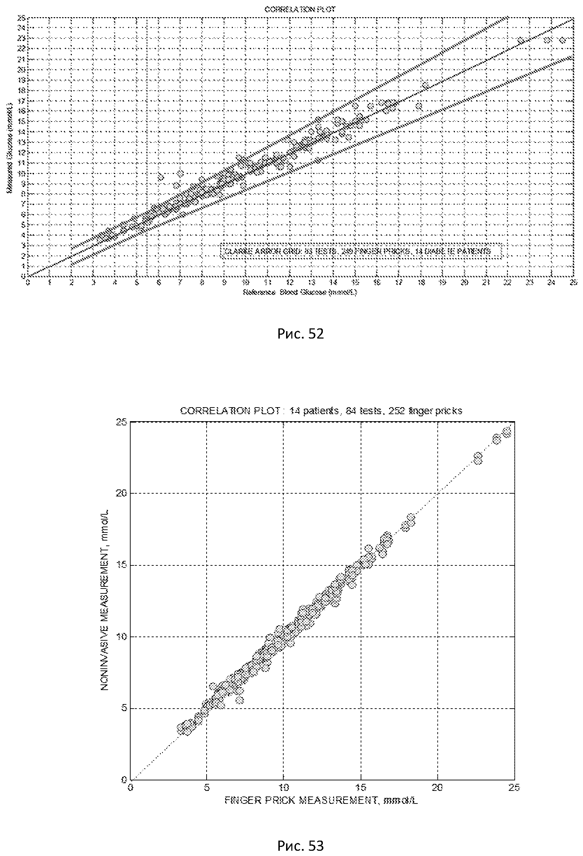

The study showed that each patient with diabetes mellitus is characterized by two values of the individual scale coefficient K01 and K02, which have a constant individual value for this patient; however, the scale coefficient varies over a wide range of values between patients from K01 = 0.7 (for patient No 1) to K01 = 480 (for patient No 25). Test results in subgroup 1 are shown in Fig. 52.

Test results in subgroup 1 (27 patients) are shown in Fig. 52.

In accordance with the international standard ISO 15197 (section 6.3.3. Minimum system accuracy performance criteria; Data analysis of clinical study according to Clause 6.3.3 of ISO 15197:2013 [p 12, pdf]) following criteria for assessing the accuracy of invasive glucometers have been adopted:
95% of the measurement results must fall within area A, the boundaries of which are determined based on the following conditions:
± 15% for values of glycemia equal to or greater than 5.55 mmol/liter;
± 0.83 mmol/liter for values less than 5.55 mmol/liter;

The purpose of the study, involving 14 patients from the second subgroup, was to evaluate the effectiveness of the blind measurement method. Each patient underwent 6 tests with 35-minute duration (with three invasive measurements) within two days, of which the results of the first 3 tests (1,2,3) were used for calibration. For each subject, an individual calibration was carried out, during which the values of the individual scale factors of this patient K01 and K02 were determined based on the results of the first 3 tests (each lasting 35 minutes).

The next 3 tests (4, 5, 6) on the next day were performed and processed using blinded method. In total, 83 tests were performed on 14 patients with type 1 and type 2 diabetes mellitus, with a total of 249 invasive measurements. Test results are shown in Fig. 53m. Test results in subgroup 2: 14 patients with type 1 and type 2 diabetes mellitus; 83 tests with a total of 249 invasive measurements. Curves in red color define zone A. Zone A includes 246 measurements out of 249 or 98% of the results.

Therefore, the results of comparative measurements on patients from subgroup 2 using semi-blind and blind methods, are in accordance with the requirements of the ISO 15197 standard for accuracy. Test results in a group of 41 patients (227 tests, 683 invasive measurements), obtained by pooling the results in subgroups 1 and 2, are shown in Fig. 53.

The pooled test results: 42 patients with type 1 and 2 diabetes mellitus; 227 tests with a total of 683 invasive measurements. Red curves indicate zone A, brown curves indicate zone B (± 25%).

Only 654 measurements out of 664 or 98.3% of the results fall into the A + B zone.

Therefore, the results of a clinical study in a group of 41 patients with type 1 and 2 diabetes mellitus are in accordance with the requirements of the ISO 15197 standard for accuracy.

### The protein folding problem.

Protein folding is the process of spatial packaging of a protein molecule, transition of a protein into a strictly defined spatial form in which it realizes its biological functions; the process of self-organization of a protein molecule - spontaneous folding of a polymer chain under specific conditions into a certain spatial structure, is shown schematically in Fig.1.

The ability of a natural polypeptide chain to spatially self-organize and acquire a specific molecular structure, which is the most striking feature of proteins. The process of self-organization of proteins is still largely unclear and represents one of the biggest problems of modern science. It is known that the functions of proteins can mostly be predicted based on their structure, although not always. Therefore, an urgent scientific problem is the prediction of the structure and, as a consequence, the function of a protein, according to the sequence of amino acids in the polymer chain.

Protein molecules are linear polymers consisting of amino acid residues (monomers); also, they may include modified amino acid residues and components of a non-amino acid nature, for example, glycoproteins characterized by a low carbohydrate content, a carbohydrate residue (oligosaccharide with an irregular structure and containing glucose, mannose, galactose and their amino derivatives, as well as N-acetylneuraminic acid) is attached to the amino acids of the protein chain either via N-glycosidic bond to the amide nitrogen of asparagine, or via O - glycosidic bond to the hydroxyl group of serine, threonine, hydroxylysine residues. When a protein is formed, as a result of the interaction of the α-carboxyl group (-COOH) of one amino acid with the α-amino group (-NH2) of another amino acid, peptide bonds are established. The ends of the protein molecule are called the N- and C-terminus, depending on which of the groups of the terminal amino acid residue is free: -NH2 or -COOH, respectively.

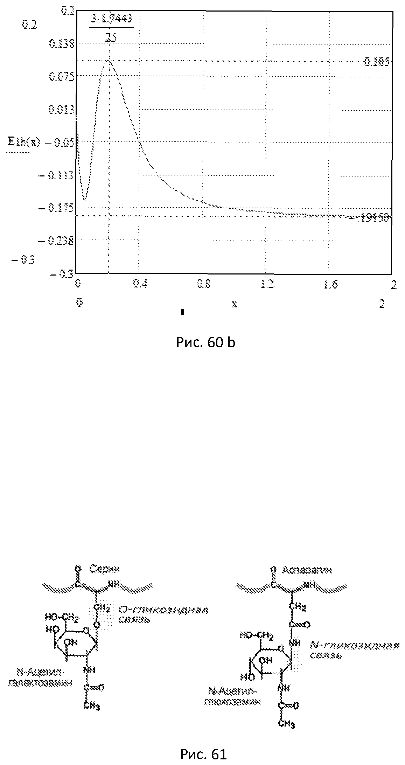

It is well known that the glucose molecule can also form a glycosidic valence bond with amino acids of protein molecules. In glycoproteins characterized by a low carbohydrate content, a carbohydrate residue (an oligosaccharide with irregular structure and containing glucose, mannose, galactose and their amino derivatives, as well as N-acetylneuraminic acid) is linked to the amino acids of the protein chain or to the amide nitrogen of asparagine via N-glycosidic bond, or to the hydroxyl group of serine, threonine, hydroxylysine residues via O-glycosidic bond. Structural formulae of glycoside bonds with amino acids are shown in fig. 61.

As it can be seen, the configuration of the tetramer in the biopolymer molecule of hyaluronic acid is formed with the participation of sodium ions, the concentration of which in the intercellular space is much higher than that of potassium ions.

On the other hand, the ionic composition of cells, as long as they exhibit the properties of a living being, differs from the ionic composition of the environment. The most significant difference is the asymmetric distribution of univalent Na⁺and K⁺ ions: cells actively accumulate K and release Na into the environment. This leads to a difference in concentration of
monovalent cations on the cell membrane.

This explains the conclusion that the monovalent metal ion M, which forms an ionic bond with the charged unit of the polymer chain -R_{b}-M of the protein molecules located in the plasmatic membrane and cytoplasm of the cell, is the monovalent ion K⁺: R_{b}-M → -R_{b}-K.

The universal model of a biopolymer molecule, on the basis of which the properties of a hyaluronic acid macromolecule are described, which, as noted, is a unique model object for assessment the properties of biopolymer molecules, allows to predict the thermodynamic properties of a random natural macromolecule, in particular, macromolecules of proteins and enzymes based on an accurate statistical description.

In general, the structure of a biopolymer protein molecule with a feature of spatial self-organization and the acquisition of a stable molecular heterophase structure due to weak volumetric interactions of a non-valence nature can be described by the following definition: a linear polypeptide chain of amino acids sequentially connected by an amide (peptide) bond -NH-CH( R)-CO-, the repeating monomer of which consists of two (or more) types of monomer units - ARₐ and AR_{b} amino acids, with different side residues Rₐ , R_{b} , one of which (for example, the AR_{b} unit) contains a charged (negatively or positively) R_{b} group, and the side residue Rₐ , of the second unit ARₐ is uncharged. The water-based solvent includes at least 3 components: one component is a (monomeric) molecule of the substrate (solvent) S, for example, glucose molecule, and two (or more) low molecular weight monovalent ions M₁ and M₂, for example sodium and potassium ions with different affinities to the monomer, the electric charge of which is inverse to the charge of the side residue AR_{b}, the monomeric solvent (substrate) molecule S can form two hydrogen bonds with the monomeric units of the polymer chain - Rₐ-S- R_{b}- ; -Rₐ-S-R_{b} -; - Rₐ-S- ; -S-R_{b} ; low molecular weight monovalent ions M₁ and M₂ can form an ionic bond with charged units of the polymer chain -R_{b}-M₁. The energy of the longitudinal covalent bonds E₀ of the adjacent links of the polymer chain is much higher than the characteristic thermal energy at physiological temperatures E₀/k_{B}T>>1. The energy of transverse hydrogen bonds of the Rₐ and R_{b} links with the monomer (substrate) molecule S of the solvent is much less than the energy of the longitudinal valence bonds E_{S} << E₀ of the polymer chain. The low molecular weight ion M₁ can be adsorbed by the polymer chain monomer R_{b}, with the formation of a weak ionic bond -R_{b}-M₁.

In general, the repeating fragment of the polymer chain can consist of the same pairs -A- B- or several types of repeating pairs of monomeric links -A-B-, -C-B-, ... that can form hydrogen bonds with substrate molecules.

Hydrogen bonds determining the most probable configuration of a bound pair of elementary chains, corresponding to the minimum free energy of volumetric interactions, and as a result, the most probable spatial configuration of a tetramer formed by a pair of non-adjacent polymer chain monomers and a pair of solvent molecules with the participation of ions can be defined as conjugated non-valence bonds or conjugated hydrogen bonds of a biopolymer molecule. An elementary cell corresponding to a bound pair of elementary chains (in its most probable configuration) forms a conjugated system - a closed system of conjugated hydrogen bonds with a delocalized electron density; It is well-known, a common feature of all conjugated systems is the spreading of the electron density of hydrogen bonds over the entire conjugated system.

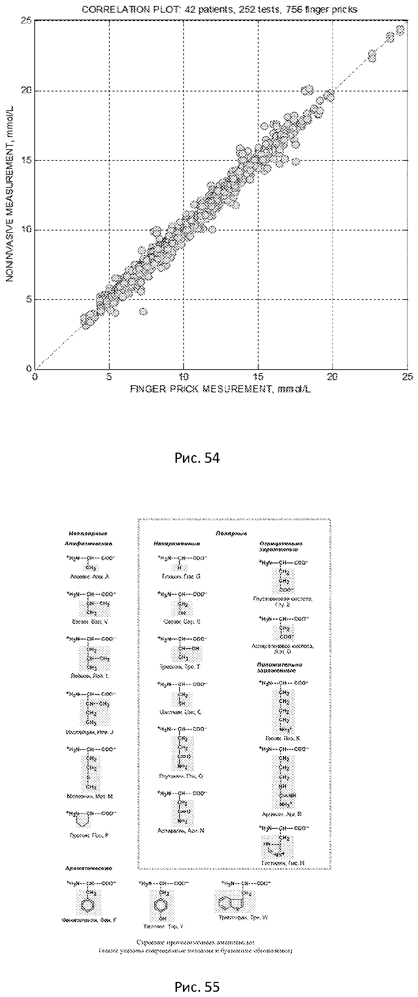

The structural formulas of amino acids that form polypeptide chains of protein molecules are shown in the table in Figure 55.

It is known that magnesium functions as a cofactor in over 300 known enzymatic reactions involved in a wide range of metabolic processes. Energy production, glucose metabolism, fatty acid oxidation and amino acid activation require magnesium. Magnesium is one of the most important minerals; being the main intracellular element, it activates enzymes that regulate carbohydrate metabolism, stimulates the formation of proteins, regulates the storage and release of energy in ATP, reduces excitation in nerve cells, and relaxes the heart muscle.

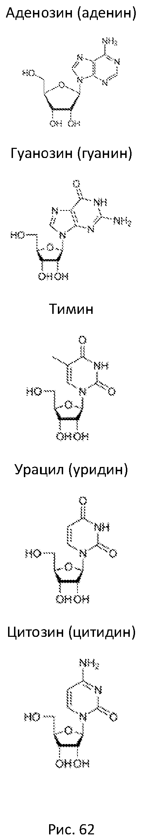

It was shown that a tetramer in the volume of a macromolecule, the polymer chain of which contains negatively charged groups, can be formed only with the participation of a monovalent ion (Na, K). A bivalent metal ion (Mg²⁺, Mn²⁺) cannot participate in the formation of a tetramer - a crosslink of 2 non-adjacent polymer chain monomers, 2 substrate molecules and 2 monovalent solvent cations, however, it can form a cross-link between two non-adjacent polymer chain monomers without participation of solvent molecules. Figure 62 schematically shows a crosslink formed by a bivalent magnesium ion between non-neighboring polymer chain monomers via an ionic bond.

*Common classification.* In accordance with the common classification, 4 levels of the structural organization of proteins can be distinguished: primary, secondary, tertiary and quaternary protein structures. The primary structure (sequence of amino acid residues) of a polypeptide is determined by the structure of its gene or genetic code, while higher-order structures are formed during protein folding. Although the spatial structure of a protein is generally determined by its amino acid sequence, it is quite labile and can depend on external conditions, so it is more correct to speak of the preferred or most energetically favorable protein conformation.

The primary structure is the sequence of amino acid residues in a polypeptide chain, which is usually described using one-letter or three-letter designations for amino acid residues. Important features of the primary structure are conservative motifs - stable combinations of amino acid residues with a specific function that are found in many proteins. Conservative motifs are preserved in the course of species evolution; they often allow to predict the function of an unknown protein.

Secondary structure - local arrangement of a fragment of a polypeptide chain, stabilized by hydrogen bonds. The following are some of the most common types of the secondary structure of protein: alpha helix (α - helix) - dense coils around the long axis of the molecule. One turn is 3,6 amino acid residues, the helix pitch is 0,54 nm (0,15 nm per amino acid residue). The helix is stabilized by hydrogen bonds between H and O peptide groups separated by 4 links; β - sheets (folded layers) - several zigzag polypeptide chains in which hydrogen bonds are formed between amino acids relatively distant from each other (0,34 nm per amino acid residue) in the primary structure or different protein chains (not adjacent, as in the α - helix).

Tertiary structure, the spatial structure of the polypeptide chain, is formed when the polypeptide is folded into a compact three-dimensional system (in the case of enzymes, this is usually a spherical globule). Structurally, it consists of secondary structural elements stabilized by various types of interactions, in which hydrophobic interactions play an important role.

As shown above, volumetric interactions of a simple polymer chain with a solvent can be described as a pairwise volumetric interaction of elementary chains forming a bonded pair, the most probable configuration of which is shown in Fig. 5a. The most probable configuration of interacting elementary chains is realized as a result of chain pairing via hydrogen bonds in the R₄ (*l* = 4) configuration of the tetramer; each elementary chain is in its most probable linear configuration with the maximum entropy corresponding to the minimum free energy of interaction between the chain and the solvent.

Investigations of the principles of protein folding have shown that it is convenient to identify and additional level between the secondary structure and the atomic spatial structure - the fold motif (architecture, structural motif). The folding motif is determined by the mutual arrangement of the elements of secondary structure (α - helixes and β - strands) within the protein domain, a compact globule that can either exist independently or be part of a larger protein along with other domains.

Quaternary structure (or subunit, domain) - the mutual arrangement of several polypeptide chains as part of a single protein complex. Protein molecules that make up a protein with a quaternary structure are formed separately on ribosomes and only after the synthesis is finished, they form a common supramolecular structure. A protein with a quaternary structure can contain both identical and different polypeptide chains. The same types of interactions participate in the stabilization of the quaternary structure as in the stabilization of the tertiary one. Supramolecular protein complexes may consist of dozens of molecules.

Protein domain, an element of the protein's tertiary structure, which is a fairly stable and independent substructure of the protein, the folding of which occurs independently of the other parts; the tertiary structure of a protein molecule is formed by folding a polypeptide into a compact three-dimensional system (in enzymes it is usually a spherical globule).

As a rule, the domain includes several elements of the secondary structure. Domains similar in structure are found not only in related proteins (for example, in the hemoglobins of different animals), but also in completely different proteins. Rather often, separate names are assigned to domains, since their presence directly affects the biological functions of the protein - for example, Ca²⁺ - binding domain of caldomulin, homeodomain, responsible for binding to DNA in various transcription factors, etc.

In oligomeric proteins, each of the subunit-forming polypeptide chains is characterized by its own secondary and tertiary structure. However, these proteins have another conformational level called a *quaternary structure*; this term describes *the position of the polypeptide chains that form individual polypeptide chains relative to each other, i.e. a method for their joint arrangement and packaging with the formation of a native conformation of an oligomeric protein.*

Since the domains are sufficiently "autonomous" in forming their structure and performing their function, with the help of genetic engineering it is possible to fuse a domain from another protein to the primary one (thus creating a chimeric protein). If you are lucky, such chimaera will combine the functions of both proteins. For example, by fusing the Cas9 DNA-binding domain with various regulatory domains, it was possible to obtain artificial transcription factors (crisprTF) selectively directed to the desired regions of the genome using tailor-made "RNA guides". Artificial restriction endonucleases, repressors, and epigenome-modifying enzymes such as DNA methylases and demethylases can also be constructed using Cas9.

### The method of calculation of a spatial structure of the protein.

The most probable linear configuration of the *elementary chain* differs from the *primary structure* configuration of the polymer chain and is formed as a result of the interaction of the polymer chain with the solvent in the equilibrium configuration of the macromolecule corresponding to the minimum free energy of spatial interactions. On the other hand, the volume of a substance in a condensed state, under the specified environmental conditions (pressure, temperature, and other characteristics), is determined only by the forces of volumetric interactions, so the three-dimensional spatial configuration of a biopolymer molecule can be calculated on the basis of the configuration of a *bound pair of elementary chains* that characterizes the equilibrium configuration of an elementary volumetric interactions.

In case of a biopolymer molecule with a simple polymer chain without subunits (protomers), the folding of a protein molecule - the process of formation of a secondary structure, with spatial packing into a compact globule, occurs spontaneously, as a phase transition of the statistical system of many interacting particles into a spatial configuration with a heterophase structure with minimum free energy of volumetric interactions. The intramolecular heterophasic structure and domain volume of a simple-chain protein molecule with a single subunit can be calculated based on the configuration of a linked pair of elementary interaction chains.

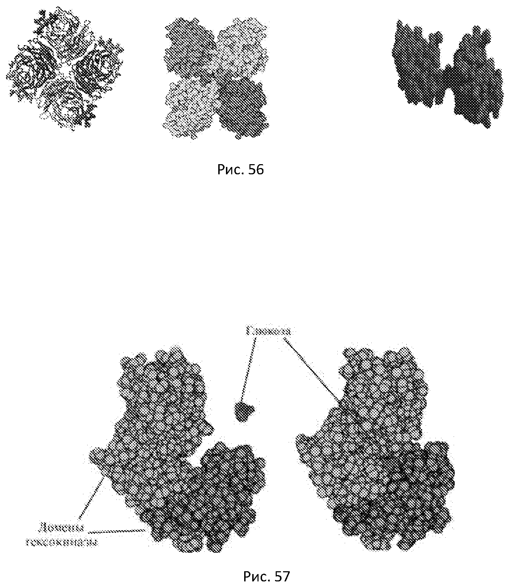

In general, the chain of a biopolymer molecule (for example, an oligomeric protein) may have a more complex primary structure, consisting of several interconnected simple chains (subunits, protomers). Oligomeric proteins - proteins containing 2 or more polypeptide chains (up to several thousand subunits). In oligomeric proteins, each of the subunit-forming polypeptide chains is characterized by its secondary and tertiary spatial structure. Examples of biopolymer molecules consisting of several subunits are shown in Figure 56.

Polypeptide chains in oligomeric proteins can be either identical or different. The number of polypeptide chains in an oligomeric protein can be determined by the number of amino-terminal residues per protein molecule. An oligomeric protein consisting of four polypeptide chains, for example hemoglobin, must have four terminal residues, one for each chain. The insulin molecule has two chains linked by covalent cross-links.

Some proteins (complex proteins), in addition to the polypeptide part, also contain a non-protein - prosthetic group, which can be associated with the protein part both with a covalent or weak non-covalent bond. Fig. 56 shows a quaternary structure - the mutual arrangement of protein subunits in space, characteristic of proteins consisting of several polypeptide chains; occurs as a result of the spatial association of several protein subunits. In oligomeric proteins, each of the subunit-forming polypeptide chains is characterized by its own secondary and tertiary structure.

### Interaction between macromolecule subunits.

Based on the current experience, the three-dimensional spatial structure of complex proteins consisting of several subunits can change in the process of interaction of polymer chains belonging to different subunits with solvent molecules. Fig. 57 shows a diagram explaining the effect of changing the conformation of a hexokinase enzyme molecule during glucose molecule binding.

Volumetric interaction between adjacent domains arises as a result of volumetric interactions between monomers (belonging to chain sections) from different subunits: a substrate molecule forms a hydrogen bond with two monomers that belong to different subunits and have different electric charge. A change in the three-dimensional spatial structure of the hexokinase protein molecule, which consists of two domains, occurs as a result of a phase transition of a substance in a local area between adjacent domains (corresponding to subunits) into a heterophase condensed state. The statistical mechanism of the transition of a substance in the region between adjacent domains to a heterophase state is described in the section Physics of the Condensed State of a Polymer Molecule above.

### Interaction between biomolecules.

In general, based on the experience, biopolymer molecules can interact not only with small molecules, but can form complexes with biomolecules (most often with a protein, for example, a cellular receptor, but sometimes, for example, with DNA) and exert some biochemical, physiological or pharmacological effects due to such binding. In biochemistry and pharmacology, a chemical compound (which is often, but not always, a small molecule) that forms a complex with a specific biomolecule is called a *ligand.* In case of binding a ligand to a protein, the ligand is typically a small signaling molecule that binds to a specific binding site on the target protein (eg., receptor). In case of ligand binding to DNA, the ligand is usually also a small molecule or ion or protein that is bound to a double DNA helix. Binding or association of a ligand to a receptor (the so-called "docking" of a ligand into a specific "niche" on the receptor) is usually reversible and short-term. The reverse process is called the dissociation of the ligand from the bond with the receptor.

In 80-90s, studies of biopolymer complexes resulting from the association of several macromolecules linked by non-valence bonds led to the development of a new interdisciplinary field of knowledge, called "supramolecular chemistry" - this is the chemistry of intermolecular bonds that studies the association of two or more chemical molecules, as well as the structure of such associates. It lies outside the boundaries of classical chemistry, which studies the structure, properties and transformations of individual molecules. If the latter deals mainly with reactions where cleavage and formation of valence bonds occur, then the objects of study of supramolecular chemistry are non-valence interactions - hydrogen bonding, electrostatic interactions, hydrophobic forces, "non-bound" structures.

The simplest example of supramolecular structures is host-guest complexes. The host (receptor) is usually a large organic molecule with a cavity in the center, and the guest is a simpler molecule or ion. For example, cyclic polyesters of various sizes (crown ethers) bind alkali metal ions quite strongly.

Supramolecular structures are characterized by the following properties: 1. the presence of not one, but several binding centers in the host (in crown ethers, this role is played by oxygen atoms with non-divided electron pairs); 2. complementarity: the geometric structures and electronic properties of the host and guest complement each other (in crown ethers, this manifests in the fact that the cavity diameter must correspond to the radius of the ion). Complementarity allows the host to selectively bind guests of a strictly defined structure. In supramolecular chemistry, this phenomenon is called "molecular recognition". 3. Complexes with a large number of bonds between complementary host and guest have a high structural organization.

An example of a supramolecular biochemical structure are host enzyme molecules. The active center of each enzyme is designed in such a way that it may host only the substance (substrate) that corresponds to it in size and energy; the enzyme will not react with other substrates. Another example of supramolecular biochemical structures are DNA molecules, in which two polynucleotide chains are complementarily linked to each other via many hydrogen bonds. Each chain is both a guest and a host for the another one.

The abovedescribed physics of the heterophase condensed state of biopolymer molecules enables interaction of molecules in supramolecular complexes. The interaction between macromolecules with the formation of supramolecular structures is based on the mechanism similar to that of interaction between subunits in an oligomeric protein, as a result of the formation of hydrogen bonds between the ligand and protein molecules and ionic bonds between the solvent ions and the charged regions of the polymer chain monomers.

### A method to determine the spatial structure of a complex macromolecule.

Three-dimensional domain spatial structure of a protein is calculated using the abovedescribed method and consists of the following steps:
- determine the number of subunits according to the number of amino-terminal residues;
- determine the primary structure of the polypeptide chain of each subunit (protomer);
- determine the structure of the prosthetic group;
- determine the structure of the elementary chain of interaction for each subunit; for this, determine an ion
- molecular pair of the solvent "substrate molecule - monovalent ion" for each subunit of the polymer chain;
- determine the tertiary structure of each independent domain; for this purpose, the equilibrium constant and activation energy are calculated for each subunit, as well as the heterophase structure and volume of the independent protein domain (the volume of localization of the subunit of the polymer chain);
- determine the location of each subunit in the chain of monomers that determine the interaction between the subunits;
- determine the structure of a connected pair of elementary chains that characterize the interaction between subunits.
- three-dimensional spatial configuration (quaternary structure) is defined as the most probable spatial configuration of independent domains, corresponding to the minimum free energy of volumetric interactions in the local region between domains.

### Heterophase biological catalysis.

A well-known effective method to accelerate the chemical reaction of inorganic molecules, which is widely used in practice, is heterophase catalysis - a change in the chemical reaction rate under the influence of catalysts that form an independent phase and are separated from the reacting substances by an interface. For example, the most common case is when a solid catalyst (contact) increases the rate of reaction between gaseous reactants or a reaction in solution. The catalytic reaction usually proceeds on the surface of a solid catalyst and is due to the activation of reagent molecules upon interaction with the surface. Therefore, for the implementation of heterophase catalysis, adsorption of the components of the reaction mixture from the volumetric phase on the catalyst surface is necessary.

Chemical catalysts that accelerate vital biochemical reactions (about ten thousand) in the cell are enzymes - protein molecules, the polymer (polypeptide) chain of which consists of amino acids.

The main features of enzymatic catalysis, which distinguish it from laboratory and industrial catalysis, are strict selectivity and a high rate of a chemical reaction.

Until recent time, it was believed that proteins are essential components of all enzymes. However, enzymes composed from molecules of ribonucleic acids (ribozymes) were discovered and became the object of intensive research. Interest in this group of enzymes has sharply increased with the development of methods for molecular selection of nucleic acids, which allows, in particular, to begin the targeted construction of ribozymes with various types of catalytic activity.

According to modern concepts (IUPAC), the specificity and catalytic activity of an enzyme molecule is determined by the active center - a special section of the molecule with a unique combination of amino acid residues (and possibly non-protein groups) that ensure direct binding of the enzyme molecule to the substrate molecule and direct participation in catalysis. The process of binding the substrate molecule by the active center of the enzyme is accompanied by a change in the spatial structure of the enzyme molecule. The active site of an enzyme, as a rule, has the form of a narrow depression or gap between adjacent domains. Diagram explaining the effect of the change in the conformation of the hexokinase enzyme molecule during binding of the glucose molecule is shown in Fig. 57.

Most often, the active centers of enzymes include functional groups of such amino acids:
OH - groups of serine, threonine, tyrosine;
SH - groups of cysteine;
NH - group of hystidine;
COOH - group of glutamate and aspartate;
NH2 - groups of arginine and lysine.

Physical nature of the biological catalysis phenomenon is currently unknown and is one of the unsolved problems of modern science.

The physics of the heterophase condensed state of a biopolymer molecule, the main provisions of which are described above, allows to explain and describe, on the basis of an accurate statistical method, the main properties of biological catalysis - a high rate of a chemical reaction and strict selectivity.

Biopolymer molecule with a heterophase structure, which has selectivity to the G molecule of the substrate, can catalyze a biochemical reaction involving this molecule. Acceleration of a biochemical reaction occurs due to the approximation of molecules in the crystalline phase of a biopolymer molecule with a heterophase structure. In the unit cell volume of a biopolymer molecule with a heterophase structure, 1/3 of the total number of substrate molecules is in a free state in the solvent, and the remaining 2/3 of the substrate molecules are bound with the monomers of the enzyme polymer chain. The concentrations of all components of the system in the condensed crystalline (globular) phase increase exponentially by a factor of the order parameter, an increase in which leads to a multiple increase in the number of effective collisions due to the proximity effect.

### Heterophasic biological catalysis rate.

Let us describe the physico-chemical mechanism of biological catalysis, using the example of a hexokinase enzyme and its isomer glucokinase, which play an important role in the biochemical regulation of glucose metabolism in a living tissue cell.

The enzymatic reaction of the first stage of glycolysis, catalyzed by both of these enzymes (hexokinase and glucokinase), has the following form: $ATP + - D - glucose \to ADP + - D - glucose - 6 - phosphate , ΔG = - 4 , 0 kcal .$

The rate of a chemical reaction, in general, is determined by the law of mass action (Guldberg and Waage) - the rate of the chemical reaction A+B → C with the formation of a new substance C from two substances A and B, is proportional to the product of the concentrations of the reacting substances (under otherwise identical conditions): $W = - k \times \left[A\right] \left[B\right] ,$ where k is the rate constant, which does not depend on the concentrations of A and B.

The kinetic scheme of reaction (50), during which the phosphate group P is transferred from an ATP molecule to a glucose molecule G (which cannot proceed spontaneously without a catalyst), can be represented as the following enzymatic reaction: $G + E + P \to PGE \to PG + E ,$ where E is a polymer chain monomer that can form a weak ionic bond with the monovalent ion M of the solvent or a hydrogen bond with the substrate molecule G of the solvent.

Enzymatic reaction (51), within the framework of the mechanism under consideration, proceeds in several stages in the active center of the enzyme molecule - a local region of the intramolecular space between the domains corresponding to the subunits of the macromolecule.

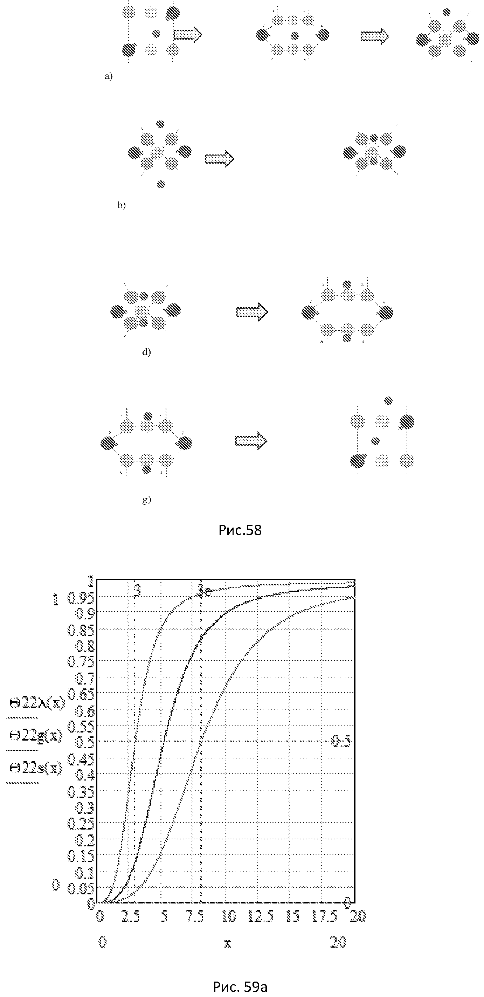

At the first stage, schematically shown in Fig. 58a, diffusion of ATP molecules (red color shows only the phosphate group of ATP) and glucose G from the solvent into the region of the active site of the enzyme (the region of the gaseous phase of a heterogeneous enzyme molecule with a low substrate concentration) occurs.

At the second stage (Fig. 58 b), the ATP molecule is absorbed by the tetramer complex in the crystalline phase: $\begin{matrix}E + G \to EG ; \\ 2 E + 2 G = EG + EG \to {E}_{2} {G}_{2} .\end{matrix}$

A tetramer complex can be defined as a substrate - an enzyme complex, which for the reaction under consideration can be denoted by the symbol E₂G₂, where G is a glucose molecule.

This stage is reversible and is not accompanied by any chemical changes in the substrate.

The transition of the phosphate group P from the ATP molecule to the glucose molecule G, which is in a bound state in the E₂G₂ tetramer complex in the crystalline (globular) phase (Fig. 44b), occurs during a chemical reaction, which can be schematically represented as: $P + GE \to PGE .$

At the third stage, schematically shown in Fig. 58 d,g, the transformation of the PGE complex into a GP product occurs as a result of a conformational transition of the tetramer, which is accompanied by the transition of the GP molecule through the energy barrier separating the crystalline (globular) phase, with an increased concentration of the substrate, and the liquid (molten globule) phase, with a reduced concentration of the substrate , followed by desorption of the product as a result of the destruction of the tetramer complex (transition from the liquid into gaseous phase): $PGE \to PG + E .$

Within the framework of the model of a biopolymer molecule with a heterophase structure described above, weak non-covalent interactions, i.e., ionic and hydrogen bonds, are involved in the formation of the GE complex, which is consistent with generally accepted concepts, which assume that the formation of an enzyme-substrate complex (ESC, Michaelis complex) occurs with participation of non-covalent interactions. Fig. 58 shows the main stages of the enzymatic reaction: a) diffusion of the ATP molecule from the liquid into crystalline phase; b) binding of a phosphate group (red) to a D-glucose molecule (yellow) to form a G6P molecule as a result of a chemical reaction in the crystalline phase of the enzyme; d) conformational transition of a tetramer with a bound G6P molecule from the crystalline to the liquid phase through an energy barrier separating liquid and crystalline phases; g) destruction of the tetramer in the liquid phase with the release of the G6P product molecule.

The rate W of the enzymatic reaction (50) can be calculated by the formula (51) if the rate constant is known; the rate constant k can be calculated, as will be shown below, as the ratio of two concentration-independent constants using the expression: k = k_{b}/K₀, where k_{b} is the rate of passage of the product PG through the energy barrier separating crystalline and liquid phases; K₀ is the equilibrium constant of the process of formation of a tetramer from 2 dimers; in this case, 1/K₀ is the dissociation constant of the tetramer with formation of 2 dimers. Here, it is taken into account that at the moment of reaching the peak of the energy barrier, on which R₂ configuration of the tetramer is realized, the mutual attraction between tetramer dimers is determined only by the contribution of internal entropy, the value of which determines the value of the dissociation constant.

The formula for calculation of the rate constant k can be derived based on the following considerations.

Reagents G (glucose molecule) and E (polymer chain monomer) are in equilibrium with EG dimers and E₂G₂ tetramers. The dissociation constant K_{G} of the tetramer with the formation of 2 dimers is determined by the following equation: ${K}_{G} = 1 / {K}_{0} = \left[{E}_{2}{G}_{2}\right] / \left[GE\right] \left[GE\right] .$

GP and E reagents are in equilibrium with the activated EGP complex (tetramer-phosphate group): $E + GP \to EGP \to E + GP .$

The equilibrium constant *K*_{GP} of the reaction (53b) is determined by the following equation: ${K}_{GP} = \left[EGP\right] / \left[GP\right] \left[E\right] .$

The equilibrium constant of the reaction G + P → GP, which cannot proceed spontaneously, is equal to the equilibrium constant of the enzymatic reaction (53 d): $\left[GP\right] / \left[G\right] \left[P\right] = \left[EGP\right] / \left[GP\right] \left[E\right] = \left[{E}_{2}{G}_{2}\right] / \left[GE\right] \left[GE\right] = {K}_{GP} .$

Therefore, the equation (50) for the reaction rate can be solved with respect to [GP]: $\begin{matrix}W = \left[GP\right] \times \left[barrier penetration rate\right] = \left[GP\right] \times {k}_{b} = {k}_{b} \times {K}_{GP} \times \left[G\right] \left[P\right] or \\ W = {k}_{b} \times {K}_{GP} \times \left[G\right] \left[P\right] = \left({k}_{b}/{K}_{0}\right) \times \left[G\right] \left[P\right] = k \times \left[G\right] \left[P\right]\end{matrix}$

Reaction rate (50) is: $k = {k}_{b} / {K}_{0} .$

The equilibrium constant K₀ is determined using expression (8): ${K}_{0} ≡ \left(1/{V}_{Q}\right) \times exp \left({E}_{b}/{k}_{B}T\right) = \left(1/{V}_{Q}\right) \times exp \left({S}_{tetr} T/{k}_{B}T\right) = \left(1/{V}_{Q}\right) \times exp \left({S}_{tetr}/{k}_{B}\right) ,$

Where E_{b} is the binding energy of a glucose molecule with a tetramer, which is determined by the internal entropy of the tetramer S*ₜₑₜᵣ*;
V_{Q} - is the quantum volume of a glucose molecule, which is determined using the following expression [14]: ${V}_{Q} = {\left({h}^{2}/2πM {k}_{B}T\right)}^{3 / 2} = 5 , 32 \times {10}^{- 21} / {\left(MT\right)}^{3 / 2} {{c}_{M}}^{3} = 3 , 646 \times {10}^{- 24} {{c}_{M}}^{3} ,$
where M is a molecular weight of glucose (substrate); h is Planck's constant.

The rate k_{b} of passage of the GP molecule through the energy barrier separating the isomeric conformations of the tetramer in the liquid and crystalline phases is determined by two factors - the height of the energy barrier Eₐ the value of which determines the probability of the passage of the molecule through the barrier, and the constant v is a constant having a frequency dimention corresponding to that of an electromagnetic quantum with an energy of k_{B}T: ${k}_{b} = ν \times {w}_{b} = ν \times exp \left(-{E}_{a}/{k}_{B}T\right) ,$ where $ν = {k}_{B} T / h ; h - Planck ′ s constant .$

A hydrogen bond, as a type of covalent bond with a delocalized electron density, is electromagnetic in nature. The energy of a hydrogen bond is determined by the energy of a pairwise interaction of a glucose molecule with a polymer chain monomer, which is quantized by a discrete portion of energy, proportional to the thermal energy quantum k_{B}T.The oscillation frequency v of the valence bond, in general, is determined by the frequency of the electromagnetic quantum, which can be calculated based on the requirement that in the equilibrium state the quantum of the electromagnetic energy of the valence bond *hv* is equal to a multiple of the number of thermal energy quanta n×k_{B}T, in relation to which equilibrium is established between the electron pair (oscillator) and thermostat:
nhv = nk_{B}T; v = k_{B}T/h = 0,75×10¹³/c, where n = 1, 2, 3, ...- is the proportion of the exchange quantum of energy of electromagnetic interaction in relation to the quantum of thermal energy k_{B}T.

The expression for the rate constant of a chemical reaction is as follows: $k = {k}_{b} / {K}_{0} = \left[{V}_{Q}\times exp\left({S}_{tetr}/{k}_{B}\right)\right] \times \left({k}_{B}T/h\right) \times exp \left(-{E}_{a}/{k}_{B}T\right) = {V}_{Q} \times \left({k}_{B}T/h\right) \times exp \left[-\left({E}_{a}-{S}_{tetr}T\right)/{k}_{B}T\right]$

Thus, the expression (54) for calculation of the reaction rate (50) has the following form: $W = \left({k}_{b}/{K}_{0}\right) \times \left[G\right] \left[P\right] = \left\{{V}_{Q}\times \left({k}_{B}T/h\right)\times exp\left[-\left({E}_{a}-{S}_{tetr}T\right)/{k}_{B}T\right]\right\} \times \left[G\right] \left[P\right] .$

The formula (54d) for calculation of the reaction rate between reagents [G] and [P] is universal, applicable for calculating the rate of an arbitrary biochemical reaction, the catalyst of which is a biopolymer molecule with a heterophase structure with selectivity to the substrate molecule G.

It should be noted that formula (54 a) can be reduced to a form that coincides with the Arrhenius formula: $k = {V}_{Q} \times \left[exp \left({S}_{tetr}/{k}_{B}\right)\right] \times \left({k}_{B}T/h\right) \times exp \left(-{E}_{a}/{k}_{B}T\right) = Aexp \left(-{E}_{a}/{k}_{B}T\right) ,$ where $A = {V}_{Q} \times \left[exp \left({S}_{tetr}/{k}_{B}\right)\right] \times \left({k}_{B}T/h\right) .$

### Glucokinase enzyme calculation.

The thermodynamic functions of an arbitrary enzyme, in particular, hexokinase isomers, can be obtained using the universal distribution functions (14), based on the known values of the Michaelis equilibrium constant for these enzymes. Glucokinase [EC 2.7.1.2] is an isotype IV of the hexokinase enzyme (a monomeric protein of 465 amino acids with a molecular weight of 50 kDa), mainly contained in hepatocytes, as well as in pancreatic cells. It catalyzes the phosphorylation only of D-glucose, by transferring the phosphate group to glucose due to the ATP.

Glucokinase is characterized by an equilibrium constant of *Kₘ* ≈ 10 mmol/L [31] and causes rapid uptake of glucose from blood in the liver during the absorption period. In this case, glucose enters hepatocytes via GLUT2 type transporters, activity of which does not depend on the level of insulin, but which affinity to glucose is low (Kₘ ≈ 10 mmol/L). Insulin affects the absorption of glucose by the liver only indirectly - it induces the synthesis of glucokinase in hepatocytes. Thus, glucokinase is designed to rapidly convert excess blood glucose after a meal into glycogen.

Saturation curves for the glucokinase enzyme can be obtained from the universal distribution function (14), in which the quantum number for calculating the interaction constant γ is determined from the condition of quantization of the free energy of interaction (31), which in general can be represented in the following form: ${F}_{0 gas} \left(α=0\right) / {nk}_{B} T = {F}_{0 gas} \left(α=0\right) / hν = {σ}_{0 gas} = {\left({γ}_{gas}\right)}^{2} ,$ where hv is quantum energy of electromagnetic interaction, proportional to the quantum of thermal energy k_{B}T; hv = n k_{B}, where n = 1, 2, 3, ....

The equilibrium constant *Kₘ* ≈ 10 mmol/L satisfies the value of the quantum of interaction energy 3k_{B}T, corresponding to the value of the quantum number n = 3. Glucokinase enzyme saturation curves obtained using the universal distribution function of the biopolymer molecule, are shown in Fig. 59a. Saturation curves and the rate of the enzymatic reaction of glucokinase, depending on the concentration of glucose, are given in units of the equilibrium constant k₀= 5,032 mmol/L (glucose concentration in blood is normal); Glucose concentration k_{mg1} = 15,096 mmol/L = 3×kₘₛ₂ on the X axis corresponds to the value of the dimensionless concentration x = 3.

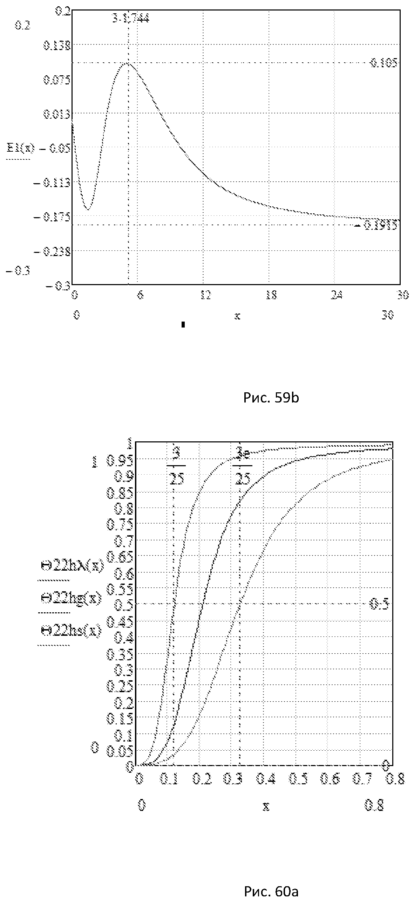

The height of the energy barrier E_{b} = Eₘₐₓ - Eₘᵢₙ, separating the crystalline and liquid phases in a glucokinase molecule can be determined directly from the free energy plot *U*_{λ*s attr*}(α) of the glucokinase tetramer versus concentration shown in Fig. 59b, which can be obtained from the distribution functions of glucokinase presented in Fig. 59a. The height of the energy barrier separating crystalline and liquid phases, corresponding to the energy of one hydrogen bond, is equal to Eₘₐₓ - Eₘᵢₙ = 3,47×(3k_{B}T) ≈ 10,41 kcal/mol.

The height of the energy barrier Eₐ in units of ε₀ = 3k_{B}T, can be derived from the energy barrier plot presented in dimensionless units in Fig. 59b.

Energy values according to the plot ${E}_{max} = 0 , 1035 …$ ${E}_{min} = - 0 , 188 …$ ${E}_{0} = - 0 , 160 …$ ${E}_{max} - {E}_{min} = 0 , 1035 + 0 , 188 = 0 , 2915 .$ ${E}_{0} - {E}_{min} = - 0 , 160 + 0 , 188 = 0 , 028 = {k}_{B} T .$

Difference between binding energies in the direct and reverse reactions is: ${E}_{min} - {E}_{0} = 0 , 188 - 0 , 160 = 0 , 028 = 3 {k}_{B} T .$

The height of the energy barrier separating the crystalline and liquid phases: ${E}_{a} = 3 , 47 {ε}_{0} = 3 , 47 \times \left({3 k}_{B}T\right) = 10 , 41 \times {k}_{B} T .$ ${E}_{a} = \left({E}_{max}-{E}_{min}\right) / \left({E}_{0}-{E}_{min}\right) = \left({E}_{max}-{E}_{min}\right) / 3 {k}_{B} T = \left(0,188+0,1035\right) / 0 , 028 = 10 , 41 .$

To calculate the rate of the glucokinase -catalyzed enzymatic reaction (50) using formula (54d), with a known glucokinase energy barrier height Eₐ = 3,47×(3k_{B}T), it is necessary to determine the dissociation constant of the tetramer with the formation of 2 dimers, which can be calculated using following expression: ${K}_{0} = \left[EG\right] \left[EG\right] / \left[{E}_{2}{G}_{2}\right] = \left(1/{V}_{Q}\right) \times exp \left({E}_{b}/{k}_{B}T\right) .$

Here, E_{b} - is the binding energy that must be expended to detach two EG complexes (glucose molecule G bonded to monomer E via one hydrogen bond) forming a tetramer complex [E₂G₂] in the R₂ configuration with 2 hydrogen bonds: EG + EG = E₂G₂.

Binding energy E_{b} is determined by the internal entropy of the tetramer in the R₂ configuration with 2 hydrogen bonds, which can be calculated using the following expression: $- {E}_{b} = {S}_{tetr} T = 4 {k}_{B} T ln g \left(l=2\right) = 4 {k}_{B} T ln 18 = 4 \times 2 , 89 {k}_{B} T ,$ where Sₜₑₜᵣ = 4k_{B} ln g(*l* = 2) - is the internal entropy of the tetramer; g(*l* = 2) - is the number of states realizing the microscopic form (configuration) of the R₂ tetramer with 2 hydrogen bonds. The factor 4 in the formula corresponds to the number of hydrogen bonds that form in the R₂ tetramer configuration as a result of electron density delocalization.

Therefore, binding energy, calculated per one hydrogen bond is: ${E}_{b} / 4 = {k}_{B} T ln Δg = {k}_{B} T ln 18 = 2 , 89 {k}_{B} T .$

It should be noted that the thermodynamic equilibrium of the system is established with respect to the energy exchange between the system and the reservoir with a quantum of thermal energy k_{B}T, in contrast to the electromagnetic equilibrium of interacting molecules, which is established with respect to the quantum of electromagnetic energy ε₀ = 3,47×(3k_{B}T).

Binding energy E_{b} is equal: ${E}_{b} = 4 {k}_{B} T ln Δg = {k}_{B} T ln 18 = 11 , 56 {k}_{B} T .$

The numerical expression for the equilibrium constant of the reaction is as follows: ${\text{K}}_{0} = \left(1 / {\text{V}}_{\text{Q}}\right) \times \text{exp} \left({\text{S}}_{\text{tetr}} / {\text{k}}_{\text{B}}\right) = 1 / 3,646 \times {10}^{- 24} {{}_{\text{CM}}}^{3} \times {\text{e}}^{11,56} = 10 {\text{N}}_{\text{A}} / 6,02 \times {3,646}_{\text{CM}} {}^{3} \times {\text{e}}^{11,56} ;$ ${K}_{0} = 4 , 385 mmol / L ,$ where N_{A} = 6,02x10²³ - Avogadro's number.

### Glucokinase enzymatic reaction rate calculation.

The rate of the enzymatic reaction (50) catalyzed by glucokinase at a known energy barrier height of glucokinase Eₐ = 3,47×(3k_{B}T) and dissociation constant 1/K₀, can be calculated using formula (54d).

Barrier height *Eₐ* calculated per one hydrogen bond in the units of quantum interaction energy ε₀ = 3k_{B}T, as it was shown, is Eₐ = Eₘₐₓ - Eₘᵢₙ = 3,47 ε₀.

Activation energy on 4 bonds per energy quantum ε₀ = 3k_{B}T is: ${E}_{max} - {E}_{min} = 4 \times 3 , 443 {ε}_{0} = 4 \times 3 , 47 \times 3 {k}_{B} T \approx 41 , 6 {k}_{B} T .$

The rate of penetration through energy barrier is: $ν \times {w}_{b} = ν \times exp \left(-{E}_{a}/{k}_{B}T\right) = 0 , 75 \times {10}^{13} / {e}^{41 , 6} = 0 , 75 \times {10}^{13} / {10}^{18} = 0 , 75 \times {10}^{- 5} .$

Thus, the numeric value of the rate constant is: $k = ν \times {w}_{b} / 1 , 735 {K}_{0} = 0 , 75 \times {10}^{- 5} / 1 , 735 \times 4 , 385 \times c \times mmol / L .$

Glucose concentration in the space unit increases proportionally to an increase in the interaction constant (order parameter): ${n}_{0 m} / {n}_{0 g} = γ = e \times {\left(e+1\right)}^{3} = 139 , 74 … = 1 , 4 \times {10}^{2} .$

A product of concentrations of reagents [G] and [P] is: $\left[G\right] \left[P\right] = 5 mmol / L \times 1 \times 2,08 \times {10}^{3} mmol / L ;$
where [P] = 1 mmol/L - ATP concentration, [G] = 5 mmol/L - glucose concentration. Here it is taken into account that glucose and ATP concentrations at α=1 are equal to 5 and 1 mmol/L, respectively.

Reaction rate is: $W = - k \left[G\right] \left[P\right] = \left(ν\times {w}_{b}/1,735 {K}_{0}\right) \times \left[G\right] \left[P\right] = 0 , 6 \times {10}^{- 3} \left(mmol/L\right) / s .$

Factor 1,1735 in the reaction rate formula appears as a result of the change in the glucose molecular mass after addition of the phosphate group. In the course of the phosphorylation reaction, D-glucose (G molecule) attaches the phosphate group PO₃H₂ with a molecular weight of 80 and turns into glucose-6-phosphate (GP molecule) with a molecular weight of 260 = 180 + 80.

The equilibrium constants of reactions (53a) and (53b) are related by the expression: ${K}_{G} / {K}_{GP} = {\left({M}_{GP}/{M}_{G}\right)}^{3 / 2} = {\left(260/180\right)}^{3 / 2} = 1 , 736 …$

This formula takes into account that the binding energy E_{b} of a glucose molecule with a tetramer, which is determined by the internal entropy of the tetramer Sₜₑₜᵣ, and does not depend on the mass of the molecule.

Therefore, K_{GP} = K_{G} /1,736 = 1/1,736 K₀.

The estimated value is in agreement with the experimental data [13], at *Kₘ* = 10: $30 \times {10}^{- 3} / 0 , 6 \times {10}^{2} \times 1 \left(mmol/L\right) / c = 50 \times {10}^{- 5} = 0 , 5 \times {10}^{- 3} \left(mmol/L\right) / c \left({V}_{max}=1\times {10}^{- 3}\right) .$

### Hexokinase reaction rate.

Hexokinase (ATP - dependent D-hexose-6-phosphotransferase) (EC 2.7.1.1) - is a cytoplasmatic enzyme from the transferase class, first enzyme in the glycolysis pathway.

Molecular weight 100000-50000, optimal pH: 7,6 - 8,2. The first isozyme, which is most typical for the brain, consists of one subunit with an established amino acid composition. The issue of the subunit structure of the remaining isoenzymes have not been solved yet. To manifest catalytic activity, it needs Mg²⁺ ions. The intrinsic substrate for this enzyme is not ATP4, but MgATP2 complex.

Activity in various organs (in µmol/1 g of wet tissue weight per 1 min): from 23 (brain) to 3,4 skeletal muscles.

The active center contains serine and histidine residues. SH groups of the polypeptide chain play an important role in the mechanism of the catalytic action. In the body, hexokinase is activated by vitamins, insulin, and also by binding to certain biological membranes, such as mitochondrial; it is inhibited by glucose-6-phosphate and corticosteroids.

Unlike glucokinase, the Michaelis constant of hexokinase is 0,01 mmol/L. Therefore, hexokinase localized in the cells of most tissues of the human body literally "catches" glucose in plasma, whereas glucokinase catalyzes glucose phosphorylation reaction only at its high concentrations. Thus, glucokinase and hexokinase enable redistribution of glucose in the body: during the absorption of nutrients in the intestine, the concentration of glucose in plasma increases, and glucose is transferred to the liver, where it is exposed to the effect of glucokinase; at the end of digestion, against the background of a decrease in the concentration of glucose, it is transferred to the skeletal muscles, where undergoes the effect of hexokinase.

Glucose metabolism begins with an irreversible reaction with hexokinase (or glucokinase), in which the transfer of the ATP phosphate group to glucose is catalyzed with formation of glucose-6-phosphate. Hexokinase reaction serves not only as a starting, but also as the main limiting reaction among other glycolysis-related reactions. Substrate specificity of hexokinase (EC 2.7.1.1) is relative - in addition to glucose, fructose, mannose, galactose, glucosamine and some type 2-deoxyglucose non-metabolizing sugars undergo phosphorylation. Hexokinase is located in the plasmatic membrane, cytoplasm, and is partially associated with mitochondria; in the brain, the activity of this fraction can reach 50% of the total activity of the enzyme. Experimental data have shown that potassium ion takes part in the excitation of hexokinase, and also it is involved in the excitation of other enzymes - pyruvate kinase, which accelerates the transport of phosphoric acid from pyruvate to adenosine diphosphate, enzymes involved in the Krebs cycle, including those assisting in the synthesis of adenosine triphosphate in the course of oxidative phosphorylation. It is quite obvious that the correlation between hexokinase activity and potassium ions indicates a direct correlation between potassium level and the level of macroergic phosphates. Thus, the importance of potassium for the abovementioned processes has been established experimentally, but the principles of activation of enzymatic systems with potassium have not yet been fully elucidated.

The Michaelis equilibrium constant for the hexokinase reaction is Kₕₑₓ = 0,02 mmol/L; the ratio of equilibrium constants is K_{gl}/Kₕₑₓ = 15/0,02 = 750.

Figure 60a shows saturation curves and the rate of hexokinase enzymatic reaction as a function of unitless glucose concentration, scaled by dividing the concentration by 0,5 mmol/L.

It can be seen that the barrier height per hydrogen bond is Eₘₐₓ - Eₘᵢₙ = 3,4436 ε₀. The value of the quantum of interaction energy in quantum thermal energy units, k_{B}T, can be determined from the plot of the energy barrier presented in Fig. 60b. The height of the energy barrier of one valence bond for the direct reaction is E_{0λhex} = 2,443×ε₀, for the reverse reaction E₀ₛₕₑₓ = 3,443×ε₀; quantum energy of interaction is ε₀ = e k_{B}T.

Activation energy value can be determined from the plot: ${E}_{max} = 0 , 1035 …$ ${E}_{min} = - 0 , 1915 …$ ${E}_{0} = - 0 , 163 …$ ${E}_{max} - {E}_{min} = 0 , 1035 + 0 , 1915 = 0 , 295 .$ ${E}_{0} - {E}_{min} = - 0 , 161 + 0 , 1915 = 0 , 0305 = {k}_{B} T .$ $\left({E}_{max}-{E}_{min}\right) / \left({E}_{0}-{E}_{min}\right) = 0 , 295 / 0 , 0305 = 9 , 67 \approx 3 , 5 {ε}_{0} = 3 , 5 \times {ek}_{B} T .$

Difference between binding energies in the direct and reverse reactions is: ${E}_{min} - {E}_{0} = 0 , 1915 - 0 , 161 = 0 , 0305 = {ε}_{0} = e {k}_{B} T .$

As it can be seen, tetramer dissociation constant can be calculated using following equation: ${K}_{0} = \left(1-{V}_{Q}\right) \times exp \left(-{S}_{tetr}/{k}_{B}\right) = \left(1/{V}_{Q}\right) \times exp \left(-ln {g}_{tetr}\right) ,$ where gₜₑₜᵣ is a number of degenerative internal states having the same energy, realizing the steady-state configuration of a tetramer with 4 valence bonds, provided that the conditions for compensation of attractive and repulsive forces are met.

According to the above described principle of free energy quantization (6), at glucose concentrations α ≥ 1, the equilibrium configuration of the tetramer, with a maximum number of microstates gₜₑₜᵣ = 25, corresponding to the minimum free energy of interaction, is established with respect to the quantum of interaction energy ε₀, a multiple of the quantum thermal energy ε₀ = n k_{B}T, where n = 1, 2, 3,... ; ε₀ ≥ k_{B}T. The microstates realizing the equilibrium configuration of the tetramer at α ≥ 1 glucose concentrations have similar energy and are degenerative.

As it was shown above (15), the change in the free energy of the transition (Na) tetramer from conformation (λ) into isomeric conformation (s) is: $TΔS = {k}_{B} T \times ln \left(25/9\right) = {k}_{B} T \times ln 2 , 777 \approx {k}_{B} T .$

At glucose concentrations α < 1, corresponding to the values of the Michaelis constant < 5 mmol/liter, the spatial configuration with the minimum free energy of volumetric interactions cannot be realized with an electromagnetic interaction energy quantum ε₀ proportional to the thermal energy quantum k_{B}T.

It can be shown that the equilibrium configuration of the tetramer, at α < 1, can be realized at the value of the interaction energy quantum ε₀ = e k_{B}T, where e is the natural logarithm base, which is a transcendent and irrational number that cannot be represented as the ratio m/n of an integer m to a natural number n, an infinite non-periodic decimal fraction, which is approximately equal to 2,71828....

The number e can be represented as the Euler formula: $e = 1 + 1 / 1 ! + 1 / 2 ! + … 1 / n ! + …$

In this case, the value of the energy quantum 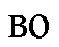 can take quantized values ε₀ₙ: corresponding to different integer values of the quantum number n: $\begin{matrix}{ε}_{01} = \left(1+1\right) \times {k}_{B} T = 2 {k}_{B} T ; \\ {ε}_{02} = \left(1+1+\frac{1}{2}\right) \times {k}_{B} T = 5 {k}_{B} T / 2 ; \\ {ε}_{03} = \left(1+1+\frac{1}{2}+1/6\right) \times {k}_{B} T = 8 {k}_{B} T / 3 ; \\ {ε}_{03} = \left(1+1+\frac{1}{2}+1/6+1/24\right) \times {k}_{B} T = 65 {k}_{B} T / 24 ; \\ {ε}_{0 n} = \left(1+1+\frac{1}{2}+1/6…+1/n!\right) \times {k}_{B} T .\end{matrix}$

The energy of the hydrogen bond, in this case, is split into an infinite number of interaction quanta, the energies of which are determined by the quantization condition (53), and, as a result, the level of the energy barrier is split into an infinite number of adjacent energy levels.

In thermal motion, tetramer configurations with different values of the distance between the monomers corresponding to different values of the interaction energy ε₀ₙ and the hydrogen bond energy can be realized with equal probability.

It should be noted that different values of intermolecular distances in the tetramer of hexokinase, in contrast to glucokinase, can be formed if the amino acid side chains are longer due to the additional monomer and additional covalent bond, which results in the flexibility of the chain of the side group. Therefore, in case of hexokinase, the amino acid with a negative side group charge is glutamic acid (Glu, E), the side group of which consists of 3 monomer units; an amino acid with an uncharged side group is asparagine (Asn, N), the side group of which consists of 3 monomer units; in case of glucokinase, the amino acid with a negative side group charge is aspartic acid (Asn, D), the side group of which consists of 2 monomer units; amino acid with an uncharged side group is an amino acid selected from the group of threonine, cysteine, serine, the side groups of which consist of 2 monomer units.

The active center of hexokinase contains serine and histidine residues. SH groups of the polypeptide chain play an important role in the mechanism of the catalytic action. Hexokinase is activated by vitamins, insulin, and also as a result of binding to certain biological membranes; it is inhibited by glucose-6-phosphate and corticosteroids.

The second difference between hexokinase and glucokinase is that the substrate specificity of hexokinase (EC 2.7.1.1) is relative - in addition to glucose, fructose, mannose, galactose, glucosamine, some non-metabolizing sugars of the 2-deoxyglucose type are subjected to phosphorylation. It can be assumed that the quantization of the hydrogen bond energy (53) may explain the relative substrate specificity of hexokinase by the fact that the listed sugar molecules form hydrogen bonds with the monomers of the polypeptide chain with close, but different energy values, which correspond to different values of the distance between the tetramer monomers.

The equilibrium configuration R₂ with 2 hydrogen bonds, corresponding to the minimum free energy at α < 1, in accordance with the scale invariance condition ( ), can be realized if the condition dS/dε ≡ 0 is met; this can be realized in a unique way as a result of splitting into e quanta of each quantum internal entropy of the tetramer, the number of which corresponds to the number of possible configurations of hydrogen bonds in the tetramer. Therefore, S/k_{B} = ln e^{g} = g, where g is the number of configurations equal to an integer.

As it was shown, at glucose concentrations of α ≥ 1, the equilibrium configuration of the tetramer, which is realized by the maximum number of microstates gₜₑₜᵣ = 25, corresponding to the minimum free energy of interaction, is established with respect to the quantum of interaction energy ε₀ = k_{B}T. The maximum interaction energy (peak of the energy barrier) is achieved at a glucose concentration corresponding to the specific point G of the process; the tetramer is in the R₂ conformation with 2 hydrogen bonds, which can be converted to the R₄ or R₀ conformation with equal probability.

The number of states g (*l* = 2) in which R₂ configuration with 2 hydrogen bonds is realized is 18, of which 6 states realize the electronic configuration and 12 states realize the monomer configuration.

The number of states g (*l* = 2) realizing the R₂ configuration with 2 hydrogen bonds, which arise upon splitting the energy of a hydrogen bond, at glucose concentrations a < 1, is equal to: ${g}_{hex} = g \left(l=2\right) = 12 {e}^{6} ; ln {g}_{hex} = 6 + ln 12 .$

The binding energy E_{b} that must be expended to detach two EG complexes (glucose molecule G bonded to monomer E via one hydrogen bond) forming a tetramer complex [E₂G₂] in the R₂ configuration with 2 hydrogen bonds can be calculated using following formula: ${E}_{b} = 4 {k}_{B} T ln g \left(l=2\right) = 4 {k}_{B} T ln 12 {e}^{6} = 4 \times \left(6+ln12\right) \times {k}_{B} T = 4 \times 8 , 48 \times {k}_{B} T ,$ where g(*l* = 2) is a number of states, realizing the configuration of R₂ tetramer with 2 hydrogen bonds.

Multiplier 4 in the formula corresponds to the number of hydrogen bonds in the tetramer complex. Therefore, binding energy, calculated per one hydrogen bond is: ${E}_{b} / 4 = {k}_{B} T g \left(l=2\right) = 4 {k}_{B} T ln 12 {e}^{6} = 4 \times \left(6+ln12\right) \times {k}_{B} T = 4 \times 8 , 48 \times {k}_{B} T .$

Equilibrium constant is: ${g}_{hex} = g \left(l=2\right) = 12 {e}^{6} ; ln {g}_{hex} = 6 + ln 12 .$ ${K}_{0} / {K}_{hex} = exp \left(6+ln12\right) / exp \left(ln18\right) = {e}^{6 + 2 , 48} / {e}^{2 , 89} = {e}^{6 - 0 , 41} = {e}^{5 , 59} = 267 , 7 .$ ${K}_{hex} = {K}_{0} / 267 , 7 = 4 , 4 / 267 , 7 = 0 , 016 mmol / L .$ $g \left(l=2\right) = 267 , 7 / 4 = 66 , 925$ $g \left(l=4\right) = {e}^{1} \times 24 = 65 , 23 .$

Activation energy on 4 bonds per energy quantum ε₀ = ek_{B}T is: ${E}_{a} = {E}_{max} - {E}_{min} = 4 \times 3 , 443 {ε}_{0} = 4 \times 3 , 443 \times {ek}_{B} T \approx 37 , 4 {k}_{B} T .$

Barrier penetration rate: $ν \times {w}_{b} = ν \times exp \left(-{E}_{a}/{k}_{B}T\right) = 0 , 75 \times {10}^{13} / {e}^{37 , 4} = 0 , 75 \times {10}^{13} / 1 , 8 \times {10}^{16} = 0 , 4 \times {10}^{- 3} .$

Reaction rate is: $\begin{matrix}W = - k \left[G\right] \left[P\right] = \left(ν\times {w}_{b}/1,735 {K}_{0}\right) \times \left[G\right] \left[P\right] = \\ = \left[0,4\times {10}^{- 3}/1,735\times 0,2\left(s\times mmol/L\right)\right] \times 0 , 2 \times 1 \times 1 , 4 \times {10}^{2} {\left(mmol/L\right)}^{2} = 0 , 32 \times {10}^{- 1} \left(mmol/L\right) / s .\end{matrix}$

Calculation results are in agreement with the experimental data [13].

### Chemical bond types in tetramers of the natural macromolecules.

Within the common classification, chemical bond in the tetramer between substrate molecules and monomers of the polymer chain may be defined as delocalized hydrogen bond.

Depending on the degree of generalization of the electronic density of the chemical bond between several atoms, the bond can be classified into localized and delocalized. Covalent bond is considered localized, if its electron pair is in the field of two nuclei and binds only two atoms. If the electronic density of the chemical bond is distributed between three or more nuclei, such bond is called three-center or multicenter bond, and in general - delocalized. Delocalized bond is a bond in which an electron pair is spread between several (more than 2) atomic nuclei (analog of metallic bond). The pattern of delocalization of electrons in chemical bond may differ depending on the spatial dimension. There are bonds delocalized in one dimension, in plane and in three-dimensional space. From the point of the abovedescribed classification, a well-known metallic bond is a short-acting and long-ranging, non-polar, highly delocalized (in three dimensions) bond.

Depending on the distance of appearance, chemical bonds can be classified into short- and long-range. The strength of the long-range bond is much lower than that of the short-range bonds. Far-range bond is also called weak or intermolecular bond. Short-range bond is manifested at the distance close to the size of an atom. It is formed between atoms within the molecule, crystal in the range between 74 - 400 pm. Short-range bond-breaking energy lies within the range from 40 to 1,000 kJ/mol. Long-range chemical bond is manifested upon transition of the substance from gaseous into liquid or solid state. It is formed between separate atoms, molecules at distances several times exceeding the length of a standard short-range bond. Short-range bonds are formed as a result of interaction where each electron may be described by an independent wave function -single-electron approximation. Long-range bonds are results of the collective electron motion.

### Glycoside bonds.

It was shown that thermodynamic properties of the biopolymer molecule can be described on the basis of a statistical model, as a result of the interaction of polymer chain monomers with solvent molecules and ions, which leads to the formation of tetramers in the volume of the macromolecule - molecular complexes consisting of 2 non-adjacent polymer chain monomers and 2 glucose solvent molecules, linked by a hydrogen bond, which can be considered a type of weak delocalized covalent bond between the glucose molecule and the monomers of the polymer chain.

It is well known that the glucose molecule can also form a glycosidic valence bond with amino acids of protein molecules. In glycoproteins characterized by a low carbohydrate content, a carbohydrate residue (an oligosaccharide with irregular structure and containing glucose, mannose, galactose and their amino derivatives, as well as N-acetylneuraminic acid) is linked to the amino acids of the protein chain or to the amide nitrogen of asparagine via N-glycosidic bond, or to the hydroxyl group of serine, threonine, hydroxylysine residues via O-glycosidic bond. Structural formulae of glycoside bonds are shown in fig. 61. A link between aminogroup or any other group containing nitrogen atom and sugar is often called glycosidic bond, although IUPAC does not recommend to do so. For example, a bond between sugar and nitrogen base in the nucleoside is called glycosidic bond. Structural formulae of glycosidic bonds in nucleosides are shown in fig. 62.

### Pharmaceutical compositions based on the hyaluronic acid.

Pharmaceutical compositions and products on the basis of hyaluronic acid are widely used in medicine and cosmetology: regeneration of skin cells in aesthetics cosmetology; healing of wounds and burns; protecting ocular structures and ensuring the depth of the anterior chamber during ophthalmic surgery and in the production of contact lenses; accelerated growth of bone cells and rapid bone fusion in fractures; delivery of drugs to the site of inflammation in pathological foci and control over the dosed release of the drug; accelerated growth of bone cells and rapid bone fusion in fractures.

New understanding of thermodynamic properties of the intercellular substance, based on the new method of calculation of polymer macromolecule of hyaluronic acid, allows to develop a fundamentally new pharmaceutical compounds on the basis of hyaluronic acid.

The invention is also related to the pharmaceutical compounds containing biopolymer molecule and one or several pharmaceutically acceptable carriers, adjuvants, solvents and/or fillers, that may be introduced into the patient's body together with the biopolymer molecule, which is the essential part of the invention, and which do not inhibit the effect of the biopolymer molecule, and are non-toxic in the amounts sufficient to deliver the biopolymer molecule in necessary amount.

Hyaluronic acid which is currently used in cosmetics and medicine, is obtained through biochemical synthesis. For this purpose, bacterial cultures, in particular, streptococci, cultivated on the herbal base (wheat broth), are used. This method is based on the ability of some organisms to synthesize hyaluronic acid. The biochemical method allows to obtain a large amount of substance having a necessary molecular weight and acceptable structure.

Stabilized hyaluronic acid-based products, which are used in cosmetics, for a long time persist in skin. They constitute a base for the water-accumulating products and stimulate regenerative processes in dermis. Hyaluronic acid molecule is highly sensitive. It sharply reacts to the chemical modification: thermal or mechanic; therefore, the product should be appropriately preserved in the course of chemical reactions. Stabilized hyaluronic acid is obtained using biochemical synthesis method, followed by fusion, which is called stabilization (formation of cross-links between hyaluronic acid molecules). Hyaluronic acid molecules undergo fusion to prevent their rapid degradation. Such hyaluronic acid demonstrates long-term clinical effects after penetration into skin. After fusion the resulting gel undergo purification, which is a meticulous process and a definitive factor in setting a price of the stabilizing hyaluronic acid-based products.

Depending on the degree of stabilization, gels with various viscosity are obtained for correction of different aesthetic issues: weakly stabilized - to remove fine wrinkles, more strongly stabilized - for correction of nasolabial folds and restoration of the lost volume.

Stabilized hyaluronic acid is used in contour correction and face-lifting, as this type of hyaluronate (hyaluronic acid sodium salt) is a potent volume-sparing compound. Thus, if you need to restore the lost volume, for example, on cheeks, to push the nasolabial folds externally, adjust facial contour and fill the gaps, use hyaluronic acid.

It must be stabilized and have characteristics, which are expressed in the native condition of the living tissue, in which the intercellular substance is in heterophase. As it was shown, normal physiological characteristics of the living tissue are achieved (at physiological temperature and acidity) at following concentrations of biochemical components in the intercellular substance:
glucose concentration (mmol/l) 5,0; temperature 36,447... C; hyaluronic acid monomers concentration (mmol/l) 45,0; [Na] - [Cl] (sodium excess, mmol/l) 40,0; external excess pressure (mm Hg) 14,6; glucose concentrations range (mmol/l) 3,7 - 13,67.

Biopolymer molecules described in this invention, may be used for prevention and/or treatment of human diseases, for example, in the form of the following compounds ("biopolymer molecule" is an active ingredient, which is a biopolymer molecule in heterophase state according to the invention).

Active ingredient concentration in the product, in particular, equivalent concentration of the hyaluronic acid monomers 45 mmol/l, is 15 mg/ml.

Glucose concentration in the product, is equivalent to the concentration of free (non-polymer-bound) glucose of 5 mmol/l, is in the range from 5 to 15 mg/ml. Sodium and potassium ion concentrations in the water solution with pH of 7,0 - 7,5 is 40 mmol/l and 5 mmol/l, respectively.

| Gel | mg/mL |
|---|---|
| Biopolymer molecule | 15,0 |
| Glucose | 5,0 |
| Water solution of Na & K ions | 980,0 |

| Gel | mg/mL |
|---|---|
| Biopolymer molecule | 15,0 |
| Glucose | 10,0 |
| Water solution of Na & K ions | 975,0 |

| Gel | mg/mL |
|---|---|
| Biopolymer molecule | 15,0 |
| Glucose | 5,0 |
| Water solution of Na & K ions | 980,0 |

### Enzyme-based drug formulations.

Type 2 diabetes mellitus is an urgent issue in current healthcare. Despite wide range of oral glucose-lowering drugs, currently, there is a high demand in the development of novel effective and safe drug products and their approval for clinical use. One of the potential targets for the new antidiabetic drugs development is glucokinase (GK). It acts as a glucose "sensor" in the pancreatic beta-cells and controls the rate of glycogen synthesis in liver. Glucokinase activator is a novel class of antidiabetic drugs with a reliable effect, that was demonstrated by the clinical data. It is important to note that glucokinase activators do not cause clinically significant hypoglycemia in experimental diabetic models, and do not affect lipid level. They also do not cause weight gain.

In 2003, a first report of a successful application of low molecular weight GK activator
(GKA) to reduce blood glucose level in animal models with type 2 diabetes mellitus. Screening of 120,000 compounds allowed to identify the formulation with the properties of allosteric GK activator. Active R enantiomer of this formulation, R082881675, improved enzymatic activity of GK, after oral administration it reduced blood glucose level in wild type mice and in diabetic models, improved glucose metabolism in liver and glucose-induced insulin secretion in isolated pancreatic islets in rats. The search and characterization of other low-molecular weight GKA have become one of the most important directions in the development of antidiabetic drugs over the next decade. Large pharmaceutical companies have got into the process of creation and testing of such formulations. Among them, "Array Biopharm Inc.", "Astra Zeneca pic, Merck & Co.", "OSI Pharmaceuticals Inc./Eli Lilly", "Pfizer Inc.", "Roche", "DaiichiSankyio", "Takeda", TransTech Pharma Inc./Forest Laboratories Inc./Novo Nordisk A/S", "Advinus", "Hua Medicine". By 2008, more than 90 low-molecular weight GKA were patented. Most of these formulations are based on the pharmacophore (its diagram is shown in Fig. 2). Most of these compounds provided potent glucose-lowering effect in various diabetic animal models. Over 20 provisional glucose-lowering products from this class were approved for clinical trials, although in most cases trials were interrupted or discontinued at stage I or II [16]. The main reason for discontinuation was an increased risk of hypoglycemia. Currently, clinical trials with only 5 products are ongoing. These are GKM8001, (Advinus Therapeutics Ltd., India) (phase II), DS87309 (Daiichi8Sankyo, Japan) (phase I), HMS5552 (Hua Medicine, China) (phase I), PF804937319 (Pfizer) (phase II) and TTP 399 (GK18399) (TransTech Pharma Inc./Forest Laboratories Inc./Novo Nordisk A/S) (phase II). Further prospects for the use of low molecular weight allosteric GKAs are associated with the possibility of development of hepatoselective drugs with a low risk of hypoglycemia.

### REFERENCES

1. John L. Smith. The Pursuit of Noninvasive Glucose: "Hunting the Deceitful Turkey". Fourth Edition, 2015.
2. USP # 5,795,305, August 18,1998, Cho et al. Process and device for non-invasive determination of glucose concentration in parts of human body.
3. USP #5,823,966, October 20, 1998, Buchet. Non - invasive continuous blood glucose monitoring.
4. USP # 5,890,489, April 6, 1999, Elden. Method for non - invasive determination of glucose in body fluids.
5. USP # 5,924,996, July 20, 1999, Cho et al. Process and device for detecting the exchange of heat between the human body and the invented device and its correlation to the glucose concentration in human body.
6. USP # 6,522,903, February 18, 2003, Berman et al. Glucose measurement assessment utilizing non-invasive assessment methods.
7. USP # 6,517,482, February 11, 2003, Elden et al. Method and apparatus for non- invasive determination of glucose in body fluids.
8. USP # 5,040,541, August 20, 1991, Poppendiek. Whole body calorimeter.
9. USP # 4,386,604, June 7, 1993, Hershey. Determination of the basal metabolic rate of humans with a whole -body calorimeter.
10. R.F. Musin Patent of the RF for the invention No.: 2629796 "Method and multisensor device for non-invasive blood glucose level monitoring".
11. R.F. Musin Patent of the RF for the invention No.: 2396897.
12. Edsall J.T., Gutfreund H. BIOTHERMODINAMICS. The Study of Biochemical Processes at Equilibrium. JOHN WILLEY & SONS, 1983.00
13. I.N. Kendysh. Carbohydrate metabolism regulation. Medicina Publishing House. Moscow.
14. Peusner Leonardo. Concepts in Bioenergetics. Peusner Biomedical Associates, Prentice - Hall, Inc., Englewood Cliffs, New Jersey, 1974.
15. Lehninger A.L. BIOCHEMISTRY. The Johns Hopkins University, School of Medicine. Worth Publishers, Inc., New York, 1972.
16. Atkins P.W. The second law. Scientific American Library, New York, 1984.
17. R.F. Musin, V.A. Morozova, E.E. Godik, Yu.V. Gulyaaev (1986). Electrical properties of the stratum corneum of the human epidermis and water transport in it. - Biophysics, vol. 31, no. 3, pp. 478-480.
18. Forbath N., Hetenyi C. Glucose dynamics in normal subjects and diabetic patients before and after a glucose load. - Diabetes, 1966, vol. 15, # 11, p. 778-789.
19. Hah S. E. H., Saunders J., Sonksen P.H. Glucose and free fatty acid turnover in normal subjects and diabetic patients before and after insulin treatment. - Diabetologia, 1979, vol.16, # 5, p. 297-306.
20. Musin R.F., Godik E.E., Gulyaev Y.V., Morozov V.A. Natural water diffusion through the stratum corneum of the human body epidermis and its electrical properties/ - 3 International conference on water and ions in biological systems. Bucharest, Romania, 1984, p.34.
21. R.F. Musin, N.Yu. Ivanova, V.A. Martynova, V.A. Morozov, E.E. Godik, Yu.V. Gulyaev (1986). On the sensitivity of human skin to infrared heat flows. - Reports of the Academy of Sciences of the USSR, volume 289, No. 3, pp. 718-720.
22. Yu.V. Gulyaev, E.E> Godik, R.F. Musin, V.A. Morozov, V.A. Martynova, I.V. Valiev (1989). Thresholds of thermal sensitivity of the skin to electromagnetic radiation. - Sensory systems, volume 3, No. 2, pp. 209-212.
23. E.E. Godik, V.A. Morozov, R.F. Musin (1985). On the dynamics of relaxation of triboelectric charge on the surface of the stratum corneum of the skin epidermis. - Biophysics, 30 (2): 309-312 (In Russian).
24. Musin R.F., Godik E.E., Gulyaev Y.V., Morozov V.A., Sudarev A.M. Membrane mechanisms of water transport in epidermis. 4 International conference on water and ions in biological systems. Bucharest, Romania, 1988, p.167-172.
25. R.F. Musin, V.A. Morozova, A.M. Sudarev (1990). On the mechanisms of water transport in the epidermis - Biophysics, vol. 35, issue 4, pp.653-656.
26. Yas Kuno. (1959). Human Perspiration. Charles & Thomas Publ., Springfield, Illinois, USA.
27. A.V. Korobkov, S.A. Chesnokova (1986). Atlas of normal physiology. M.: Higher school, p. 351
28. Homeostasis. Ed. by: P.D. Gorizontov M., "Medicine", 1976, 464 p., il
29. E. Calve, A. Prat. Microcalorimetry. M.: Foreign literature publishing house. 1963. p. 477).
30. F. Dyson, E. Montroll, M. Cutz, M. Fisher. Resistance and phase transitions. Mir Publishing House, Moscow, 1973.
31. Lehninger A., Biochemistry, Worth Publishers, New York, 1972.
32. C. B. Anfinsen. "Molecular Organization and Biological Function (Modern Perspectives in Biology)". New York. Harper & Row, 1966.
33. C. R. Cantor, Paul R. Schimmel. Biophysical Chemistry. Parts 1, 2, 3. W. N. Freeman and Company. San Francisco. 1980. - 536pp.
34. J.M. Guss, D.W.L. Hukins, P.J.C. Smith, W.T. Winter, S. Arnott, R. Moorhouse, D.A. Rees Hyaluronic Acid, Molecular Conformations and Interactions in Two Sodium Salts, J. Mol. Biol. v. 95, 359 (1975).
35. A.G. Marshall, Biophysical Chemistry, Parts 1,2. John Wiley and Sons, Inc. 1978.
36. L.E. Boltzmann, Lectures on Gas Theory, Berkley, (1964).
37. J.W. Gibbs, Elementary principles in statistical mechanics, developed with especial reference to the rational foundation of thermodynamics, Yale Univ. Press, 1902.
38. J.W. Gibbs, Collected Works, New Haven, (1948).
39. E. Fermi. Thermodynamics. New York, Prentice-Hall, Inc, 1937.
40. E. Fermi, Notes on thermodynamics and statistics, Univ. Chicago Press, 1966.
41. R.P. Feynman. Statistical Mechanics. Set of Lectures. California Institute of Technology. W.A. Benjamin, Inc., Massachusetts (1972).
42. C. Kittel. Thermal Physics. John Willey and Sons, Inc. New York. 1969.
43. C. Bohr, Zbl. Physiologie 17, 682 (1903).
44. J. Monod, J. Wyman, J. P. Changeux, J. Mol., Biol. 12, 88 (1965).
45. J.T. Edsall, H. Gutfreund. Biothermodynamics. John Wiley & Sons, 1983.
46. Paul J. Flory, The Configuration of Real Polymer Chains, J. Chem. Phys. 17, 303 (1949).
47. A.Yu. Grosberg, A.R. Khokhlov "Statistical physics of macromolecules" - M.: Nauka, 1989.
48. A. Yu. Grosberg, A. R. Khokhlov, Statistical Physics of Macromolecules, Springer, 1994.
49. A.Yu. Grosberg, A.R. Khokhlov Physics of chain molecules - M.: Znanie, 1983.
50. I. M. Lifshitz, A. Yu. Grosberg, A. R. Khokhlov, Volume interactions in statistical physics of a polymer macromolecule, UNF 127 353-389 (1979)
51. Ptitsyn O. B. The molten globule state (Review). In: "Protein Folding" (T.E. Creighton, ed.), W.H. Freeman & Co., 1992, 243-300.
52. Hadler N.M. Enhanced Diffusivity of Glucose in a Matrix of Hyaluronic Acid. The Journal of Biological Chemistry. 1980; 255(8): 3532-5.
50. Ya. S. Brodsky. Statistics. Probability. Combinatories. - M.: "Mir i Obrazovanie" publishing house, 2008. - p. 544.

## Claims

1. The method of isothermal calorimetric spectroscopy of biochemical components of the patient's living tissue, including the following steps:
- apply at least one heat- and waterproof applicator on a skin surface with a dosed pressure, forming a closed system in the local area of tissue under the applicator;
- providing a source of electromagnetic radiation with constant and/or modulated intensity;
- exert through said source of electromagnetic radiation a local effect on the tissue area under the applicator by electromagnetic radiation at one or several wavelengths corresponding to the characteristic frequencies of absorption of biochemical components of intercellular and/or intracellular substance, moreover, the intensity of electromagnetic radiation can be constant or variable, varying with constant speed and/or modulated frequency and/or amplitude;
- measure the value of physiological parameter, characterizing the thermodynamic phase state of the intercellular substance under the applicator and its temporal dynamics, depending on the power of incident electromagnetic radiation;
- determine the concentration of the biochemical component of the intercellular substance and/or intercellular fluid and/or cell and/or blood and its temporal dynamics based on the temporal dynamics of the measured physiological parameter,
wherein the physiological parameter characterizing the thermodynamic phase state of the intercellular substance is the osmotic pressure of the intercellular substance of the living tissue under the applicator.

2. The method according to claim 1, distinctive in that the biochemical components of the intercellular and/or intracellular substance are selected from the group consisting of water, hyaluronic acid, glucose, triglycerides and other biochemical components of the intercellular substance, cells and blood.

3. The method according to any of claims 1 to 2, distinctive in that the concentration of the biochemical component of the intercellular substance and /or intercellular fluid and/or blood is additionally measured based on the diffuse reflection spectroscopy method and/or method of Raman spectroscopy.

4. The method according to claim 1, distinctive in that the biochemical component is the blood glucose, the concentration of which is determined by the concentration of glucose associated with the monomers of the polymer chain of the intercellular substance.

5. The method according to claim 1, distinctive in that the osmotic pressure of the intercellular substance or the amount of water in the intercellular space is determined from the amount of water in the stratum corneum based on measurements of physical characteristics of the stratum corneum, which are selected from the group consisting of electrophysical characteristics, spectral and optical-acoustic characteristics, and thermal physical characteristics.

6. The method according to claim 1, distinctive in that the osmotic pressure of the intercellular substance or the amount of water in the intercellular space is determined from the amount of water in the stratum corneum based on measurements of spectral characteristics of the stratum corneum at wavelengths corresponding to characteristic frequencies of water in the stratum corneum, using a spectral method that is selected from the group consisting of: IR spectroscopy, Raman spectroscopy, optical-acoustic spectroscopy, double-beam spread spectroscopy.

7. The method according to claim 1, distinctive in that the osmotic pressure of the intercellular substance or the amount of water in the intercellular space is determined from the amount of water in the stratum corneum by measuring the electrical characteristics of the stratum corneum, which are selected from the group consisting of the transverse electrical conductivity of the stratum corneum at DC and/or AC currents, as well as the dielectric permittivity.

8. The method according to claim 4, distinctive in that the concentration of glucose in the blood and its physiological changes are determined based on the temporal dynamics of the physiological parameter, characterizing the phase state of the intercellular substance, and the temporal dynamics of the biomarker, characterizing the metabolic rate of the cell, and the physiological parameter is the osmotic pressure of the intercellular substance and/or the amount of water in the intercellular space, and the biomarker is the osmotic pressure of the intracellular substances and/or the amount of water in the intracell volume, moreover, the indicated intercellular substance under the applicator is a natural biosensor with a hetero phase structure with selectivity to the glucose molecule and sensitivity to heat flow, preferably the biomarker, characterizing the metabolic rate of a cell, is the total amount of water in the tissue area under the applicator, measured by an electrometric and/or spectral method, which is selected from a group including: isothermal calorimetric spectroscopy, diffuse reflection spectroscopy, Raman spectroscopy, optical-acoustic spectroscopy.

9. A device for isothermal calorimetric spectroscopy of biochemical components of living tissue containing heat- and a waterproof applicator made with the possibility of applying to the patient's skin with a dosed pressure, a temperature sensor, one or several sensors of physiological parameters, characterizing the thermodynamic phase state of the intercellular substance under the applicator, one or several sources of electromagnetic radiation, at that the source of electromagnetic radiation is made with the possibility of radiating with constant and/or modulated intensity and/or frequency modulation; a device for creating a calibration effect, while the sensors of physiological parameters are located under the applicator, while the signals from the above sensors are received sequentially on the inputs of the amplifier module and/or synchronous detector and/or analog-to-digital converter installed on the upper surface of the applicator, on the information processing unit and the information display unit.

10. The device according to claim 9, distinctive in that the sensors of physiological parameters characterizing the thermodynamic state of the intercellular substance under the applicator are made as sensors of the osmotic pressure of the intercellular substance and/or sensors of the amount of water in the intercellular tissue space in the local volume under the applicator and/or sensors of the elastic pressure of the living tissue under the applicator.

11. The device according to claim 9, distinctive in that the device for creating a calibration effect is a source of thermal power, made in the form of a resistor and/or a Peltier element and/or a source of electric current and/or voltage; a device for creating dosed pressure on the surface of the applicator.

12. The device according to claim 10, in which an osmotic pressure sensor of the intercellular substance or a sensor of the amount of water in the intercellular space of tissue in a local volume under the applicator is an electrometric sensor based on measuring the electrophysical characteristics of the stratum corneum of epidermis under the applicator, the measurement principle of which is selected from a group including measurement of the transverse electrical conductivity of the stratum corneum at a constant and/or alternating current; measurement of dielectric permittivity of the stratum corneum; measurement of electrical conductivity and/or dielectric permittivity of the tissue under the applicator.

13. The device according to claim 10, distinctive in that the spectral sensor, containing a source and a receiver of electromagnetic radiation, is based on spectral measurements of the amount of water in the stratum corneum by the characteristic frequencies of water in the stratum corneum based on a spectral method selected from a group including isothermal calorimetric spectroscopy, absorption spectroscopy, diffusion reflection spectroscopy, Raman spectroscopy, optical-acoustic spectroscopy.

14. The device according to any of claims 9 to 10, distinctive in that it additionally contains a spectral device including a source and a receiver of electromagnetic radiation for spectral measurements based on diffusion reflection spectroscopy.

## Patentansprüche

1. Verfahren zur isothermen kalorimetrischen Spektroskopie biochemischer Komponenten des lebenden Gewebes eines Patienten, das folgende Schritte umfasst:
- Aufbringen mindestens eines hitze- und wasserbeständigen Applikators auf eine Hautoberfläche mit einem dosierten Druck, wobei ein geschlossenes System im lokalen Bereich des Gewebes unter dem Applikator gebildet wird;
- Bereitstellen einer Quelle elektromagnetischer Strahlung mit konstanter und/oder modulierter Intensität;
- Ausüben einer lokalen Wirkung auf den Gewebebereich unter dem Applikator durch die Quelle elektromagnetischer Strahlung mittels elektromagnetischer Strahlung bei einer oder mehreren Wellenlängen, die den charakteristischen Absorptionsfrequenzen biochemischer Komponenten interzellulärer und/oder intrazellulärer Substanz entsprechen, wobei die Intensität der elektromagnetischen Strahlung konstant oder variabel sein kann und mit konstanter Geschwindigkeit und/oder modulierter Frequenz und/oder Amplitude variiert;
- Messen des Wertes eines physiologischen Parameters, der den thermodynamischen Phasenzustand der interzellulären Substanz unter dem Applikator und deren zeitliche Dynamik in Abhängigkeit von der Leistung der einfallenden elektromagnetischen Strahlung charakterisiert;
- Bestimmen der Konzentration der biochemischen Komponente der interzellulären Substanz und/oder der interzellulären Flüssigkeit und/oder der Zelle und/oder des Blutes und deren zeitlicher Dynamik basierend auf der zeitlichen Dynamik des gemessenen physiologischen Parameters,
wobei der physiologische Parameter, der den thermodynamischen Phasenzustand der interzellulären Substanz charakterisiert, der osmotische Druck der interzellulären Substanz des lebenden Gewebes unter dem Applikator ist.

2. Verfahren nach Anspruch 1, **dadurch gekennzeichnet, dass** die biochemischen Komponenten der interzellulären und/oder intrazellulären Substanz ausgewählt sind aus der Gruppe, bestehend aus Wasser, Hyaluronsäure, Glukose, Triglyzeriden und anderen biochemischen Komponenten der interzellulären Substanz, Zellen und Blut.

3. Verfahren nach einem der Ansprüche 1 bis 2, **dadurch gekennzeichnet, dass** die Konzentration der biochemischen Komponente der interzellulären Substanz und/oder interzellulären Flüssigkeit und/oder des Blutes zusätzlich basierend auf dem Verfahren der diffusen Reflexionsspektroskopie und/oder dem Verfahren der Raman-Spektroskopie gemessen wird.

4. Verfahren nach Anspruch 1, **dadurch gekennzeichnet, dass** die biochemische Komponente die Blutglukose ist, deren Konzentration durch die Konzentration von Glukose bestimmt wird, die mit den Monomeren der Polymerkette der interzellulären Substanz assoziiert ist.

5. Verfahren nach Anspruch 1, **dadurch gekennzeichnet, dass** der osmotische Druck der interzellulären Substanz oder die Wassermenge im Interzellularraum aus der Wassermenge im Stratum corneum basierend auf Messungen physikalischer Eigenschaften des Stratum corneum bestimmt wird, die ausgewählt sind aus der Gruppe, bestehend aus elektrophysikalischen Eigenschaften, spektralen und optisch-akustischen Eigenschaften sowie thermisch-physikalischen Eigenschaften.

6. Verfahren nach Anspruch 1, **dadurch gekennzeichnet, dass** der osmotische Druck der interzellulären Substanz oder die Wassermenge im Interzellularraum aus der Wassermenge im Stratum corneum basierend auf Messungen spektraler Eigenschaften des Stratum corneum bei Wellenlängen bestimmt wird, die charakteristischen Frequenzen von Wasser im Stratum corneum entsprechen, unter Verwendung eines Spektralverfahrens, das ausgewählt ist aus der Gruppe, bestehend aus: IR-Spektroskopie, Raman-Spektroskopie, optisch-akustischer Spektroskopie, Doppelstrahl-Spektroskopie.

7. Verfahren nach Anspruch 1, **dadurch gekennzeichnet, dass** der osmotische Druck der interzellulären Substanz oder die Wassermenge im Interzellularraum aus der Wassermenge im Stratum corneum durch Messen der elektrischen Eigenschaften des Stratum corneum bestimmt wird, die ausgewählt sind aus der Gruppe, bestehend aus der transversalen elektrischen Leitfähigkeit des Stratum corneum bei Gleich- und/oder Wechselstrom sowie der Dielektrizitätskonstante.

8. Verfahren nach Anspruch 4, **dadurch gekennzeichnet, dass** die Konzentration von Glukose im Blut und deren physiologische Veränderungen basierend auf der zeitlichen Dynamik des physiologischen Parameters, der den Phasenzustand der interzellulären Substanz charakterisiert, und der zeitlichen Dynamik des Biomarkers, der die Stoffwechselrate der Zelle charakterisiert, bestimmt werden, und der physiologische Parameter der osmotische Druck der interzellulären Substanz und/oder die Wassermenge im Interzellularraum ist, und der Biomarker der osmotische Druck der intrazellulären Substanzen und/oder die Wassermenge im Intrazellularvolumen ist, wobei zudem die angegebene interzelluläre Substanz unter dem Applikator ein natürlicher Biosensor mit einer Heterophasenstruktur mit Selektivität gegenüber dem Glukosemolekül und Empfindlichkeit gegenüber Wärmefluss ist, vorzugsweise der Biomarker, der die Stoffwechselrate einer Zelle charakterisiert, die Gesamtmenge an Wasser im Gewebebereich unter dem Applikator ist, gemessen durch ein elektrometrisches und/oder spektrales Verfahren, welches ausgewählt ist aus einer Gruppe, umfassend: isotherme kalorimetrische Spektroskopie, diffuse Reflexionsspektroskopie, Raman-Spektroskopie, optisch-akustische Spektroskopie.

9. Vorrichtung zur isothermen kalorimetrischen Spektroskopie biochemischer Komponenten von lebendem Gewebe, enthaltend einen hitze- und wasserbeständigen Applikator, der zur Aufbringung auf die Haut des Patienten mit einem dosierten Druck ausgebildet ist, einen Temperatursensor, einen oder mehrere Sensoren für physiologische Parameter, die den thermodynamischen Phasenzustand der interzellulären Substanz unter dem Applikator charakterisieren, eine oder mehrere Quellen elektromagnetischer Strahlung, wobei die Quelle elektromagnetischer Strahlung mit der Möglichkeit zur Abstrahlung mit konstanter und/oder modulierter Intensität und/oder Frequenzmodulation ausgebildet ist; eine Vorrichtung zur Erzeugung eines Kalibrierungseffekts, wobei die Sensoren für physiologische Parameter unter dem Applikator angeordnet sind, während die Signale der vorgenannten Sensoren sequenziell an den Eingängen des Verstärkermoduls und/oder des Synchrondetektors und/oder des Analog-Digital-Wandlers empfangen werden, die auf der Oberseite des Applikators, auf der Informationsverarbeitungseinheit und der Informationsanzeigeeinheit installiert sind.

10. Vorrichtung nach Anspruch 9, **dadurch gekennzeichnet, dass** die Sensoren für physiologische Parameter, die den thermodynamischen Zustand der interzellulären Substanz unter dem Applikator charakterisieren, als Sensoren für den osmotischen Druck der interzellulären Substanz und/oder Sensoren für die Wassermenge im interzellulären Geweberraum im lokalen Volumen unter dem Applikator und/oder Sensoren für den elastischen Druck des lebenden Gewebes unter dem Applikator ausgebildet sind.

11. Vorrichtung nach Anspruch 9, **dadurch gekennzeichnet, dass** die Vorrichtung zur Erzeugung eines Kalibrierungseffekts eine Wärmequelle ist, die in Form eines Widerstands und/oder eines Peltier-Elements und/oder einer elektrischen Stromquelle und/oder Spannungsquelle ausgebildet ist; eine Vorrichtung zur Erzeugung eines dosierten Drucks auf die Oberfläche des Applikators.

12. Vorrichtung nach Anspruch 10, bei der ein Sensor für den osmotischen Druck der interzellulären Substanz oder ein Sensor für die Wassermenge im interzellulären Raum von Gewebe in einem lokalen Volumen unter dem Applikator ein elektrometrischer Sensor ist, der auf der Messung der elektrophysikalischen Eigenschaften des Stratum corneum der Epidermis unter dem Applikator basiert, dessen Messprinzip ausgewählt ist aus einer Gruppe, umfassend eine Messung der transversalen elektrischen Leitfähigkeit des Stratum corneum bei Gleich- und/oder Wechselstrom; Messung der Dielektrizitätskonstante des Stratum corneum; Messung der elektrischen Leitfähigkeit und/oder Dielektrizitätskonstante des Gewebes unter dem Applikator.

13. Vorrichtung nach Anspruch 10, **dadurch gekennzeichnet, dass** der Spektralsensor, der eine Quelle und einen Empfänger elektromagnetischer Strahlung enthält, auf spektralen Messungen der Wassermenge im Stratum corneum anhand der charakteristischen Frequenzen von Wasser im Stratum corneum basiert, basierend auf einem Spektralverfahren, das ausgewählt ist aus einer Gruppe, umfassend isotherme kalorimetrische Spektroskopie, Absorptionsspektroskopie, diffuse Reflexionsspektroskopie, Raman-Spektroskopie, optisch-akustische Spektroskopie.

14. Vorrichtung nach einem der Ansprüche 9 bis 10, **dadurch gekennzeichnet, dass** sie zusätzlich eine Spektralvorrichtung enthält, die eine Quelle und einen Empfänger elektromagnetischer Strahlung für spektrale Messungen basierend auf diffuser Reflexionsspektroskopie umfasst.

## Revendications

1. Procédé de spectroscopie calorimétrique isotherme des composants biochimiques du tissu vivant du patient, comprenant les étapes suivantes :
- appliquer au moins un applicateur imperméable à la chaleur et à l'eau sur une surface de la peau avec une pression dosée, formant un système fermé dans la zone locale du tissu sous l'applicateur ;
- fournir une source de rayonnement électromagnétique d'intensité constante et/ou modulée ;
- exercer à travers ladite source de rayonnement électromagnétique, un effet local sur la zone tissulaire sous l'applicateur par rayonnement électromagnétique à une ou plusieurs longueurs d'onde correspondant aux fréquences caractéristiques d'absorption de composants biochimiques de substance intercellulaire et/ou intracellulaire, de plus, l'intensité du rayonnement électromagnétique peut être constante ou variable, variant à vitesse constante et/ou fréquence et/ou amplitude modulée ;
- mesurer la valeur du paramètre physiologique, caractérisant l'état de phase thermodynamique de la substance intercellulaire sous l'applicateur et sa dynamique temporelle, en fonction de la puissance du rayonnement électromagnétique incident ;
- déterminer la concentration de la composante biochimique de la substance intercellulaire et/ou du fluide intercellulaire et/ou de la cellule et/ou du sang et sa dynamique temporelle sur la base de la dynamique temporelle du paramètre physiologique mesuré,
dans lequel le paramètre physiologique caractérisant l'état de phase thermodynamique de la substance intercellulaire est la pression osmotique de la substance intercellulaire du tissu vivant sous l'applicateur.

2. Procédé selon la revendication 1, **caractérisé en ce que** les composants biochimiques de la substance intercellulaire et/ou intracellulaire sont choisis dans le groupe constitué par l'eau, l'acide hyaluronique, le glucose, les triglycérides et d'autres composants biochimiques de la substance intercellulaire, les cellules et le sang.

3. Procédé selon l'une quelconque des revendications 1 à 2, **caractérisé en ce que** la concentration du composant biochimique de la substance intercellulaire et/ou du fluide intercellulaire et /ou du sang est en outre mesurée sur la base du procédé de spectroscopie par réflexion diffuse et/ou du procédé de spectroscopie Raman.

4. Procédé selon la revendication 1, **caractérisé en ce que** le composant biochimique est la glycémie, dont la concentration est déterminée par la concentration de glucose associée aux monomères de la chaîne polymère de la substance intercellulaire.

5. Procédé selon la revendication 1, **caractérisé en ce que** la pression osmotique de la substance intercellulaire ou la quantité d'eau dans l'espace intercellulaire est déterminée à partir de la quantité d'eau dans la couche cornée sur la base de mesures de caractéristiques physiques de la couche cornée, qui sont sélectionnées dans le groupe constitué de caractéristiques électrophysiques, de caractéristiques spectrales et optiques-acoustiques et de caractéristiques physiques thermiques.

6. Procédé selon la revendication 1, **caractérisé en ce que** la pression osmotique de la substance intercellulaire ou la quantité d'eau dans l'espace intercellulaire est déterminée à partir de la quantité d'eau dans la couche cornée sur la base de mesures des caractéristiques spectrales de la couche cornée à des longueurs d'onde correspondant aux fréquences caractéristiques de l'eau dans la couche cornée, en utilisant une méthode spectrale qui est sélectionnée dans le groupe constitué de : spectroscopie IR, spectroscopie Raman, spectroscopie optique-acoustique, spectroscopie d'étalement à double faisceau.

7. Procédé selon la revendication 1, **caractérisé en ce que** la pression osmotique de la substance intercellulaire ou la quantité d'eau dans l'espace intercellulaire est déterminée à partir de la quantité d'eau dans la couche cornée en mesurant les caractéristiques électriques de la couche cornée, qui sont sélectionnées dans le groupe constitué par la conductivité électrique transversale de la couche cornée à des courants continu et/ou alternatif, ainsi que la permittivité diélectrique.

8. Procédé selon la revendication 4, **caractérisé en ce que** la concentration de glucose dans le sang et ses changements physiologiques sont déterminés sur la base de la dynamique temporelle du paramètre physiologique, caractérisant l'état de phase de la substance intercellulaire, et de la dynamique temporelle du biomarqueur, caractérisant le taux métabolique de la cellule, et le paramètre physiologique est la pression osmotique de la substance intercellulaire et/ou la quantité d'eau dans l'espace intercellulaire, et le biomarqueur est la pression osmotique des substances intracellulaires et/ou la quantité d'eau dans le volume intracellulaire, de plus, la substance intercellulaire indiquée sous l'applicateur est un biocapteur naturel avec une structure en phase hétéro avec une sélectivité pour la molécule de glucose et une sensibilité au flux thermique, de préférence le biomarqueur, caractérisant le taux métabolique d'une cellule, est la quantité totale d'eau dans la zone tissulaire sous l'applicateur, mesurée par une méthode électrométrique et/ou spectrale, qui est sélectionnée dans un groupe comprenant : spectroscopie colorimétrique isotherme, spectroscopie par réflexion diffuse, spectroscopie Raman, spectroscopie optique-acoustique.

9. Dispositif pour la spectroscopie calorimétrique isotherme de composants biochimiques de tissu vivant contenant de la chaleur et un applicateur étanche à l'eau fait avec la possibilité d'appliquer sur la peau du patient avec une pression dosée, un capteur de température, un ou plusieurs capteurs de paramètres physiologiques, caractérisant l'état de phase thermodynamique de la substance intercellulaire sous l'applicateur, une ou plusieurs sources de rayonnement électromagnétique, à ce que la source de rayonnement électromagnétique soit faite avec la possibilité de rayonner avec une intensité constante et/ou modulée et/ou une modulation de fréquence ; un dispositif pour créer un effet d'étalonnage, tandis que les capteurs de paramètres physiologiques sont situés sous l'applicateur, tandis que les signaux des capteurs ci-dessus sont reçus séquentiellement sur les entrées du module amplificateur et/ou du détecteur synchrone et/ou du convertisseur analogique-numérique installé sur la surface supérieure de l'applicateur, sur l'unité de traitement de l'information et l'unité d'affichage de l'information.

10. Dispositif selon la revendication 9, **caractérisé en ce que** les capteurs de paramètres physiologiques caractérisant l'état thermodynamique de la substance intercellulaire sous l'applicateur sont réalisés en tant que capteurs de la pression osmotique de la substance intercellulaire et/ou capteurs de la quantité d'eau dans l'espace tissulaire intercellulaire dans le volume local sous l'applicateur et/ou capteurs de la pression élastique du tissu vivant sous l'applicateur.

11. Dispositif selon la revendication 9, **caractérisé en ce que** le dispositif pour créer un effet d'étalonnage est une source d'énergie thermique, réalisée sous la forme d'une résistance et/ou d'un élément Peltier et/ou d'une source de courant électrique et/ou de tension ; un dispositif pour créer une pression dosée sur la surface de l'applicateur.

12. Dispositif selon la revendication 10, dans lequel un capteur de pression osmotique de la substance intercellulaire ou un capteur de la quantité d'eau dans l'espace intercellulaire du tissu dans un volume local sous l'applicateur est un capteur électrométrique basé sur la mesure des caractéristiques électrophysiques de la couche cornée de l'épiderme sous l'applicateur, dont le principe de mesure est choisi dans un groupe comprenant la mesure de la conductivité électrique transversale de la couche cornée à un courant constant et/ou alternatif ; la mesure de la permittivité diélectrique de la couche cornée ; la mesure de la conductivité électrique et/ou de la permittivité diélectrique du tissu sous l'applicateur.

13. Dispositif selon la revendication 10, **caractérisé en ce que** le capteur spectral, contenant une source et un récepteur de rayonnement électromagnétique, est basé sur des mesures spectrales de la quantité d'eau dans la couche cornée par les fréquences caractéristiques de l'eau dans la couche cornée basées sur une méthode spectrale sélectionnée dans un groupe comprenant la spectroscopie calorimétrique isotherme, la spectroscopie d'absorption, la spectroscopie de diffusion-réflexion, la spectroscopie Raman, la spectroscopie optique-acoustique.

14. Dispositif selon l'une quelconque des revendications 9 à 10, **caractérisé en ce qu'**il contient en outre un dispositif spectral comprenant une source et un récepteur de rayonnement électromagnétique pour des mesures spectrales basées sur la spectroscopie de diffusion-réflexion.
